# EUROPEAN PATENT APPLICATION

(11) **EP 4 810 096 A1**
(43) Date of publication of application: **23.09.2026**
(21) Application number: 26155229.3
(22) Date of filing: 30.01.2026
(51) Int. Cl.: A61M 25/01, A61B 17/00

(54) **STEER MECHANISMS AND INDEPENDENT TIP ROTATION FOR CATHETERS**

(30) Priority: 31.01.2025 US 202519042885
(71) Applicant: Biosense Webster (Israel) Ltd., 2066717 Yokneam (IL)
(72) Inventor: GRIFFIN, Wyatt, Irvine, 92618 (US); RAMPA, Sreekanth Reddy, Irvine, 92618 (US); VU, Audrey, Irvine, 92618 (US); GOVEA, Michael, Irvine, 92618 (US); RODRIGUEZ, Jasson, Irvine, 92618 (US)
(74) Representative: Carpmaels & Ransford LLP

(57) **Abstract**

Disclosed herein are assemblies that allow for 4-way steering of a catheter tip (1008), independent tip rotation, detent features for a home position, and/or auto-lock features. An example assembly may be at least partially disposed in a handle assembly (1002) of a catheter (1004). The example assembly may control deflection of a catheter tip.

## Description

### INCORPORATION BY REFERENCE

This application incorporates by reference herein the entire disclosures of the following applications, which are incorporated by reference herein for all purposes: PCT/US2019/061228 filed November 13, 2019; U.S. Prov. App. No. 62/760,784 filed November 13, 2018; WO2018/017717, published 1/25/2018; US20220401070A1; and WO 2018/182836, published 10/4/2018.

All publications and patent applications mentioned in this specification are herein incorporated by reference to the same extent as if each individual publication or patent application was specifically and individually indicated to be incorporated by reference.

### BACKGROUND

A wide variety of intravascular medical devices are known. Improved systems, devices, and methods that facilitate better control, positioning, and usability of medical devices are needed.

### SUMMARY

It is to be understood that both the following general description and the following detailed description are exemplary and explanatory only and are not restrictive. Methods and systems for catheter configurations are described.

Systems or devices, which may be referred to as an assembly or assemblies in the present disclosure, may include an elongate shaft having a generally disc-shaped flange disposed adjacent a proximal end thereof. Systems or devices may include a first control knob having a first main body and a first aperture formed in the first main body configured to receive the elongate shaft therethrough. Systems or devices may include a second control knob having a second main body and a second aperture formed in the second main body configured to receive the elongate shaft therethrough. Systems or devices may include a first pin carrier having a first carrier body and a first carrier aperture formed in the first carrier body configured to receive the elongate shaft therethrough. Systems or devices may include a second pin carrier having a second carrier body and a second carrier aperture formed in the second carrier body configured to receive the elongate shaft therethrough. Systems or devices may include a plurality of pins disposed partially within the first pin carrier and disposed partially within the second pin carrier. Systems or devices may include a plurality of pull lines disposed through at least a portion of the elongate shaft and extending external to the elongate shaft about one or more of the plurality of pins. At least a first pull line of the plurality of pull lines may be coupled to the first control knob and may be configured to move in response to an adjustment of the first control knob. At least a second pull line of the plurality of pull lines may be coupled to the second control knob and may be configured to move in response to an adjustment of the second control knob. The plurality of pins may be configured to apply tension to at least one of the first pull line or the second pull line.

Systems or devices may include an elongate shaft having a generally disc-shaped flange disposed adjacent a proximal end thereof. Systems or devices may include a first control knob having a first main body and a first aperture formed in the first main body configured to receive the elongate shaft therethrough. The first control knob may include a first keyed feature configured for a first knob cover. The first control knob may include a first anchor. Systems or devices may include a second control knob having a second main body and a second aperture formed in the second main body configured to receive the elongate shaft therethrough. The second control knob may include a second keyed feature configured for a second knob cover. The second control knob may include a second anchor. Systems or devices may include a first pin carrier having a first carrier body and a first carrier aperture formed in the first carrier body configured to receive the elongate shaft therethrough. Systems or devices may include a second pin carrier having a second carrier body and a second carrier aperture formed in the second carrier body configured to receive the elongate shaft therethrough. Systems or devices may include a plurality of pins disposed partially within the first pin carrier and disposed partially within the second pin carrier. Systems or devices may include a plurality of pull lines disposed through at least a portion of the elongate shaft and extending external to the elongate shaft about one or more of the plurality of pins. At least a first pull line of the plurality of pull lines may be coupled to the first anchor and may be configured to move in response to an adjustment of the first control knob. At least a second pull line of the plurality of pull lines may be coupled to the second anchor and may be configured to move in response to an adjustment of the second control knob. The plurality of pins may be configured to apply tension to at least one of the first pull line or the second pull line.

Systems or devices may include an elongate shaft having a generally disc-shaped flange disposed adjacent a proximal end thereof. Systems or devices may include a first control knob having a first main body and a first aperture formed in the first main body configured to receive the elongate shaft therethrough. The first control knob may include a first keyed feature configured for a first knob cover. The first control knob may include a first anchor and a second anchor. Systems or devices may include a second control knob having a second main body and a second aperture formed in the second main body configured to receive the elongate shaft therethrough. The second control knob may include a second keyed feature configured for a second knob cover. The second control knob may include a third anchor and a fourth anchor. Systems or devices may include a first pin carrier having a first carrier body and a first carrier aperture formed in the first carrier body configured to receive the elongate shaft therethrough. Systems or devices may include a second pin carrier having a second carrier body and a second carrier aperture formed in the second carrier body configured to receive the elongate shaft therethrough. Systems or devices may include a plurality of pins disposed partially within the first pin carrier and disposed partially within the second pin carrier. Systems or devices may include a plurality of pull lines disposed through at least a portion of the elongate shaft and extending external to the elongate shaft about one or more of the plurality of pins. At least a first pull line of the plurality of pull lines may be coupled to the first anchor and may be configured to move in response to an adjustment of the first control knob. At least a second pull line of the plurality of pull lines may be coupled to the second anchor and may be configured to move in response to an adjustment of the first control knob. At least a third pull line of the plurality of pull lines may be coupled to the third anchor and may be configured to move in response to an adjustment of the second control knob. At least a fourth pull line of the plurality of pull lines may be coupled to the fourth anchor and may be configured to move in response to an adjustment of the second control knob. The plurality of pins may be configured to apply tension to at least one of the first pull line, the second pull line, the third pull line, or the fourth pull line.

These and other features and advantages are described in greater detail below.

### BRIEF DESCRIPTION OF THE DRAWINGS

Some features are shown by way of example, and not by limitation, in the accompanying drawings. In the drawings, like numerals reference similar elements.
Figure 1A illustrates an example embodiment of a system that includes steering and a medical device.
Figure 1B illustrates a cross section A-A of the steering and device portion of the medical device of Figure 1A.
Figure 2 illustrates an example system that includes a handle assembly with a plurality of actuators, a steerable sheath and medical tool.
Figures 3Ai-3Aiii illustrate an example steerable shaft with pull wires.
Figures 3Bi and 3Bii illustrate an example steerable shaft with pull wires.
Figures 3Ci and 3Cii illustrate an example steerable shaft with pull wires.
Figures 3Di-3Div illustrate an example steerable shaft with pull wires.
Figure 3E illustrates an example steerable shaft with one or more pull wires circumferentially interwoven into braid wires of the shaft.
Figure 4 illustrates an example portion of an example system that includes a bundle.
Figure 5 illustrates an example proximal end of a medical tool, the tool including a conductor bundle that extends into a proximal connector within which is housed a printed circuit board (PCB).
Figure 6A illustrates a portion of an example medical tool that includes a flexible circuit strip.
Figure 6B illustrates an example proximal portion of a strip.
Figure 6C illustrates a detailed view of an example proximal portion of a strip.
Figure 6D illustrates an end view of an example flex strip.
Figure 6E illustrates an example stack of flex strips.
Figure 6F illustrates an example stack of flex strips and ground and shield strips.
Figure 6G illustrates an example bundle including a tubing material around a stack of strips and shield and ground strips.
Figure 7 illustrates an integrated system of the steerable sheath and medical tool wherein the system is connected to a console via a connector cable.
Figures 8A and 8B illustrate an example handle assembly that can be used with any of the inner and outer elongate bodies or shafts herein.
Figure 9A illustrates a portion of an example inner elongate body, or inner shaft.
Figure 9B illustrates a portion of an example outer elongate body, or outer shaft.
Figure 9C illustrates a portion of an example medical device including the elongate bodies (or shafts) from figures 9A and 9B.
Figure 9D illustrates a section of the device in the deflectable portion from figure 9C.
Figure 10A illustrates a portion of an example handle assembly.
Figure 10B is an exploded view illustrating an example outer elongate body (or outer shaft) movement subassembly.
Figure 10C illustrates a side sectional view of the handle assembly from Figure 10A.
Figure 11 illustrates an example handle assembly that includes rotation indicators for first and second actuators.
Figure 12A illustrates internal components of an example handle assembly that includes a tensioning member, or flattening member.
Figure 12B illustrates internal components of an example handle shell that include one or more guiding features positioned to help stabilize a tensioning member in the handle assembly.
Figure 12C illustrates a side view of an example handle shell.
Figure 12D illustrates (in a side view) internal components of an example handle assembly that includes a tensioning member, or flattening member.
Figure 12E illustrates a side view of an example handle shell.
Figure 12F illustrates (in a side view that is the other side relative to figure 12D) internal components of an example handle assembly that includes a tensioning member, or flattening member.
Figure 13A illustrates an example flexible cable bundle with at least a portion that is twisted relative to a long axis.
Figure 13B illustrates an example flexible cable bundle with at least a portion that is twisted relative to a long axis.
Figure 13C illustrates an example flexible cable bundle with at least a portion that is twisted relative to a long axis.
Figure 13D illustrates an example flexible cable bundle with at least a portion that is twisted relative to a long axis.
Figure 13E illustrates a detail from figure 13D.
Figures 14-18 illustrate example medical devices with navigation interface.
Figures 19-24 illustrate example transducer tips for medical devices.
Figure 25 illustrates an example assembly for a medical device.
Figure 26 illustrates an arrangement of navigation sensors for a medical device.
Figure 27A illustrates an example assembly.
Figure 27B illustrates an exploded view of the example assembly of Figure 27A.
Figure 27C illustrates an example pin carrier configured to be disposed in the example assembly of Figure 27A.
Figure 27D illustrates an example knob configured to be disposed in the example assembly of Figure 27A.
Figure 27E illustrates an example shaft configured to be disposed in the example assembly of Figure 27A.
Figure 28A illustrates an example assembly.
Figure 28B illustrates an exploded view of the example assembly of Figure 28A.
Figure 28C illustrates an example pin carrier configured to be disposed in the example assembly of Figure 28A.
Figure 28D illustrates an example knob configured to be disposed in the example assembly of Figure 28A.
Figure 28E illustrates an example shaft configured to be disposed in the example assembly of Figure 28A.
Figure 28F illustrates an example knob cover configured to be in communication with the example assembly of Figure 28A.
Figure 28G illustrates an example tip rotation base configured to be in communication with the example assembly of Figure 28A.
Figure 28H illustrates an example inner shaft insert configured to be in communication with the example assembly of Figure 28A.
Figure 28I illustrates an exploded view of a handle assembly configured to comprise the example assembly of Figure 28A.
Figure 28J illustrates a handle assembly configured to comprise the example assembly of Figure 28A.
Figure 29A illustrates an example assembly.
Figure 29B illustrates an exploded view of the example assembly of Figure 29A.
Figure 29C illustrates an example pin carrier configured to be disposed in the example assembly of Figure 29A.
Figure 29D illustrates a relationship between an effective diameter created by the pin receptacles of the pin carrier and a wrap angle experienced by pull lines associated with the pin carrier.
Figure 29E illustrates an example knob configured to be disposed in the example assembly of Figure 29A.
Figure 29F illustrates an example shaft configured to be disposed in the example assembly of Figure 29A.
Figure 29G illustrates an example knob cover configured to be in communication with the example assembly of Figure 29A.
Figure 29H illustrates an example tip rotation base configured to be in communication with the example assembly of Figure 29A.
Figure 29I illustrates an example inner shaft insert configured to be in communication with the example assembly of Figure 29A.
Figure 29J illustrates an exploded view of a handle assembly configured to comprise the example assembly of Figure 29A.
Figure 29K illustrates a handle assembly configured to comprise the example assembly of Figure 29A.
Figure 30A illustrates an example assembly.
Figure 30B illustrates an exploded view of the example assembly of Figure 30A.
Figure 30C illustrates an example pin carrier configured to be disposed in the example assembly of Figure 30A.
Figure 30D illustrates an example knob configured to be disposed in the example assembly of Figure 30A.
Figure 30E illustrates an example shaft configured to be disposed in the example assembly of Figure 30A.
Figure 30F shows a relationships between several components of the assembly of Figure 30A to apply tension to a pull line.
Figure 30G illustrates an example knob cover configured to be in communication with the example assembly of Figure 30A.
Figure 30H illustrates an example tip rotation base configured to be in communication with the example assembly of Figure 30A.
Figure 30I illustrates an example inner shaft insert configured to be in communication with the example assembly of Figure 30A.
Figure 30J illustrates an exploded view of a handle assembly configured to comprise the example assembly of Figure 30A.
Figure 31A illustrates an example assembly.
Figure 31B illustrates an exploded view of the example assembly of Figure 31A.
Figure 31C illustrates an example pin carrier configured to be disposed in the example assembly of Figure 31A.
Figure 31D illustrates an example knob configured to be disposed in the example assembly of Figure 31A.
Figure 31E illustrates an example shaft configured to be disposed in the example assembly of Figure 31A.
Figure 31F illustrates a top-down view of the example assembly of Figure 31A.
Figure 31G shows a relationships between several components of the assembly of Figure 31A to apply tension to a pull line.
Figure 31H illustrates an example knob cover configured to be in communication with the example assembly of Figure 31A.
Figure 31I illustrates an example tip rotation base configured to be in communication with the example assembly of Figure 31A.
Figure 31J illustrates an example inner shaft insert configured to be in communication with the example assembly of Figure 31A.
Figure 31K illustrates an exploded view of a handle assembly configured to comprise the example assembly of Figure 31A.
Figure 32 shows an alternate example design for auto-lock rings.

The accompanying drawings show examples of the disclosure. It is to be understood that the examples shown in the drawings and/or discussed herein are non-exclusive and that there are other examples of how the disclosure may be practiced.

### DETAILED DESCRIPTION

The accompanying drawings, which form a part hereof, show examples of the disclosure. It is to be understood that the examples shown in the drawings and/or discussed herein are non-exclusive and that there are other examples of how the disclosure may be practiced.

The systems and methods described herein may utilize an inner and outer catheter shaft. The inner shaft may control deflection in 4-directions, anterior and posterior (A/P) and left and right (L/R). The outer shaft may control independent tip rotation. An assembly may be created with a central shaft, deflection knobs, pin carriers, dowel pins, retaining rings, and ball detents. The central shaft may act as a base for the other subassembly components and may contain features to attach and retain components in translation and/or rotation. The deflection knobs may rotate freely on the central shaft and may contain rotation limiting features and tie points for the pull lines. The pin carriers may be fixed in rotation and translation on the central shaft and may house dowel pins to act as low friction pull line guides. The pin carriers may also contain features to limit the amount of deflection knob rotation. An insert may be attached (e.g., bonded, coupled to, in communication with, etc.) to the inner shaft and the insert/inner shaft may be placed into the assembly while the pull lines may be routed to respective deflection knobs. The deflection planes may be aligned and the insert/inner shaft may be attached to the assembly. The pull lines may be tensioned and tied to the deflection knobs.

Deflection knob covers may be attached to the deflection knobs and may comprise usability features (e.g. grooves, texturing) for the user to interface with. A tip rotation base may be used as a base component for a rubberized grip and the outer shaft. The tip rotation base may comprise one or more threaded holes for one or more ball detents. The outer shaft may be attached to the tip rotation base. The outer shaft/tip rotation base may be disposed over the inner shaft and may attach to the assembly using a retaining groove and ball detent. The rubberized grip may be disposed over the outer shaft and may be attached to the tip rotation base. The assembly of all components may be disposed into one or more handle pieces.

Disclosed herein is a catheter comprising features such as detent features for home (e.g., start, initial, original, etc.) position and independent tip rotation for the transducer. Disclosed herein is a catheter combining 4-way steer, detent features, and independent tip rotation, giving a user options for catheter maneuverability and usability.

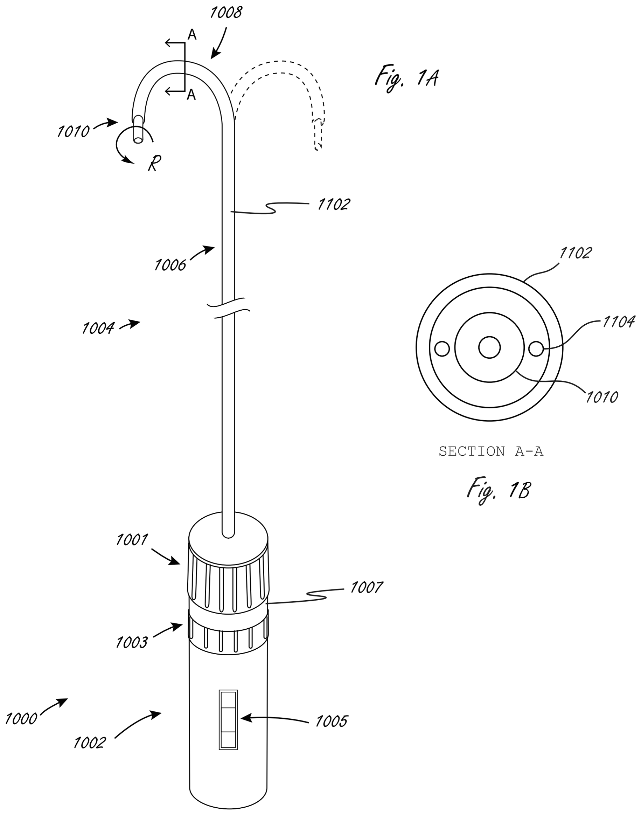

Figure 1A illustrates an example embodiment of a system that integrates steering and a medical device. System 1000 includes handle assembly 1002 and steering and medical device portion 1004. Steering and medical device portion 1004 includes a proximal portion 1006 and steerable portion 1008. The system is adapted so that handle assembly 1002 can be actuated to cause steering of the steerable portion 1008, and optionally can be further actuated to cause movement of medical device 1010 relative to steering and medical device portion 1004. In this example embodiment, handle assembly 1002 includes first actuator 1001, second actuator 1003, and third actuator 1005. First actuator 1001 is adapted to be actuated (in this example rotated) relative to handle body 1007 to cause the steering of steerable portion 1008, and specifically steering outer sheath 1102. Steerable portion 1008 in this embodiment can be steered, or bent, into the configuration shown in Figure 1A in solid lines, and can also be steered into the configuration shown in dashed lines, or anywhere in between, and in some embodiments the opposite steering function is limited to simply straightening the shaft from an initial bent configuration, such as the solid line bent configuration in Figure 1A. The term "steer" in this disclosure means to deflect or bend, optionally via actuation of at least one pull wire, but in some instances the term can include shaft rotation (torqueing) and axial movement. The term "pull wire" herein refers to any element that may transmit a tensile force from the proximal end of the device to the distal end region. Pull wires may be comprised of metal wire such as stainless steel or nickel titanium, either solid or stranded/braided, or it may be comprised of a polymer such as aramid fiber (Kevlar^{®}), polyethylene, ptfe, eptfe, etc., preferably stranded/braided, but also in monofilament form. In a preferred embodiment, the pull wire is constructed from an aramid fiber bundle having four 50 denier multifilament (approximately 25 filaments) threads braided together at a high picks per inch. The wire cross-sectional diameter is typically in the .005"-.012" range, more preferably .008"-.010", although braided or stranded wire may flatten or ovalize in the device lumen. The preferred construction embodiments are believed to provide optimized strength and wear resistance for the size necessary to keep the shaft diameters to a minimum. Optional second actuator 1003 is adapted to be actuated relative to handle body 1007 (in this example rotated) to cause rotation of medical tool 1010 relative to shaft 1102 (labeled as rotation movement "R"), and optional actuator 1005 is adapted to be actuated relative to handle body 1007 (in this example axially) to cause axial (distal-proximal) movement of medical device 1010 relative the outer sheath 1102. Proximal portion 1006 is not configured to bend significantly when steerable portion 1008 is steered (bent/deflected), although the proximal portion may flex and bend to conform to the anatomy within which it is used. In many embodiments, this is accomplished by constructing the steerable portion 1008 from a softer or less rigid material and/or composite construction than the proximal portion 1006.

The embodiment shown in Figure 1A is an example of an apparatus that includes an integrated handle assembly that is in operable communication with both a steerable outer shaft and an inner medical tool. The handle assembly is integrated in that it is assembled and constructed to be in operable communication with the outer shaft and the inner medical tool prior to packaging and use. "Integrated" as that term is used in the context of an integrated handle assembly refers to a handle assembly in which at least one part of the handle assembly has to be broken or taken apart before the medical tool can be removed from within the outer shaft.

Figure 1B illustrates an example cross section A-A (shown in Fig. 1A) of the steering and device portion 1004, and specifically in the steerable portion 1008. In this embodiment medical device 1010 is sized and configured to be disposed within a steerable sheath. The steerable sheath includes an outer shaft 1102 and a set of pull wires 1104, which are axially fixed in a distal region of steerable portion 1008.

The medical tool in Figures 1A and 1B can be, for example, any medical tool herein, such as an ultrasound tool. When "ultrasound probe" is used herein, it generally refers to an elongate tool that includes at least one ultrasound transducer and one or more conductive elements that electrically connect the at least one ultrasound transducer to a proximal region of the elongate tool. A proximal region of the ultrasound probe includes, or is modified to include, at least one proximal contact, which is in electrical communication with the at least one ultrasound transducer, and which can be put into electrical communication with, optionally via attachment to, an electrical contact on another device, cable, or connector.

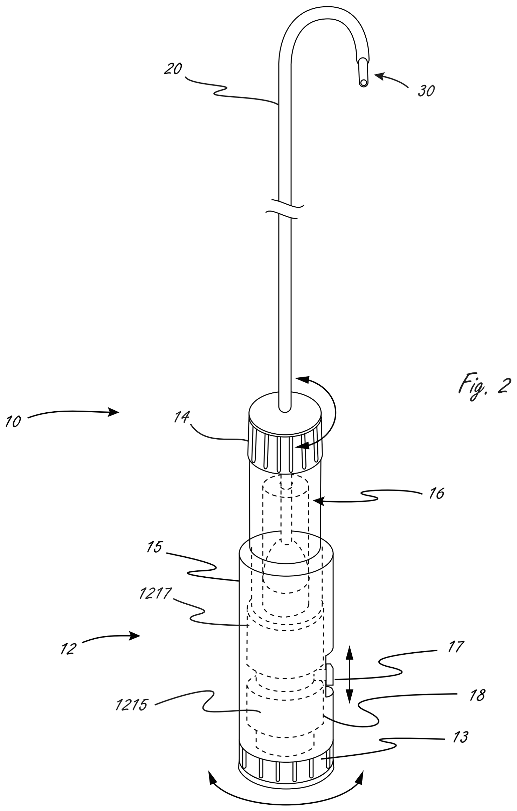

Figure 2 illustrates an example system 10 that is adapted to function similarly to the system in figures 1A and 1B, and also illustrates example internal components of handle assembly 12 (internal components shown as dashed lines). Handle assembly 12 is integrated and in operable communication with outer steerable shaft 20 and medical tool 30. Handle assembly 12 includes actuator 14 that is adapted to, when actuated relative to handle body 15, cause steering of steerable shaft 20. Actuator 14 is in operable communication with steerable shaft 20 via steering control 16 disposed in handle assembly 12. Medical tool 30 includes a proximal portion 18 disposed within and incorporated into handle assembly 12. Actuator 13 is in operable communication with medical tool 30, and actuation of actuator 13 (in this example rotation) relative to handle body 15, causes rotation of medical tool 30 relative to outer shaft 20 via rotation control 1215. Optional third actuator 17 is also in operable communication with medical tool 30, and is adapted to be actuated, in this embodiment, axially (relative to handle body 15), to cause axial movement of medical tool 30 relative to outer steerable shaft 20 via axial control 1217.

The medical tool in Figure 2 can be, for example, any medical tool herein, such as an ultrasound tool.

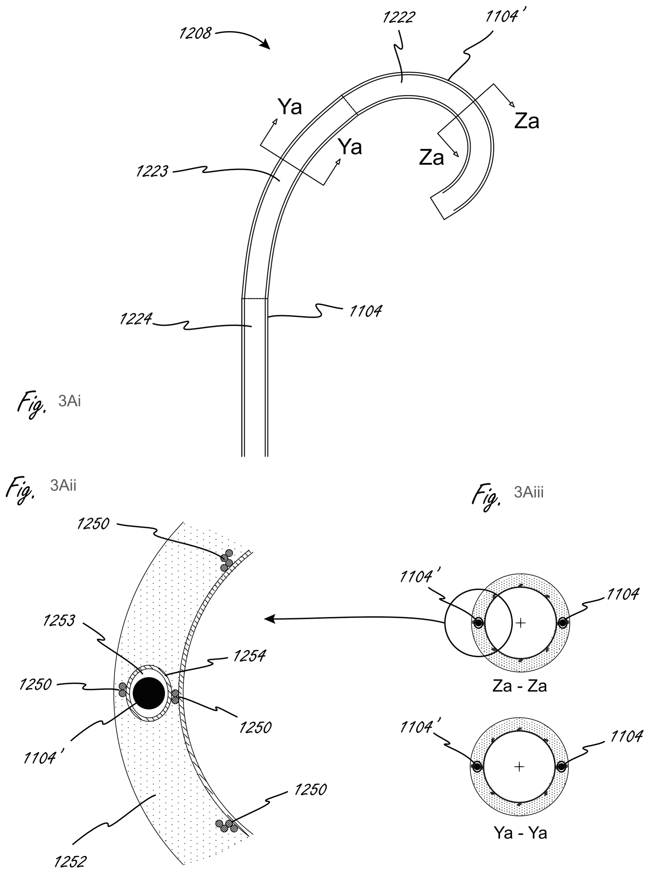

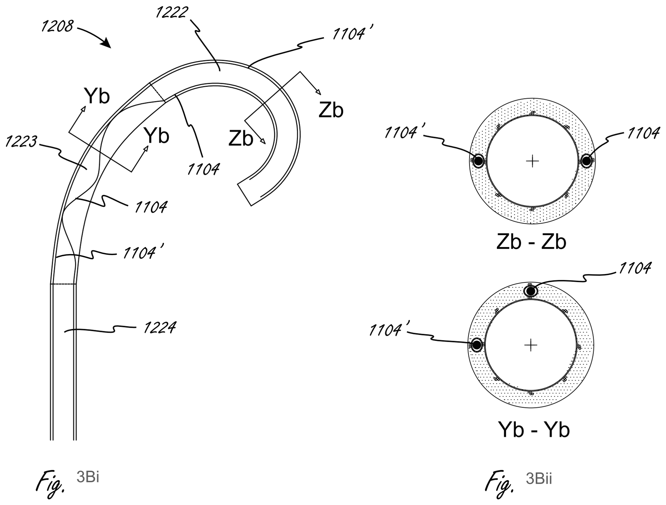

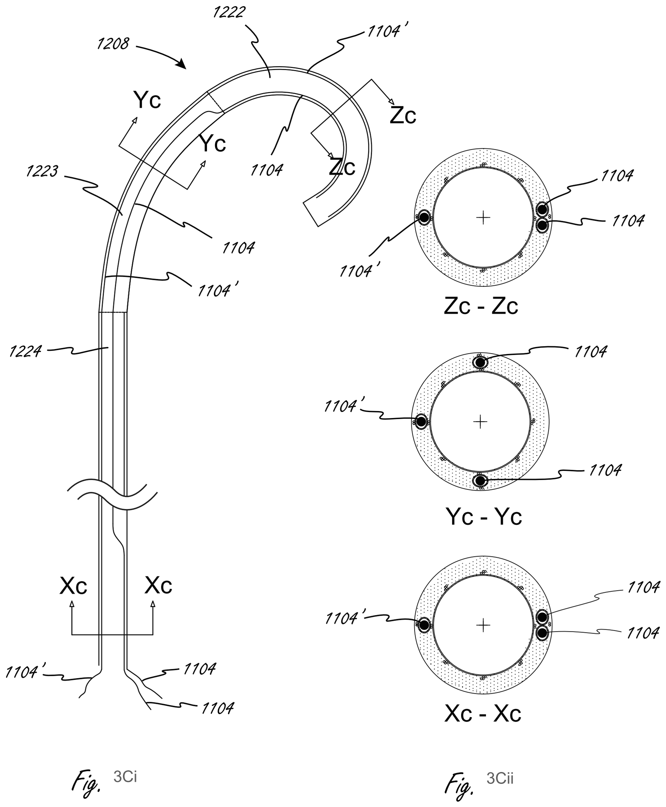

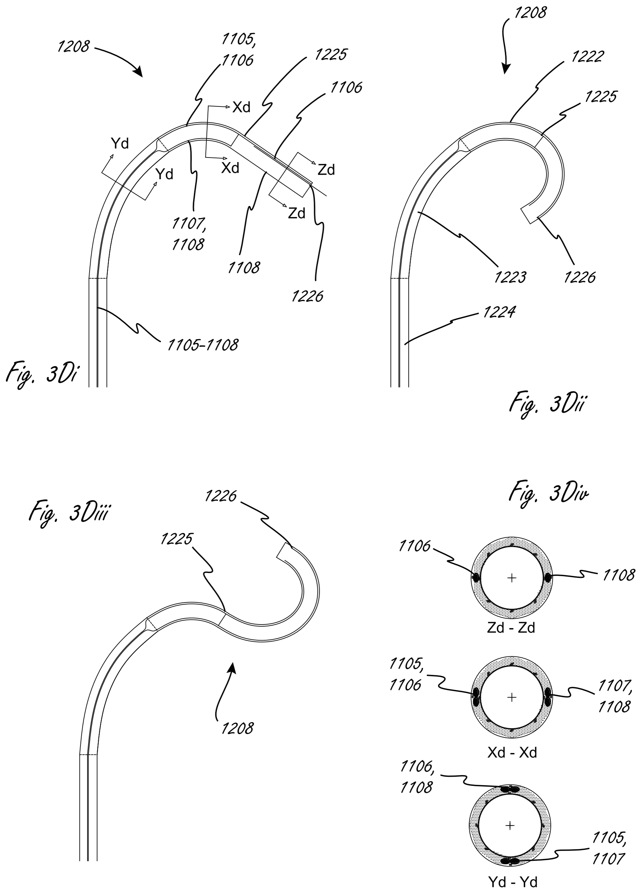

Figures 3A-3E represent example embodiments of a distal region of the sheath portion 1208 of steerable sheath 1202 in system 1200. For simplicity, the illustrated cross-sections show only the outer sheath 1208 and not the inner tool 1212. The outer sheath 1208 preferably has a composite construction to improve torque transmission applied to the outside of the shaft from the proximal end, or to resist torque forces applied to it from within the shaft, such as from tool 1212. As illustrated in Figure 3Ai-iii, in order to form the composite, multiple braid elements 1250, preferably formed from metal wire (round, pairs of round, or ribbon shaped) and/or multiple fibers (e.g., aramid or nylon), may be braided directly over a thin wall (e.g., .0010" ± .0005") lubricious liner tube 1251, such as a PTFE or FEP material. A thermoplastic polymer 1252 (such as Pebax in a range of durometers from 25D-72D, or nylon, or other common catheter materials) may be laminated with heat using heat shrink tubing (such as FEP) to reflow the polymer over the braid elements 1250 and liner tube 1251 to form a uniform member. The thermoplastic polymer 1252 may also have radiopaque compounds that include materials such as bismuth, barium sulfate, or tungsten in order that the tip of the sheath be visible to the user under fluoroscopy.

In the embodiment of Figure 3Ai-iii, the pull wire 1104 is preferably parallel to the central access in the steerable (deflectable) portion 1222 of the sheath and also preferably provided in a lumen 1253 created within the wall of the steerable sheath 1208. This lumen may be created during the thermoplastic polymer tubing extrusion process or during a shaft heat lamination fusing process with the aid of a removable mandrel. The pull wire lumen 1253 may further be created by incorporating a pull wire tube 1254, preferably temporarily supported by a removable mandrel, within the wall. The removable mandrel may also be placed alongside the pull line 1104 or 1104' during the fusing process, resulting in a somewhat ovalized lumen 1253 within which a fiber pull wire may be allowed to flatten into, allowing space for free movement of the pull wire. The tube 1254 may include PTFE, FEP, polyimide, or another material which maintains its wall integrity during a heat lamination process up to approximately 500 °F. The tube is preferably surrounded and supported by the thermoplastic polymer 1252 which is preferably heat laminated against the tube.

In another embodiment, the pull wire lumen, preferably comprising the pull wire tube, is incorporated within the weave of the braid elements 1250. For example, braid elements 1250 running in one direction would pass under the pull wire lumen, while those running in the opposite direction would pass over the pull wire lumen. The braid reinforcement provides a more dimensionally stable lumen during catheter manipulations and also helps assure the straightness of the lumen as needed. Proximal to the steerable portion, the pull wire may continue proximally parallel to the central axis on the same side of the outer sheath 1208, such as is illustrated in Figure 3Ai-iii. In this embodiment and others that follow, an additional pull wire 1104' within an additional pull wire lumen routed within the wall of sheath 1208, up through the steerable portion 1222, may be required to straighten the steerable portion of the device. This straightening pull wire 1104' is preferably routed within steerable portion 1222 on the side opposite from the pull wire(s) 1104 used for steering (deflection) in the steerable portion 1222. In another embodiment, not shown, two lumens and two straightening pull wires 1104' could be used, essentially mirroring the paired 1104 pull wire configuration. These straightening wires could also be constructed to allow deflection in the opposite direction by tensioning a greater distance (beyond just straightening) within the handle.

During use, a portion 1223 of the distal catheter just proximal to the steerable (deflectable) portion 1222 may be forced to conform to a curve based on the constraints of the anatomy in which it is used. For a specific embodiment where the device is advanced into the heart chambers from a groin access, the portion 1223 forced into a curve is expected to range from 5 to 25 cm in length. During rotation of the sheath shaft 1208 from the proximal end, torque is transmitted through this distal curved region 1223 to the catheter tip. A non-uniform cross section and/or tension of the device in this region 1223 may induce a tendency for the shaft to build up and suddenly release torque, causing a "whip" or sudden jerk in rotation as it is torqued. To minimize the potential for whip, it is optional to distribute the pull wire tension and construction material around the surface of the curved region 1223. In one embodiment, such as is illustrated in Figure 3Bi-iii, the pull wire 1104 may spiral around the central axis of the sheath in at least the curved region 1223 proximal to portion 1222. The pull wire of this embodiment may make a full circumferential wrap over approximately 10 cm of length, with this value ranging 5-15 cm. The spiral may only need to be present in the curved region 1223, continuing straight proximally thereafter through proximal portion 1224 (similar to 1006), which may minimize the friction in the pull wire lumen and the associated pull wire force required to steer (deflect) the steerable portion 1222. The spiral may also make a minimum of one turn before continuing straight, or spiral the full length of the shaft. In another embodiment to minimize whip, it may only be necessary to distribute the pull wire tension to opposite sides of the shaft. As illustrated in Figure 3Ci-ii, deflection of the steerable section 1222 is accomplished with two parallel pull wires 1104 positioned adjacent one another on the same side of the sheath 1208. In the curved region 1223 and proximal portion 1224 (similar to 1006) proximal to the steerable section 1222, the pull wires are routed to opposite sides of the shaft, each 90° from the position in the steerable section 1222, to distribute the tension more evenly. While it is preferable to actuate the two parallel pull wires at the same time with equal force with the handle actuator, in other embodiments, a differential in force could be applied to steer the tip to one side or the other of the plane formed when the two are actuated with equal force. In other embodiments, any plurality of pull wires could be routed in the same configuration as illustrated in Figures 3B or Figure 3C, with the multiple proximal pull wires distributed uniformly around the shaft circumference. Also, as illustrated in Figure 3Ci-ii, the pull wires 1104 may be routed proximally along the opposite sides of the shaft for most of the shaft proximal portion 1124 length, but preferably brought back together adjacent one another near the proximal end portion of the shaft to allow the wires to exit the same side of the proximal shaft together to facilitate them being secured together to a handle component for simultaneous actuation tension.

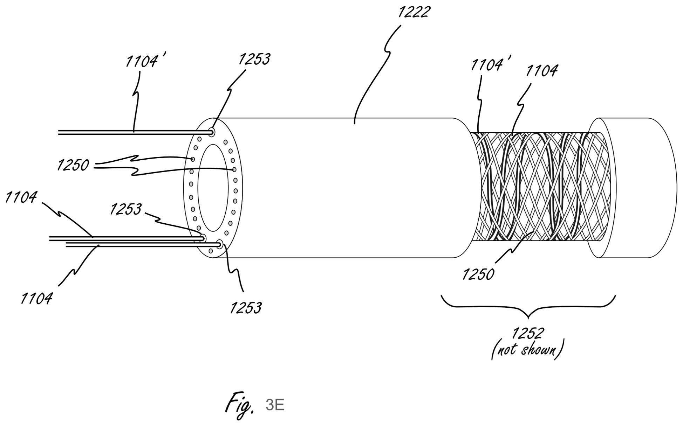

Figures 3Di-iv illustrate another embodiment of the distal region of catheter with construction similar to that previously described, but instead configured to provide a distal steerable portion 1222 which can be deflected into two different directions. As illustrated, a two pairs of pull wires 1105/1107 and 1106/1108 are along the proximal shaft region 1224 and curved region 1223. This is similar to Figure 3Ai-iii, except that the wires are paired on each side of the shaft. The routing could also be spiraled as in Figure 3Bi-ii, or other configurations discussed. Within distal steerable portion 1222, the wires are routed 90° from the proximal portions, although other angles are contemplated. At a junction 1225 within 1222 one or more of the pull wires (e.g., 1105 and 1107) may be terminated and anchored to the shaft, with the remaining pull wires (e.g., 1106 and 1108) continuing to a more distal tip location 1226 where they are anchored. This configuration allows independent actuation of pull wires terminated at 1225 and 1226 such that different shapes may be created during actuation. Figure 3Dii shows both lines 1107 and 1108 tensioned to create a variable curve in the same direction. Figure 3Diii shows lines 1107 and 1106 tensioned to create an "S" curve. Other configurations are also possible.

The pull wires (such as 1104 and 1104') may be terminated at their distal end in a manner that reliably affixes them to the wall of the distal steerable shaft portion 1222, such that they do not break or pull free under repeated applications of tension. In a preferred embodiment, shown in Figure 3E, the pull wires 1104 and 1104', upon exiting the distal pull wire lumen 1253, are circumferentially interwoven into the braid wires 1250 of the distal shaft 1222 (shown without the thermoplastic polymer 1252). One or more of the pull wires 1104 or 1104' may also be additionally or instead wrapped and/or tied around the outside of the braid wires 1250 for additional securing. The braid wires 1250 may be then trimmed distal to the securing point, with the interwoven and/or wrapped pull wires preventing the braid wires from expanding and/or unraveling. Additional adhesives such as UV cured or cyanoacrylates may also be used to secure the pull wires to the braid wires. The weave and/or wrap of the pull wires and braid wires is then laminated with a thermoplastic polymer which melts within the space around the wires and cools to secure them in place. The thermoplastic polymer may also have radiopaque compounds that include materials such as bismuth, barium sulfate, or tungsten in order that the tip of the sheath be visible to the user under fluoroscopy.

In additional embodiments, the tool 1212 may also or alternatively be constructed with one or more pull wires to deflect the tip in a manner similar to any of the previous embodiments described for the outer sheath 1208. In addition to routing the pull wires within the wall of the tubular member of the tool 1212, the pull wires could be routed next to the conductors inside the lumen of the tubular element 1212. Actuation of the pull wires could be from an actuator located in the proximal handle 1206. The distal shaft of tool 1212 may also be formed into a particular shape (e.g., an arc) such that it bends into the shape as it exits the tip of the steerable portion 1222 of outer sheath 1208. The stiffness of the distal shaft of tool 1212 is such that it does not substantially deform outer sheath 1208 while inside, but upon exiting is allowed to bend. The shape may be set by any one or combination of the following means: heat setting the polymeric material, using a moveable or fixed shaped stylet within the inner lumen of shaft 1212 or within a lumen within the wall of shaft 1212. Such a stylet could be round, oval, or rectangular in cross section, and be formed of stainless steel, nitinol, or a rigid polymer such as PEEK, Vestamid, or similar. The outer steerable sheath could alternatively be made to bend with a similar method as above, with or without additional pull wire deflection, and with or without additional shape or deflection of the distal portion of tool shaft 1212.

One aspect of the disclosure includes methods of disassociating at least a portion of the system from other components, optionally as part of a reposing process. In some embodiments the medical tool includes one or more electrical contacts that are coupled to other electrical contacts, which are in electrical communication with an energy console, and examples of consoles are known in the ultrasound art.

In an embodiment, the pull wires described in Figures 3Ai-3E may exist in a symmetric design and may be bifurcated and change relative position to each other in various sections of a respective shaft. By controlling a stiffness in a shaft and a positioning of the pull lines (e.g., moving a bifurcation point more distally), access cull may be reduced, while providing more precise control of a deflection of the distal end of the shaft. An associated shaft may comprise a plurality of bobbins (e.g., sixteen (16) bobbins), which may be tuned for increased torque response. An associated shaft may comprise a plurality (e.g., four (4)) of pull lines) for balanced braid wire, which may decrease deflection orientation changes due to annealing and sterilization. As a further example, an associated shaft may comprise a flaring inside diameter of a tip to not bind on bundle, just proximate to post.

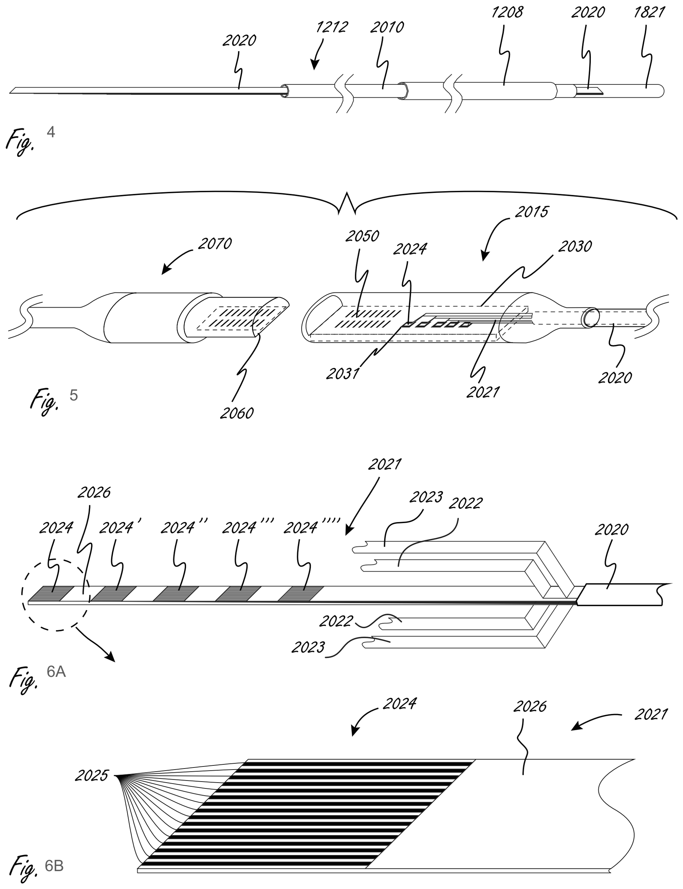

Figure 4 illustrates a portion of an example medical tool, such as an ultrasound probe, that can be electrically coupled directly or indirectly to an energy console, such as an ultrasound console.

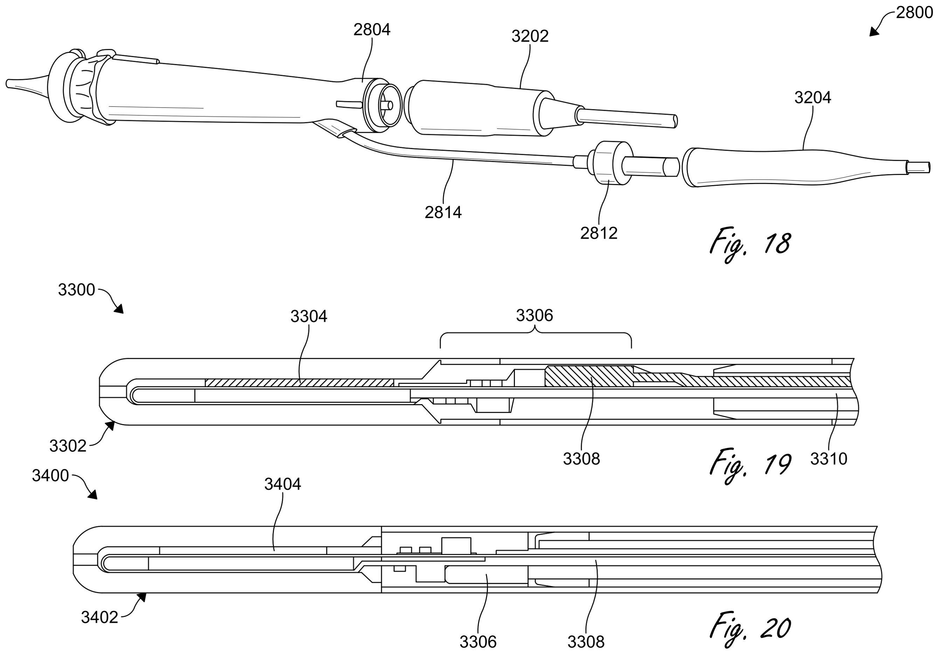

Reposing the device can involve disconnection of one or more proximal electrical contacts and moving the tool portion distally out of the distal end of the sheath portion. In this embodiment tool portion 1212 comprises at least a tool outer sheath or member 2010, distal working end 1821 (which can include at least one ultrasound transducer), and conductor bundle 2020. The conductor bundle 2020 extends from the distal working end 1821, through the tool outer member 2010 to a proximal connector (the connector and handle mechanism are not shown in Fig. 18 for clarity). In some embodiments the medical tool is used for ultrasound imaging, optionally where the distal working end 1821 comprises a two-dimensional (2D) array of piezo electric components mounted on an ASIC (application specific integrated circuit).

Figure 5 illustrates a merely example proximal end of a medical device (the medical device is shown on the right), and in this embodiment the medical device is an ultrasound probe. The proximal end 2015 of the medical device is adapted to be electrically coupled to connector cable 270, which is directly or adapted to be indirectly electrically coupled to an energy console, such as an ultrasound energy console. As illustrated in Figure 5, flexible conductor bundle 2020 extends from a distal region of the medical tool (distal region not shown) into a proximal connector 2015 within which is housed a rigid or flexible printed circuit board ("PCB") 2030. The connector bundle 2020 includes a plurality of contacts 2024 (examples of which are described below) that are attached to PCB board contacts 2031. Each individual trace from each contact 2031 is linked to individual exposed contacts 2050 on another portion, optionally more proximal, of the PCB. The individual PCB traces may also pass through other useful circuitry on the PCB. The exposed contacts 2050 are configured for a mechanical mating for electrical conduction to similar contacts 2060 on mating connector cable 2070, similar in concept to the proximal tool connector 1990 described previously, which links the tool 1204 to a user-interface console. Proximal connector 2015 can be incorporated into any of the systems, handles, steerable sheaths, medical tools, etc., herein.

"Conductor bundle" as that term is used herein may be interchangeably used with "flexible conductor bundle" unless indicated to the contrary herein.

Figures 6A and 6B illustrate an example conductor strip (also referred to herein as a flexible circuit strip) 2021 that can be included in any of the conductor bundles herein. The embodiment in Figures 6A and 6B is an example of a conductor strip that can be included in bundle 2020 from Figures 4 and 5. The embodiment in Figures 6A and 6B can be incorporated into any other system herein.

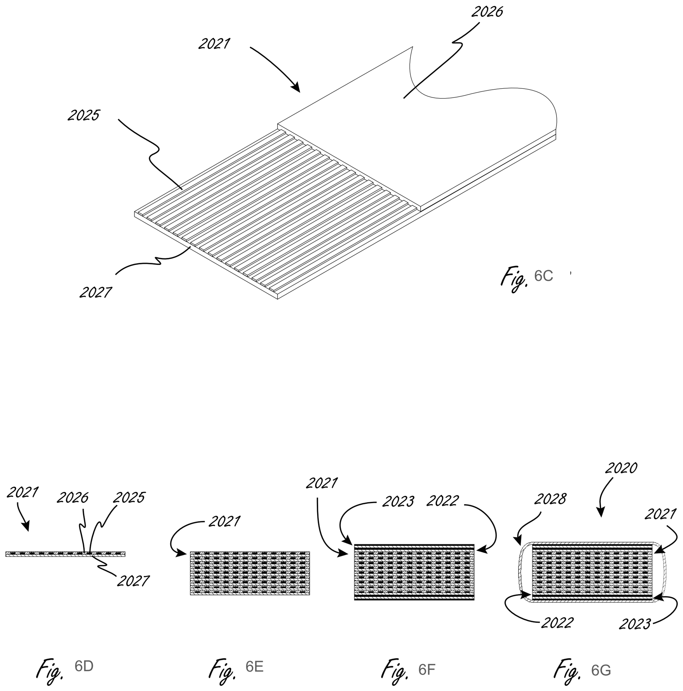

As shown in Figures 6A, 6B and 6G, conductor bundle 2020 comprises a plurality of flex circuit strips, including multi-trace strips 2021, as well as conductive strips for grounding 2022 and shielding 2023 (only a portion of which are shown). Each multi-trace strip comprises a plurality of conductive traces 2025, which can be seen clearly in Figures 6B, 6C and 6D. The number traces 2025 in Figures 6D-G is twelve, and the number of traces in Figures 6A-6C is sixteen, and they are both example as to the number of traces 2025 that can be used. Each strip 2021 can be approximately .072" wide and .0022" thick, and can optionally comprise sixteen .0022" wide x about.0007" thick conductive (e.g., copper) traces, each spaced approximately .0022" apart. The traces are disposed on an insulating substrate layer 2027, such as a polyimide substrate, and the traces can be at least partly covered by a cover layer 2026, such as a photoimageable film cover ("PIC") layer or other dry film solder mask (DFSM) or other similar material. The cover layer generally extends along most of the bundle, except at discrete locations in proximal and distal regions for electrical coupling. In other embodiments, the strip 2021 is approximately .055" wide and comprises twelve conductive traces (see figures 6D-G). In other embodiments, the strip 2021 is approximately .037" wide and comprises eight copper conductive traces. The outer strips 2022 and 2023 used for grounding and shielding may have a similar construction and dimension except they can comprise a single full width strip of copper. As optimized for a 2D piezo array, a stack of approximately seven 16-trace strips 2021 would be required (or nine 12-trace, or fourteen 8-trace), along with one each of strips 2022 and 2023 on each side of the stack of multi-trace strips. Figure 6E illustrates a portion of an example bundle 2020 with nine strips 2021 stacked together. Figure 6F illustrates a portion of the bundle that includes nine strips 2021 stacked, as well as ground strip 2022 and shield strip 2023 (only those on top are labeled). The complete bundle may optionally be held together with a, for example without limitation, about.001" wall thickness shrink tube, such as the tubing 2028 in Figure 6G. The flex circuit dimensions and number of traces discussed above are for a particular configuration of a piezo-electric array (and/or an ASIC controller thereof) and may be varied depending on how the number and size of array elements are optimized for the particular application.

The proximal end of each flex circuit strip has the conductive material (e.g., gold-plated copper) exposed over a length of approximately, for example, 3mm through removal of the cover layer 2026 at location 2024. Location 2024, and other exposed locations 2024', 2024", 2024‴, 2024ʺʺ described herein, is generally referred to as a "contact." It is understood that when used in this context, the contact actually includes a plurality of separated conductive traces (such as shown in region location), each of which is adapted to be in electrical communication with its own corresponding conductive element. "Contact" is therefore not limited to mean only a single electrical connection between two conductive elements. While Figure 6A shows a plurality of exposed regions 2024, the embodiment in Figure 6A will first be described herein as if there is only one exposed region (i.e., region 2024 at the proximal end). The strip 2021 can be made to create an electrical connection to matching exposed contacts 2031, shown in Figures 6A-C, for conductive traces on the PCB 2030. In some embodiments, sixteen individual traces, sized and spaced to match sixteen traces in the multi-trace strip 2021, would be provided within a given contact 2031. An ACF (anisotropic conductive film), soldering, conductive adhesive, mechanical connection, or any combination of these may be used to achieve a suitable electrical connection (electrical coupling) between the strip traces and the PCB contacts.

The flexible strips shown in Figures 6A-6G may comprise a variety of configurations. A modified M-fold configuration that pulls application-specific integrated circuit (ASIC) flex pads further distal may allow for more distal positioning of the inner shaft 132.

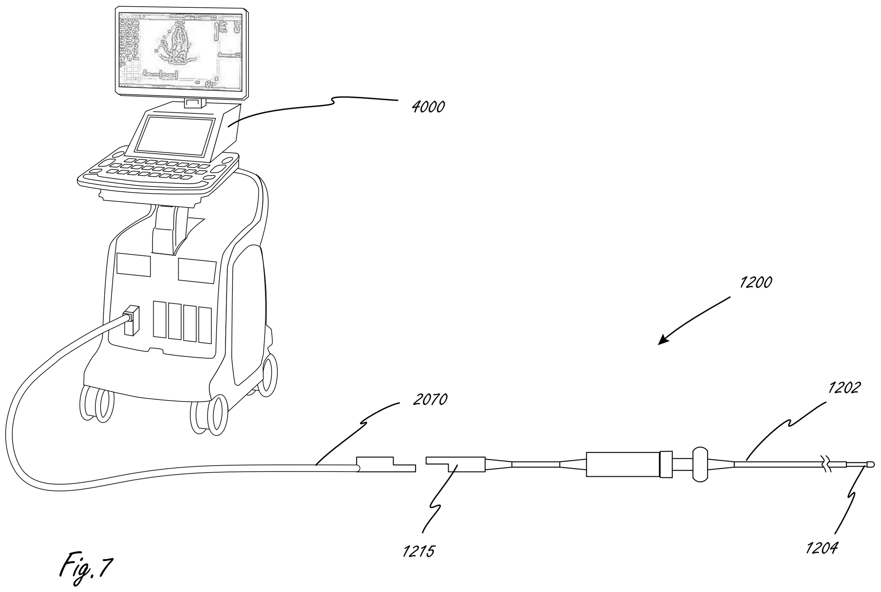

Figure 7 illustrates the integrated system 1200 of the steerable sheath 1202 and medical tool 1204 wherein the system 1200 is connected to console 4000 via the connector cable 2070. As previously described, such as for Figure 5, the tool 1204 comprises a proximal connector 2015 which forms a mating connection to cable 2070. As previously described, it is desirable to repose (e.g., reprocess and reuse) the system 1200. It is further desirable to ensure that the system is reposed only by the original manufacturer and not an unaffiliated third-party, and to ensure the device is only reused a specified number of times. To control the reposing process, a crypto-authentication chip (crypto-chip) is incorporated into the tool 1204, preferably on the PCB 2030, although other locations, such as within the steerable handle 1206, or within the tip 3000, are contemplated. The crypto-chip is programmable only by the original manufacturer who controls the authentication keys. The console 4000 to which the system 1200 is connected has a Trusted Platform Module (TPM) which also has the authentication keys. During use of the system 1200, the console 4000 is able to authenticate the system 1200 via the crypto-chip and as desired may read and write information to the chip (e.g., via an EEPROM feature). In any of the scenarios discussed, RFID chips, preferably encrypted, may be used to read and transmit data between the console, connector, and the device.

As used herein, "cleaning" can refer to any type of cleaning, such as without limitation: cleaning an interior of an outer shaft using a flushing system of cleaner and/or disinfectant and optionally mechanical scrubbing with small brushes; mechanical cleaning (e.g., wipes, brushes) an outer portion of an outer shaft and/or outer portion of a medical device shaft (e.g., ultrasound probe) with a cleaner/disinfectant, and optionally submerging the shaft in an ultrasound bath of cleaner/disinfectant for a specified period of time; and optical cleaning methods such as comprising using UV light. "Cleaning" as used here does not refer to a specific cleaning process, but rather refers to the general idea of cleaning an object.

The disclosure herein also includes methods of assembling or reassembling any of the systems, devices, subassemblies, or assemblies herein, including any of the subassemblies within any of the handle assemblies herein. For example, without limitation, the disclosure here includes methods of spooling one or more pull wires over a bearing surface in a spindle support and then around the spindle.

The methods herein also include manufacturing or constructing any of the individual components of any of the subassemblies or assemblies herein. For example, the disclosure includes methods of manufacturing handle shell components that have particular configurations (e.g., guides, walls, etc.) that can accommodate the internal parts that allow the assemblies or subassemblies herein to function as intended.

Regardless of the reference number with which they are labeled, any of the handle assemblies, medical tools, steerable sheaths, and electrical connections herein can be used together in a system in any combination with each other.

Any of the technology, including ultrasound and steering technology, in any of the following U.S. patent references may be incorporated into any of the medical tools, devices, systems, or methods of use thereof herein, the disclosures of which are incorporated by reference herein: 6100626, 6537217, 6559389, 7257051, 7297118, 7331927, 7338450, 7451650, 7451650, 7527591, 7527592, 7569015, 7621028, 7731516, 7740584, 7766833, 7783339, 7791252, 7791252, 7819802, 7824335, 7966058, 8057397, 8096951, 8207652, 8207652, 8213693, 8364242, 8428690, 8451155, 8527032, 8659212, 8721553, 8727993, 8742646, 8742646, 8776335, 8790262, 8933613, 8978216, 8989842, 9055883, 9439625, 9575165, 9639056, and 20080287783.

Any suitable disclosure above can be incorporated into any of the embodiments below. For example, aspects of devices, systems, and methods of manufacture and use are incorporated herein and can be incorporated into any of the embodiments below unless specifically indicated to the contrary.

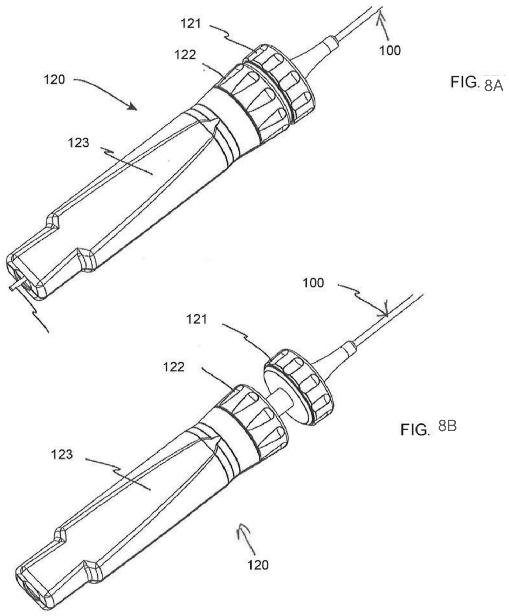

Figures 8A and 8B illustrate a merely example handle assembly that may be in operable communication with outer shaft 131 and inner shaft 132. In this example implementation, handle assembly 120 includes handle body 123 that has an outer surface that can be gripped by a user, first actuator 121, and second actuator 122. Actuator 121 can be in operable communication with outer shaft 131, and actuator 122 can be in operable communication with inner shaft 132. Actuator 121 is adapted to be both rotated and moved axially relative to handle body 123 (and relative to second actuator 122). This allows actuator 121 to cause axial movement of the medical tool 103 and rotation of the medical tool 103 relative to a distal end of inner shaft 132. Second actuator 122 is adapted to be actuated (e.g., rotated in this embodiment) relative to handle body 123 to cause deflection of the inner shaft 132. For example, the handle assembly can have internal components that interface with proximal ends of pull wires such that actuation of actuator 122 tensions one or more pull wires to cause deflection of the inner shaft. In this embodiment actuator 121 is distal to actuator 122, but in other designs their relative positions could be reversed. Figure 8B shows handle assembly 120 after actuator 121 has been advanced distally relative to its position in figure 8A. This distal advancement causes outer shaft 131 to be advanced distally, and thus causes the medical tool to be advanced distally. Actuator 121 can similarly be retracted proximally relative to its position in figure 8B. Tensioning members described further below are referenced with respect to actuator 121 being retracted proximally.

In other designs, actuator 121 could be in operable communication with an inner shaft and actuator 122 may be in operable communication with an outer shaft.

As described herein, the outer shaft can be moved axially relative to the inner deflectable shaft. The outer shaft may be constructed with sections of materials that vary in stiffness (e.g., durometer) along the length of at least a portion of the outer shaft. For example, a first portion that is distal to a second portion can have a lower durometer than the second portion. Because the outer shaft can be moved axially relative to the deflectable inner shaft, and because the stiffness of the outer shaft can vary along its length, the deflection, including the degree (or amount), of the overall device can be selectively controlled by controlling the axial position of the outer shaft (relative to the inner shaft). Axial movement of the outer shaft can thus selectively control deflection of the device. For example, a user (e.g., physician) can change or control where the bend occurs along the length of the device (measured from the distal end) by axially moving the outer shaft relative to the inner shaft. Additionally, for example, sections of varying stiffness in the outer shaft can allow for more or less deflection depending on the relative position of the outer shaft relative to the deflectable inner shaft. For example, deflecting the inner shaft at a region where the outer shaft has a relatively higher stiffness can result in less deflection than when the inner shaft is deflected at a region where the outer shaft has less stiffness. Although reference is made to distinctions in characteristics and control of the inner and outer shafts, such control of the inner shaft and outer shaft may be reversed, wherein the inner shaft is configured for rotation and the outer shaft is configured for deflection.

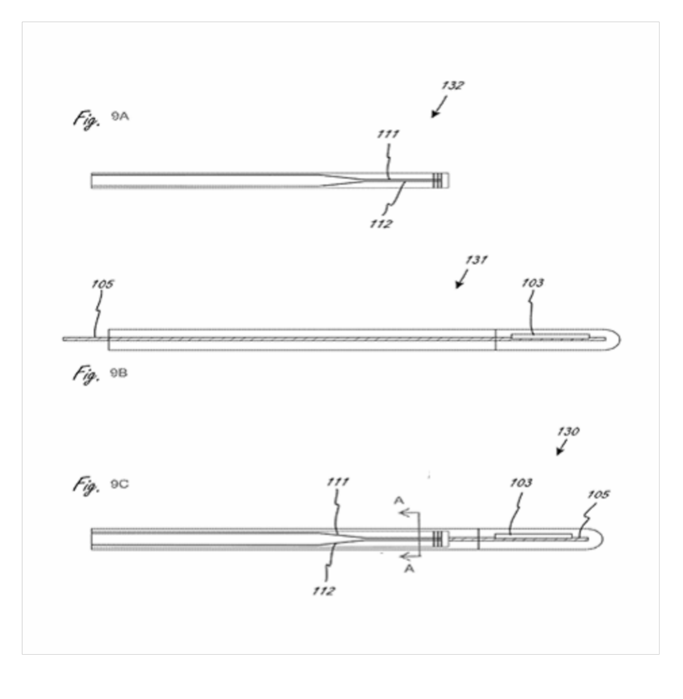

Figure 9C shows example apparatus medical apparatus 130, which includes elongate inner shaft 132 (see figure 9A) and elongate outer shaft 131 (see figure 9B). Medical apparatus 130 may also be referred to herein as a "catheter," or other medical device that includes at least one elongate shaft.

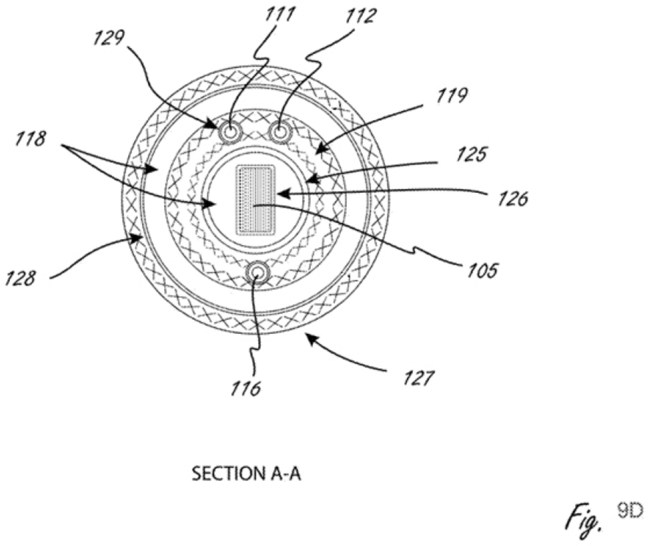

Figure 9D illustrates Section A-A shown in the assembly in figure 9C, which is a section in the deflectable section of the device. Parts from figures 9A-9C are similarly labeled. As can be seen in figure 9D, pull wires 111 and 112 are very near to one another and about 180 degrees away from straightening pull wire 116.

As is also shown in figure 9D, elongate inner shaft 132 includes two layers of braided material 119, and the pull wires are, at least at the location of this section, essentially sandwiched between the two layers of braided material. Annular spaces 118 allow freedom of movement and space for optional lubricant. Inner shaft 132 may be made from, for example without limitation, a polymeric material such as Pebax, optionally with a lubricious additive. Inner shaft 132 may include liner 125, such as a PTFE liner. The flexible cable bundle 105 may be surrounded by one or more layers of insulation 126, such as PTFE insulation. Outer shaft 131 may comprise a polymeric material 127, such as Pebax. Outer shaft 131 may also include a radially inner liner 128, such as a PTFE liner. Any of the pullwires (e.g., 111, 112, 116) may be disposed in a lumen with a liner, such as PTFE liner 129.

Medical apparatus 130 (or either of elongate shaft 132 and elongate shaft 131, individually) can be in operable communication with any of the handle assemblies herein, including handle assembly 120 shown in figures 8A and 8B.

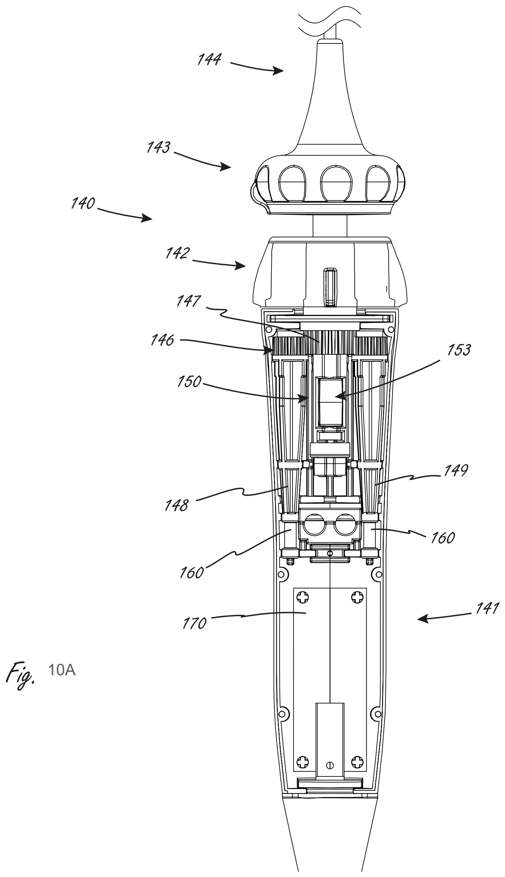

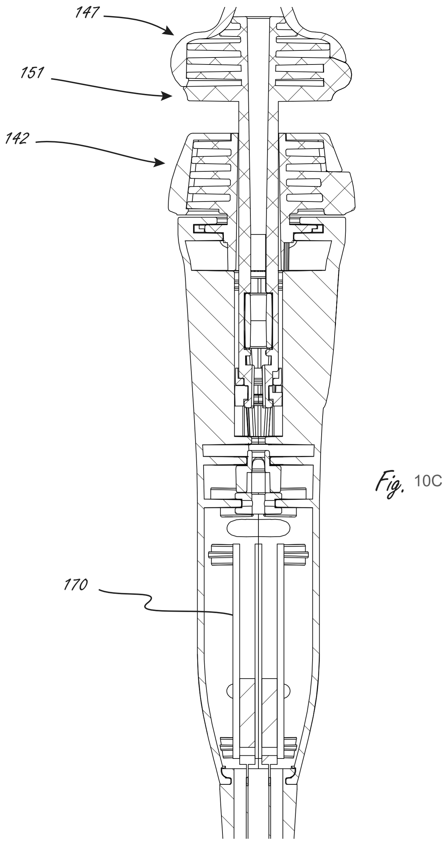

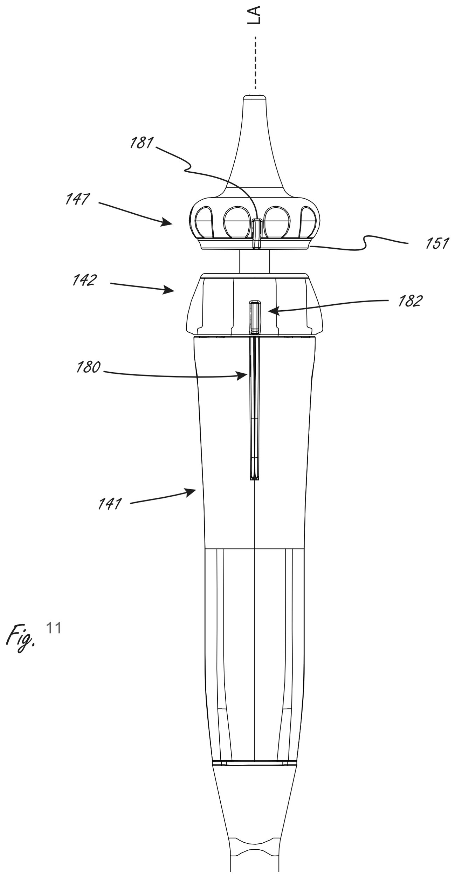

Figures 10A-10C and figure 11 illustrate an additional example handle assembly that can be in operable communication with any of the medical devices, including ultrasound probes, herein. For example, the example handle assembly shown in figures 10A-10C can be coupled to (directly or indirectly) and in operable communication with medical apparatus 130 shown in figures 9A-9D. In a particular embodiment, both elongate outer shaft 131 and elongate inner shaft 132 are coupled to and in operable communication with the handle assembly shown in figures 10A-10C.

The handle assembly in figures 10A-10C and figure 11 has some similarities to the handle assemblies, the individual components, and subassemblies that are shown in figures 8A and 8BB. Unless indicated to the contrary, concepts, features and methods of use from figures 8A and 8B that can be incorporated into the handle assembly in figures 10A-C are hereby incorporated by reference for all purposes into the disclosure of the handle assembly shown in, and described with respect to, figures 10A-C. Similarly, concepts, features and methods of use that are shown in, and described with respect to, figures 10A-C that can be incorporated into other handle assemblies herein are hereby incorporated by reference for all purposes into the disclosure of any of the handle assemblies set forth herein.

Figure 10A is side view of handle assembly 140 with a portion of handle body 141 removed so that some internal components of the handle assembly can be seen. Handle assembly 140 includes first actuator 143 and second actuator 142, and in this embodiment first actuator 143 is distal to second actuator 142. First actuator 143 can be both moved axially and rotated relative to the handle body and relative to a second actuator (in this embodiment actuator 142). First actuator 143 is in operable communication with an outer elongate body, such as outer shaft *131 (see* figure 9B). Axial movement of actuator 143 (distally or proximally) causes axial movement of the outer shaft 131, while rotation of actuator 143 causes rotation of the outer shaft. Second actuator 142 is in operable communication with an inner shaft, such as inner shaft 132 (figure 9A). Actuation of second actuator 142, in this embodiment rotation, causes deflection of the inner shaft. In this embodiment a rotatable and axially movable actuator (i.e., first actuator 143) is in operable communication with an outer shaft. Although reference is made that actuation of second actuator 142 may cause deflection of the inner shaft, alternatively, actuation of second actuator 142 may cause rotation of the inner shaft. Likewise, actuation of actuator 143 may alternatively cause deflection of the outer shaft.

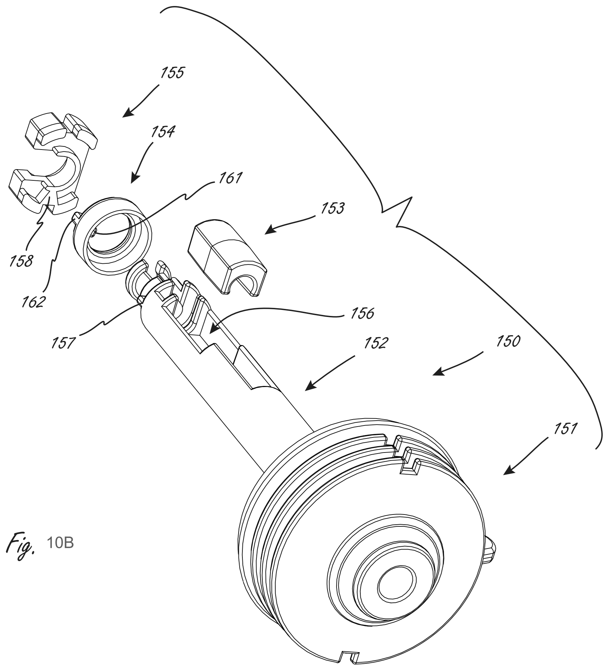

First actuator 143 is coupled to elongate outer shaft movement assembly 150 shown in the exploded view in figure 10B, such that movement of first actuator 143 causes movement of assembly 150. Elongate outer shaft movement assembly 150 is similarly coupled to the elongate outer shaft so that movement of first actuator also causes movement of the elongate outer shaft. In this embodiment, the outer elongate shaft is attached to removable part 153 after it is inserted into channel 156. Removable part 153 and channel 156 are configured so that removable part 153 is constrained by at least one inner surface of channel 156 when it is inserted therein. Elongate outer shaft movement assembly 150 also includes a distal head portion 151 that is secured to first actuator. Elongate outer shaft movement assembly 150 also includes a rotation limiting mechanism similar to that which is described herein, which limits the rotation of first actuator 143, and thereby limits the rotation of the outer elongate shaft. Any of the disclosure above related to rotation limiting subassemblies, functionality, and use, is incorporated into this embodiment for all purposes and may be incorporated into this and similar designs. During rotation, part 157 (see fig. 10B) interacts with part 161, and part 162 interacts with part 158. The physical interactions of these two sets of parts limits rotation to the desired rotation limit, e.g., such as limiting rotation up to 630 degrees of rotation of the outer body (in other embodiments the allowed rotation could be more than 630 degrees, such as up to and including 720 degrees).

If it is desired to clean the outer shaft, for example after use, removable part 153 can be detached from the outer shaft to allow the outer shaft to be removed from the handle assembly and cleaned, before being reinserted and reattached to removable part 153 or a new removable part if part 153 is damaged or broken.

Handle assembly 140 also includes inner shaft deflection assembly 146, which is in operable communication with second actuator 142. Inner shaft deflection assembly 146 includes central gear 147 adapted and configured to rotate when second actuator 142 is rotated. Central gear 147 interfaces first spindle 148 and second spindle 149 via a geared interface, such that rotation of central gear 147 causes rotation of the spindles in the opposite direction. The inner shaft deflection assembly 146, including the spindles, extends further proximally than the elongate outer body movement assembly 150. The inner shaft extends through the outer shaft and extends further proximally than the outer shaft within handle assembly 150. This allows one or more pull wires that are part of the inner shaft to extend radially outward and interface with reels 160.

Although the central gear 147 and the geared interfaces are shown with spur gears, in other embodiments helical gears may be used. Helical gears may provide for smoother control and reduced slop of the first actuator 143 and the second actuator 142. The helical gear design may provide smoother operating performance than spur gears. The helical gears may have a larger number of "virtual teeth" when compared to physical teeth. The helical gears may have the same number of "virtual teeth" as spur gears have physical teeth and "virtual teeth", while having less physical teeth than the spur gears, allowing the device to have the same or better functionality while using a smaller area. The helical gears may have stronger teeth, as measured by bending and surface fatigue, when compared to spur gears with the same number of physical teeth. The helical gears may reduce undercutting, allowing for fewer physical teeth when compared to a minimum number of physical teeth needed for spur gears. An example helical gear configuration may comprise twenty-six (26) gear teeth, seventeen (17) pinion teeth, a twenty-six to seventeen (26:17) gear ratio, a twenty degree (20°) pressure angle, and a twenty degree (20°) helix angle. An example helical gear configuration may comprise one point zero degree (1.0°) gears meshed to align, allowing for easy manufacturability. The helical gears may be molded. The helical gears may comprise a configuration similar to spur gears. The helical gear configuration may comprise spindles, such as the first spindle 148 and the second spindle 149, comprising thru holes, as spindles would have in a spur gear configuration.

The lack of interaction between elongate outer shaft movement assembly 150 and elongate inner shaft movement assembly 146 allows for the inner and outer elongate shaft to be independently controlled by first actuator 143 and second actuator 142.

Handle assembly 140 also includes printed circuit board ("PCB") 170 disposed within handle body 141, the PCB being in electrical communication with a cable bundle, such as flexible cable bundle 105 in figure 53, or any of the cable bundles herein that are in communication with the medical tool, such as an ultrasound transducer.

Handle assembly 140 also includes a rotation indicator 180 that can be used to show a user the extent to which at least one of the first actuator and the second actuator are rotated relative to a home, or neutral position. First actuator 143 can include a rotation indicator 181 that is aligned along an axis with rotation indicator 180 when first actuator 143 is in a neutral position, as shown in figure 11. When first actuator 143 is rotated, rotation indicator 181 is rotated relative to the axis along which rotation indicator 180 extends, which enables the user to visually understand that first actuator 143, and thus the outer shaft, is rotated to some extend relative to the neutral position. Similarly, second actuator 142 can also have rotation indicator 182 that is aligned along an axis with rotation indicator 180 when second actuator 142 is in a neutral position, as shown in figure 11. When second actuator 143 is rotated, rotation indicator 182 is rotated relative to the axis along which rotation indicator 180 extends, which enables the user to visually understand that second actuator 143, and thus the inner shaft, is deflected to some extent relative to its neutral position.

In some alternative embodiments, the handle assembly can include one or more sensors to track how much rotation has occurred for the outer shaft, or how much deflection has occurred in the inner shaft. In some embodiments the handle assembly can include an encoder for each actuator.

In any of the embodiments herein that include an outer shaft and an inner shaft, the device can include one or more lubricants between the inner and outer shafts to make it easier to move the inner and outer shafts relative to one another by reducing friction between the two. If the medical device needs to be cleaned for reuse, additional lubricant can be added between the inner and outer shafts after the cleaning process.

In some embodiments herein the medical device may include a flexible member, such as a flexible conductor bundle (which may be referred to herein as a conductor bundle, flex bundle or other similar derivative thereof), coupled to and extending from a distal region (e.g., probe tip) towards a proximal region of the medical device (see, for example, conductor bundle 2020 shown in example figures 4-6G; or bundle 105 from figure 9B). A probe tip may include an ultrasound transducer in electrical communication with a flexible conductor bundle. In some embodiments herein (e.g., figures 9A-11), the probe tip and conductor bundle can be axially displaced (proximally and/or distally) by actuation of a handle actuator (e.g., actuator 143 as shown in figure 10A). In some instances, the conductor bundle is disposed within an elongate member (e.g., steerable inner elongate body 132; or elongate member 131) and moves axially relative thereto when the probe tip is advanced distally or retracted proximally. When the distal region (e.g., ultrasound probe) and conductor bundle are retracted proximally (after being advanced distally), the conductor bundle may tend to fold up, bunch up, or otherwise bend, near or adjacent to its distal end due to friction between the bundle and, for example, the elongate member (e.g., steerable inner shaft 132) in which the conductor bundle is disposed. Bunching may occur if the medical device is in a straight configuration as well as if the medical device has some degree of bend (e.g., after being deflected from a straight or linear configuration).

To reduce the degree of, or even prevent completely, a tendency to bunch up or bend, any of the medical devices herein may include a structural tensioning member that is adapted and configured to apply or maintain tension on the flexible member, such as a flexible conductor bundle, at a location that is proximal to where the conductor bundle is coupled to the distal medical tool. By tensioning the flexible member, folding or bunching up of the flexible member can be minimized or even prevented. When used in this context, the "tensioning" member is adapted and configured to reduce distal region(s) of the flexible conductor from bunching (compared to a device without a tensioning member) by proximally moving at least a distal portion of the flexible conductor bundle as the distal probe is retracted proximally. In some embodiments, the structural tensioning member (e.g., a tensioning bar) may be physically secured to the flexible conductor bundle (e.g., direct or indirect attachment). In general, the tensioning members herein are in operable communication with the flexible members (e.g., a flex conductor bundle) such that movement or actuation of the tensioning member applies some force to the flexible member, and may cause movement (e.g., proximal) of the flexible member. In some example embodiments the structural tensioning member may be disposed in or carried by the handle assembly of the medical device. Structural tensioning member in this context may be a single component or an assembly of separate components.

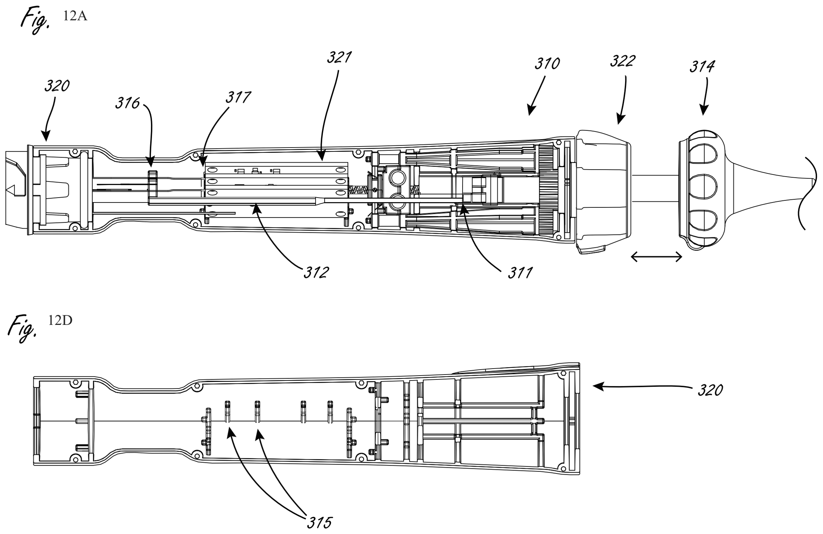

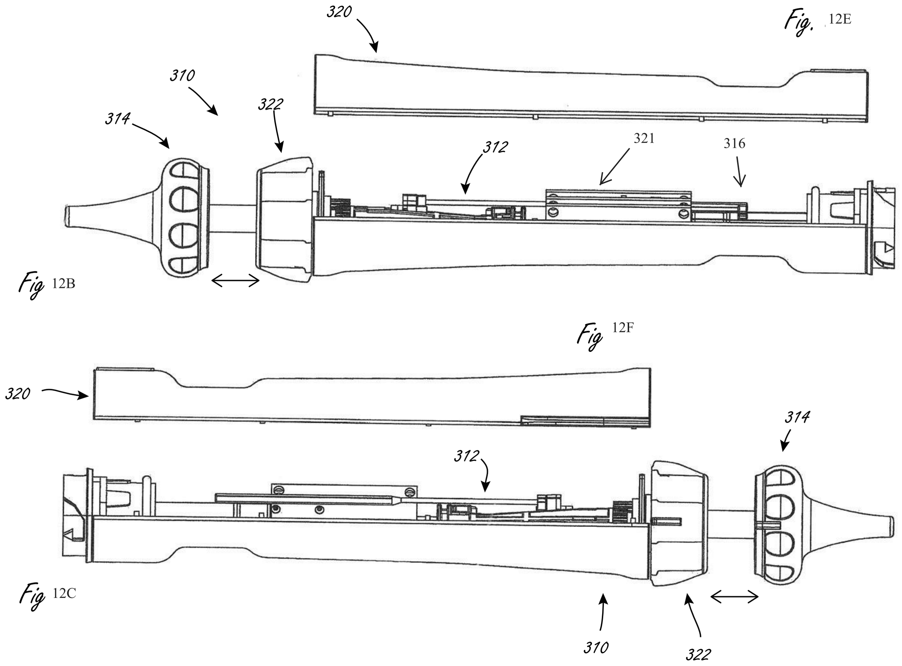

Figures 12A-12F illustrate a handle assembly portion of an example medical device, which may be incorporated into any suitable medical device herein. For example, the handle assembly in Figures 12A-12F may be part of a medical device that includes a medical tool (e.g., an ultrasound imaging probe) at its distal end or near its distal end, examples of which are described herein. Any other embodiment or feature herein is incorporated by reference into the example handle assembly shown in figures 12A-12F.

Example handle assembly 310 includes first actuator 314 and second actuator 322, where first actuator 314 is distal to proximal actuator 322. First actuator 314 is adapted and configured to be moved axially (distally and proximally) relative to second actuator 322 (and optionally also rotatable relative thereto), and may be in operable communication with an elongate body (e.g., 131 or 132) that may include a medical tool (e.g., 103) in a distal region. Handle assembly 310 (including actuators) may incorporate any of the relevant disclosure from any other handle assembly herein. The medical device of which handle assembly 310 is a part also includes a flexible member (e.g., flexible conductor bundle 105 in figure 9B) securely coupled to (directly or indirectly) the medical tool at a first distal location (e.g., as shown in figures 9B and 9C where medical tool 103 is secured to flexible member 105) and extends proximally from the medical tool towards handle assembly 310. The flexible member may be a flexible conductor bundle and can extend into handle assembly 310, as shown. A portion of the flexible member is disposed within an outer surface of the elongate body (e.g., within 131 and/or 132). The medical device also includes tensioning member that is secured to the flexible member at a second location 316, which is proximal to the first location ("first location" in this context may be referred to as a first distal location or derivative thereof). Figure 12A illustrates example tensioning member 312, while reference number 312 in figure 12A also points to an optional elongate rigid member (in this embodiment the elongate rigid member is linear and extending axially) of the tensioning member. Tensioning member 312 is adapted and configured to tension the flexible member as the medical tool is retracted proximally. This may be referred herein as applying a tensile force to the flexible member, or tensioning the flexible member, or maintaining tension in the flexible member. In this example embodiment, tensioning member 312 is adapted and configured to tension the flexible member as the medical tool is retracted proximally, which in this embodiment occurs when first actuator 314 is retracted proximally from its position shown in figures 12A, 12D, and 12F. The tensioning members herein can apply tension when the medical device is in a straight configuration and when the medical device is in a non-straight (linear) configuration, such as when the device may be deflected or bent.

In this embodiment, tensioning member 312 (which may include the tensioning bar shown in figure 12A) is secured to (e.g., attached directly) the flexible conductor bundle at location 316, the location of which is inside the handle assembly in this embodiment. In another embodiment, the tensioning member 312 may be secured to the flexible conductor bundle at an alternate location 317. A tensioning member may alternatively be secured to the flexible member at a location that is inside a handle or outside of the handle, such as inside one or both of outer and inner shafts. In this embodiment the tensioning member is also axially secured relative to first actuator 314, such that axial movement of first actuator 314 causes axial movement of tensioning member 312 (which may be in a 1:1 movement ratio). Because tensioning member 312 is also secured to the flexible member (e.g., at location 316), axially movement of tensioning member 312 also causes axial movement of the flexible member at location 316. By securing tensioning member 312 to the flexible member, the flexible member is tensioned distal to where the tensioning member is secured to the flexible member when first actuator 314 is retracted proximally, which prevents the flexible member from folding or bunching up at its distal region near or adjacent to the medical tool (or at least reduces the extent of folding/bunching up compared to a device without a tensioning member).

The flexible member may include a flexible conductor bundle, such as any of the flexible conductor bundles herein. In figures 12A-F, the tensioning member comprises a rigid elongate member (generally referred to as 312 in figure 12A) with a fixed length. The rigid tensioning member as shown has a general longitudinal axis, which in this embodiment is parallel with a longitudinal axis of the medical device and/or a longitudinal axis of the handle assembly. The rigid tensioning member may be made of a variety of materials, such as a rigid plastic member.

The tensioning members herein can ensure that the distance traveled by the medical tool is the same as the distance traveled by any point on the flexible member between the first and second locations. The tensioning members herein can ensure that the distance traveled by the medical tool is the same as the distance traveled by the location where the tensioning member is secured to the flexible member (e.g., location 316 in figure 12A).

The secured relationship between the tensioning member and the flexible member maintains the flexible member in a substantially flat or straight configuration between the first and second locations as the medical tool is retracted proximally (when the medical device is a straight configuration). A flat configuration in this context can include embodiments in which the flexible member may also be twisted (i.e., the flexible member can be flat and still be twisted, but not bunched/folded). A flattened configuration as used herein indicates a lack of a fold or bunching of the flexible member.

Medical devices in which a tensioning member is incorporated may also be steerable or deflectable. The tensioning members herein can be adapted and configured to apply a tensile force to the flexible member even if the medical device, including the flexible member, are in non-straight (e.g., deflected, steered, bent) configurations. When this disclosure refers to maintaining a substantially flat configuration in the flexible member, it refers to instances where the medical device may be in a straight configuration, which need not be the case, such as when a medical device has been steered, bent, or deflected.

The secured relationship between the tensioning members and the flexible members herein prevents the flexible member from forming a fold (i.e., bending, or bunching up) between the first and second locations as the medical tool is retracted proximally. Folding, bending, and bunching-up in this context includes a first region of the flexible member axially overlapping with a second region of the flexible member, and also includes general bending and bunching of the flexible member, such as regions of the flexible member that are not flat and, for example, form a bend, curved region, and/or meander back and forth.

The flexible member may have flat top and bottom surfaces (e.g., a conductor bundle with one or more flat surfaces), and optionally the tensioning member may be secured to at least one of the top and bottom surfaces. For example, figures 6A-6G illustrate a flexible member with flat or generally flat first and second surface (e.g., top and bottom surfaces), and a tensioning member may be secured to one or both of the flat or generally flat surfaces (e.g., such as at location 316). They may be secured using a variety of techniques, such as using adhesive, welding, or other bonding techniques.

The tensioning member may be in operative communication (directly or indirectly) with a handle actuator such that axial movement of the actuator axially moves the tensioning member. The handle actuator may be further adapted and configured to be rotated (e.g., actuator 314) to cause rotation of the medical tool, and optionally wherein rotation of the actuator does not cause rotation of the tensioning member. The tensioning member can thus be adapted to be moved axially when the actuator is moved axially, but not to rotate when the actuator is rotated. This can be accomplished by the manner in which the tensioning member is operatively in communication (directly or indirectly) with the actuator.

The medical device may include an inner elongate body (e.g., 132) including a lumen in which at least a portion of the flexible member is disposed. An inner elongate body may be independently steerable, such as with a separate independently actuatable handle actuator (e.g., actuator 322). Aspects of other embodiments herein in which the medical device includes an inner member and outer member, the inner member independently controllable (e.g., axially and rotationally) are fully incorporated in any of the embodiments herein.

The flexible member may be coupled to a printed circuit board (e.g., 321 "boards" as shown in figure 12A) in the handle assembly. The tensioning member may be coupled to a flexible member proximal to a printed circuit board. In alternative embodiments the tensioning member may be coupled to a flexible member distal to a printed circuit board.

Figures 12A-12F is an example of a portion of a medical device that includes an elongate outer body (e.g., 131) including a probe tip in a distal region of the elongate body; a flexible conductor bundle (e.g., 105) securely coupled to the probe tip at a first location (shown in figure figures 9B and 9C) and extending proximally from the medical tool and into a handle assembly, the flexible member disposed within an outer surface of the elongate body; an inner elongate body (e.g., 131), at least a portion of which is disposed within the elongate outer body, the inner elongate body optionally steerable, wherein at least a portion of the flexible conductor bundle is disposed within the inner elongate body and configured to be axially movable relative to the inner elongate body; a tensioning member secured to the flexible member at a second location in the handle assembly, the tensioning member adapted and configured to apply tension on the flexible member as the probe tip is retracted proximally. Probe tips as used herein may include one or more ultrasound transducers.

Figures 12C and 12E illustrate a half of a handle outer shell 320, two of which form a portion of the outer surfaces of the handle assembly 310. Handle shells 320 include radially inwardly extending features 315 that are adapted to interface with a control the movement of the tensioning member 312. Features 315 can include guides that are configured to interface with the tensioning member at one or more locations and help stabilize the tensioning member.

Any other handle assembly component in any other embodiment herein that can be suitably integrated into handle assembly 310 is incorporated by reference herein.

In any of the embodiments and claims herein, the phrase "tensioning member" may be replaced with "straightening member," "flattening member," or a derivative thereof. As described herein, a straightening member or flattening member refers to maintaining a substantially straight or flattened configuration in the flexible member when the medical device is in a straightened configuration, and does not require that the device always has a straightened configuration. Thus, even if the flexible member is not necessarily being placed under tension, it may still be maintained in a straightened (i.e., not folded configuration) along at least a portion of its length due to a straightening member. Member 312 in figure 12A, for example, is an example of a straightening member, even if it also functions as a tensioning member. This applies to all tensioning members described, shown, and claimed herein. In some embodiments herein, the "member" can be a straightening member (or flattening member) and can also function as a tensioning member. Additionally, the phrase "tensioning member" herein may be replaced with "fold-prevention member," "bend-prevention member," "bunching-prevention member," or a derivative thereof.

The disclosure above describes that in some embodiments the flexible member, such as conductor bundle 2010, may be twisted along a portion of its length. For example, a conductor bundle may be twisted to provide a more balanced cross-section along a portion of the length of the medical device. A conductor bundle may be twisted only in a portion of the medical device that will experience deflection.

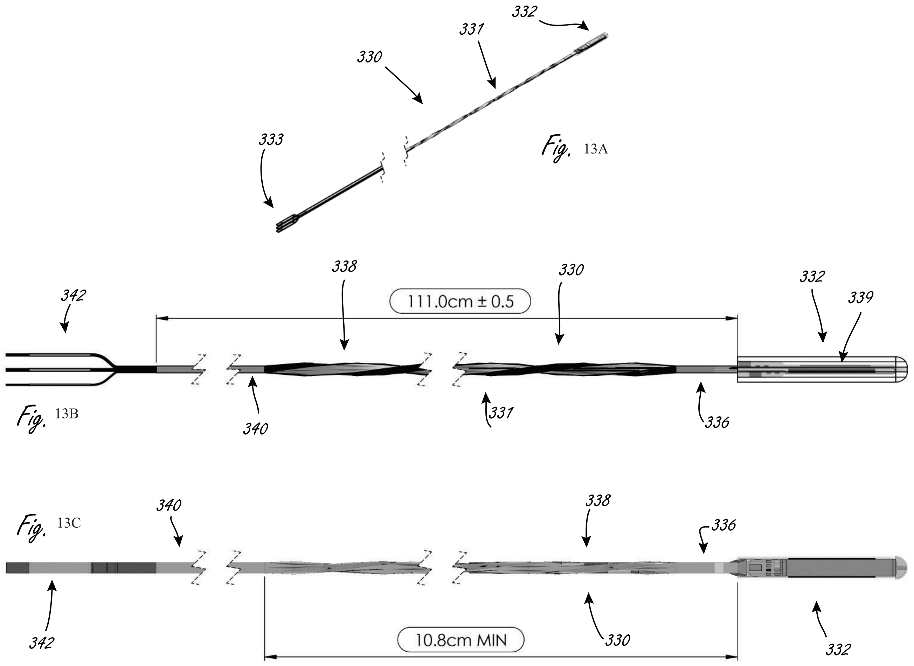

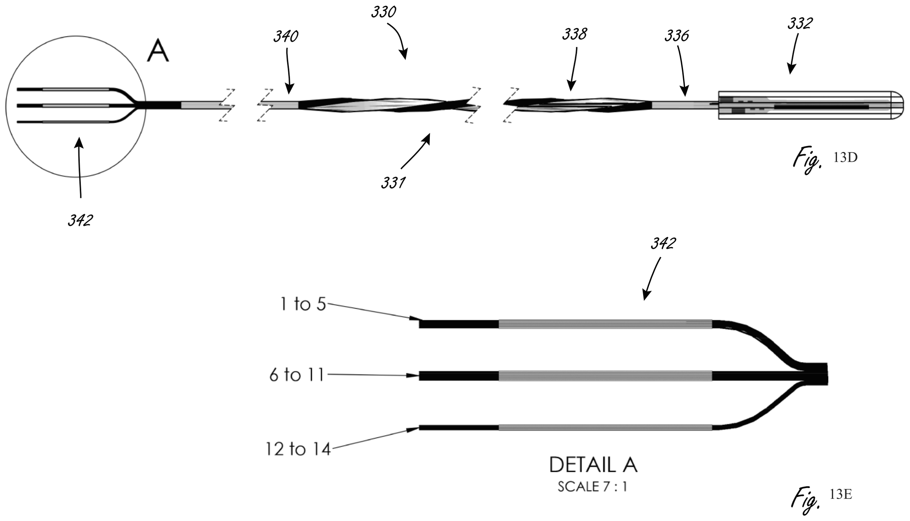

Figures 13A-13E illustrate a portion of an example medical device that includes a twisted flexible member, such as a flexible conductor bundle. The embodiment in figures 13A-E may be incorporated with any other suitable feature and/or medical device described herein.

The portion 330 of the medical device shown in figures 13A-E includes a medical tool 332 at a distal region, a flexible member 331 coupled thereto and extending proximally therefrom, and a proximal end region 333 comprising a plurality of electrical connectors. Flexible member 331 may be a flexible conductor bundle, such as any of the bundles herein. Medical tool 332 may include an ultrasound imaging transducer 339. Flexible conductor bundle 331 has a region 338 in which the conductor bundle is twisted, the twisted region 338 having a distal end and a proximal end. Flexible conductor bundle 331 also includes a region 336 distal to twisted region 334 that is not twisted, and a region 340 proximal to twisted region 336 that is not twisted. Medical tool 332 may be coupled to an outer shaft, such as outer shaft 131 shown in figure 9B.

The length of twisted region 338 from its distal end to its proximal end can vary, and in some embodiments is from 5-15 cm, such as from 8-15 cm, such as 11 cm. The length over which a complete turn is formed can vary well, such as from 1-5 cm, such as 3 cm.

The number of twists over the length of the twisted region can vary as well, such as, for example without limitation, 7-9 full twists.

An example manner in which to form the twisted region of the flexible member (e.g., flexible conductor bundle) is to couple the flexible member to the medical tool at the distal end (e.g., probe tip). A thin section of PET heat shrink may then be advanced over the flexible conductor bundle. A portion of the device can be held in place while another portion is twisted to the desired number of turns to form the twisted region. While the twisted configuration is maintained, the PET can be heat shrunk over the twisted bundle region. Additional layers of PET may be subsequently added. An elongate member (e.g., shaft 131) may then be placed over the bundle, including the twisted region, and the elongate member can be bonded to the medical tool.

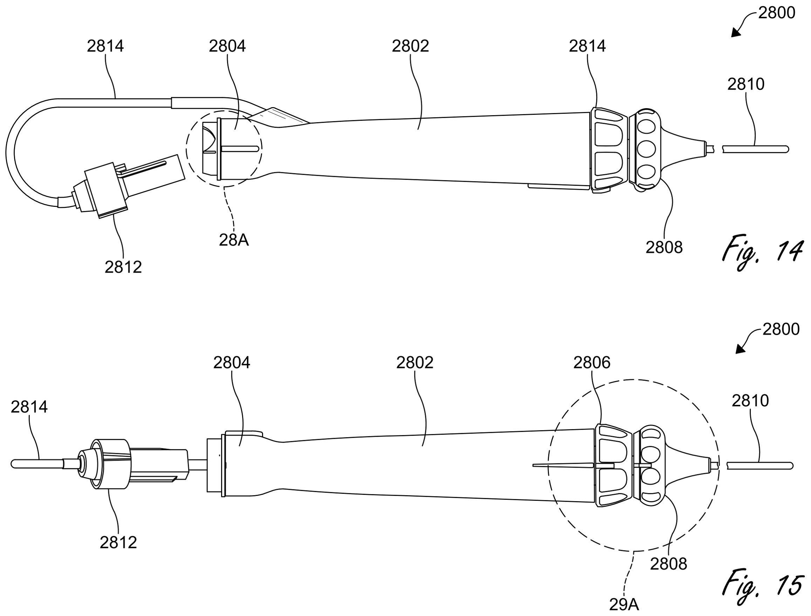

A Referring to FIGS. 14-18, a system 2800 includes a handle assembly 2802 and steering and medical device portion 2810 (e.g., shown as an outer sheath similar to sheath 1208 (FIG. 4), which may further enclose an inner shaft and tool. The system is adapted so that handle assembly 2802 can be actuated to cause steering of a steerable portion of the steering and medical device portion 2810, and optionally can be further actuated to cause movement of a medical device coupled thereto. As an illustrative example, the handle assembly 2802 includes a first actuator 2806 and a second actuator 2808. One or more of the first actuator 2806 or the second actuator 2808 is adapted (e.g., configured) to be actuated (in this example rotated) relative to a handle body of the handle assembly 2802 to cause the steering of steerable portion 2810, such as steering an outer sheath. Steering may include rotating or deflecting. As an example, one of the actuators 2806, 2808 controls rotation, while another of the actuators 2806, 2808 controls deflection. As a further example, one of the actuators 2806, 2808 controls rotation of one or more of the inner shaft or outer shaft, while another of the actuators 2806, 2808 controls deflection of one or more of the inner shaft or the outer shaft.

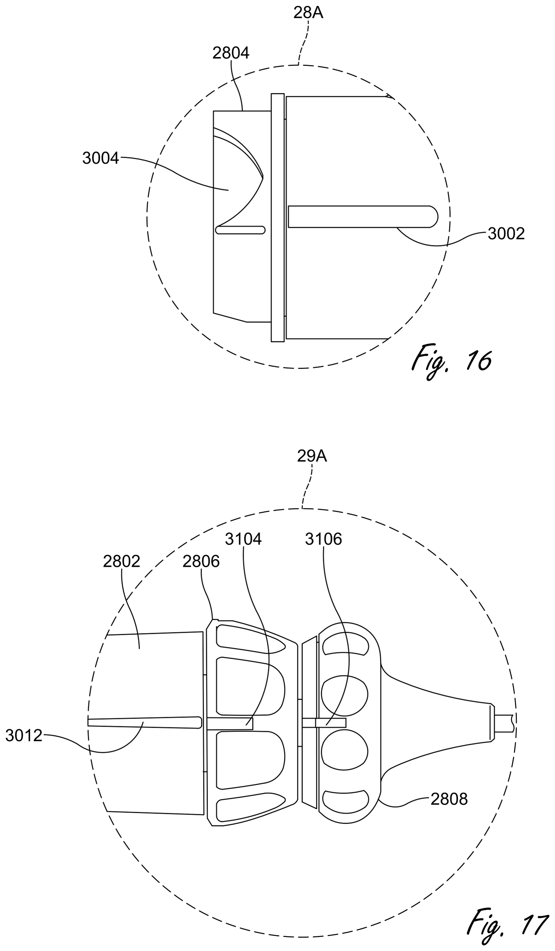

As more clearly shown in FIG. 17, a neutral position marker 3102 may be disposed on a body of the handle assembly 2802 to indicate (e.g., visually, tactilely, etc.) a fixed reference point. One or more of the actuators 2806, 2808 may further include a marker 3104, 3106 to indicate alignment or position of the actuators 2806, 2808 relative to the neutral position marker 3102. As an example, the marker 3104 may indicate a deflection of the shaft relative to the neutral position marker 3102 and the marker 3106 may indicate a rotation of a component (e.g., transducer face disposed in the sheath 2810) relative to the neutral position marker 3102, or vice versa. The markers 3104, 3106 may indicate other positions. Additionally or alternatively, mechanical registration mechanisms, such as detents, may be used to provide tactile feedback to a user to indicate certain positions of the actuators 2806, 2808, such as when the actuators 2806, 2808 are in the neutral position.

The handle assembly 2802 may include any other handle component or functionality described in any of the other handles herein. For example, a receptacle 2804 may be disposed at a proximal end of the handle assembly 2802 and adapted to receive a plug such as an umbilical plug. As more clearly shown in FIG. 16, the receptacle 2804 may comprise visual and/or tactile orientation markers for registration and alignment of an umbilical plug and the receptacle 2804.

A navigation connector 2812 may be coupled to the handle assembly 2802 via a navigation conduit 2814 (e.g., navigation cable sleeve). The navigation connector 2812 may be configured to interface with a navigation system such that the system 2800 may communicate with the navigation system. As an example, the navigation system may provide tracking and navigation of a portion of the system (e.g., the medical device) while in use. As shown, for example, the navigation conduit 2814 may couple to a body of the handle assembly 2802 to provide access for one or more cables to pass from the navigation connector 2812 to components within the housing of the handle assembly 2802. As more clearly shown in FIG 18, the navigation connector 2812 may be configured to interface with a navigation system connector 3204 (e.g., receptacle), which may be proprietary to the navigation system manufacturer. Other configurations may be used.

FIGS. 19-24 illustrate an example distal region of a steerable system that includes a medical tool. As an illustrative example, FIG. 19 shows the medical tool may comprise a steerable tip 3300 disposed at the distal end of a sheath. The steerable tip 3300 may include a transducer 3304 (e.g., ultrasound transducer) at least partially enclosed by a material 3302 (e.g., polymer). The tip 3300 may comprise an electronic module 3306, which may include sensors, control boards, thermistors, and the like. As an example, a TAS sensor 3308 may be disposed in communication with the module 3306. As a further example, a sensor cable bundle 3310 may be disposed to provide electrical communication to one or more components coupled to the module 3306.

As an illustrative example, FIG. 20 shows the medical tool may comprise a steerable tip 3400 disposed at the distal end of a sheath. The steerable tip 3400 may include a transducer 3404 (e.g., ultrasound transducer, folded transducer) at least partially enclosed by a material 3402 (e.g., polymer). The tip 3400 may include sensors, control boards, thermistors, and the like. As an example, a TAS sensor 3406 may be disposed in in a configuration that is closer to the distal end of the tip 3400, when compared to the tip 3300 configuration in FIG. 19. By shifting the sensor 3406 the proportion of the tip 3400 that may be deflected is increased.

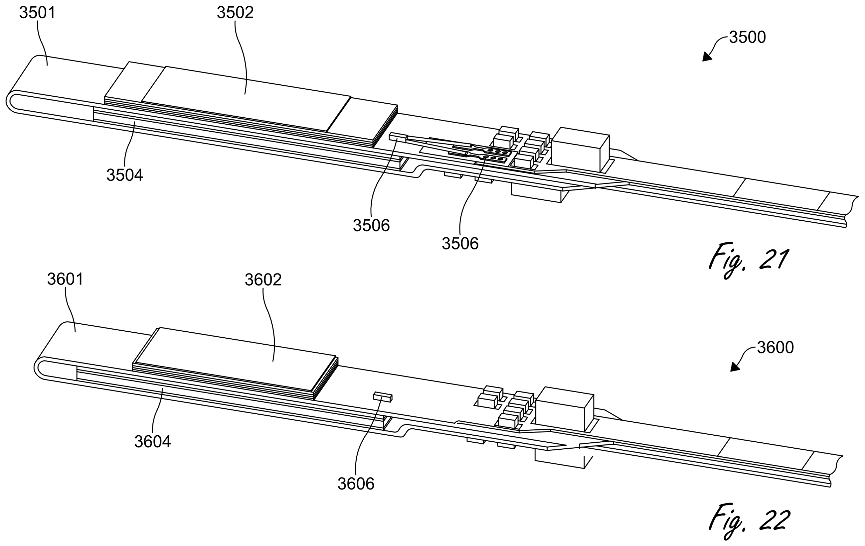

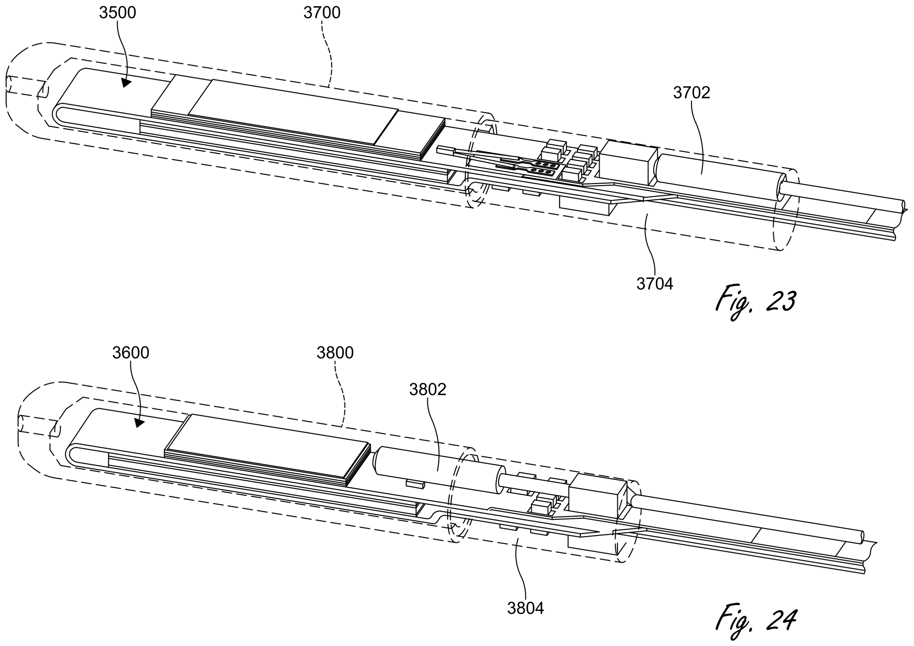

As an illustrative example, FIG. 21 shows the medical tool may comprise a steerable tip 3500 disposed at the distal end of a sheath. The steerable tip 3500 may include a transducer 3502 (e.g., ultrasound transducer, folded transducer). As an example, an ASIC 3504 may be disposed adjacent the transducer 3502. As a further example, inactive piezo elements may be disposed adjacent the transducer 3502. The tip 3500 may include various sensors, control boards, thermistors, and the like. As shown, the tip 3500 includes thermistors 3506 disposed proximal to the transducer 3502. As more clearly shown in FIG. 23, at least a portion of the tip 3500 may be at least partially enclosed by a first material 3700 (e.g., polymer) and at least a portion of the tip 3500 may be at least partially enclosed by a second material 3704. The first and second materials 3700, 3704 may be configured with the same or different characteristics such as differing stiffness (e.g., durometer). As an illustrative example, the first material 3700 may have a higher durometer than the second material 3704. As further illustrated in FIG. 23, a navigation sensor 3702 may be disposed at or adjacent a proximal end of the tip 3500. The navigation sensor 3702 may be in communication with a navigation system to provide tracking and position of the tip 3500 and to provide feedback to a user.

As an illustrative example, FIG. 22 shows the medical tool may comprise a steerable tip 3600 disposed at the distal end of a sheath. The steerable tip 3600 may include a transducer 3602 (e.g., ultrasound transducer, folded transducer). When compared to tip 3500, the tip 3600 may include a shortened folded end 3601. Inactive piezo material may be minimized adjacent the transducer 3602. As an example, an ASIC 3604 may be disposed adjacent the transducer 3602. The tip 3600 may include various sensors, control boards, thermistors, and the like. As shown, the tip 3600 includes thermistors 3606 disposed proximal to the transducer 3602. Circuits such as flex circuits may be in electrical communication with one or more of the components in the tip 3600. As an example, the flex circuit may be divided into layers and selectively in communication with the components. As more clearly shown in FIG. 24, at least a portion of the tip 3600 may be at least partially enclosed by a first material 3800 (e.g., polymer) and at least a portion of the tip 3600 may be at least partially enclosed by a second material 3804. The first and second materials 3800, 3804 may be configured with the same or different characteristics such as differing stiffness (e.g., durometer). As an illustrative example, the first material 3800 may have a higher durometer than the second material 3804. As further illustrated in FIG. 24, a navigation sensor 3802 may be disposed adjacent the transducer 3602. The navigation sensor 3802 may be in communication with a navigation system to provide tracking and position of the tip 3600 and to provide feedback to a user.

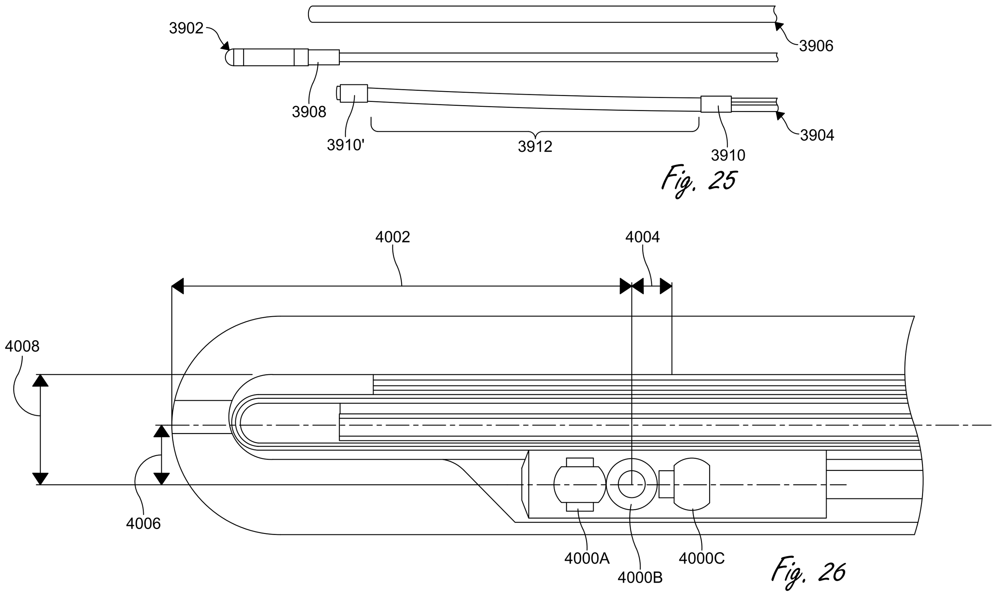

FIG. 25 illustrates a medical tool that may comprise a steerable tip 3902 disposed at the distal end of a sheath 3906. As shown, a DAS sensor 3908 may be disposed at or adjacent a proximal end of the tip 3902. An inner sheath 3904 may comprise a section configured for deflection 3912 and may be disposed adjacent the tip 3902 and within the sheath 3906. One or more DAS sensors 3910, 3910' may be disposed along a length of the sheath 3904.

FIG. 26 illustrates example configurations of navigation transducers 4002. For example, from a tip of the device to a center of a y-coil may be measured at a first length 4003. From the center of an active transducer 4002 to the y-coil may be a second length 4004. From a sensor axis to a tip axis may be a third length 4006. From a sensor axis to a face of an ultrasound transducer may be a fourth length 4008.

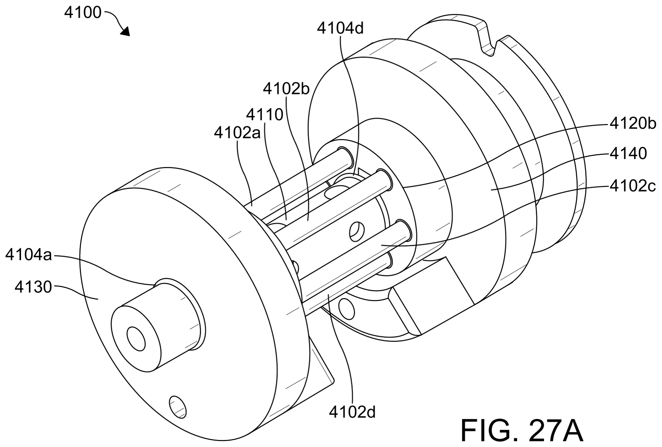

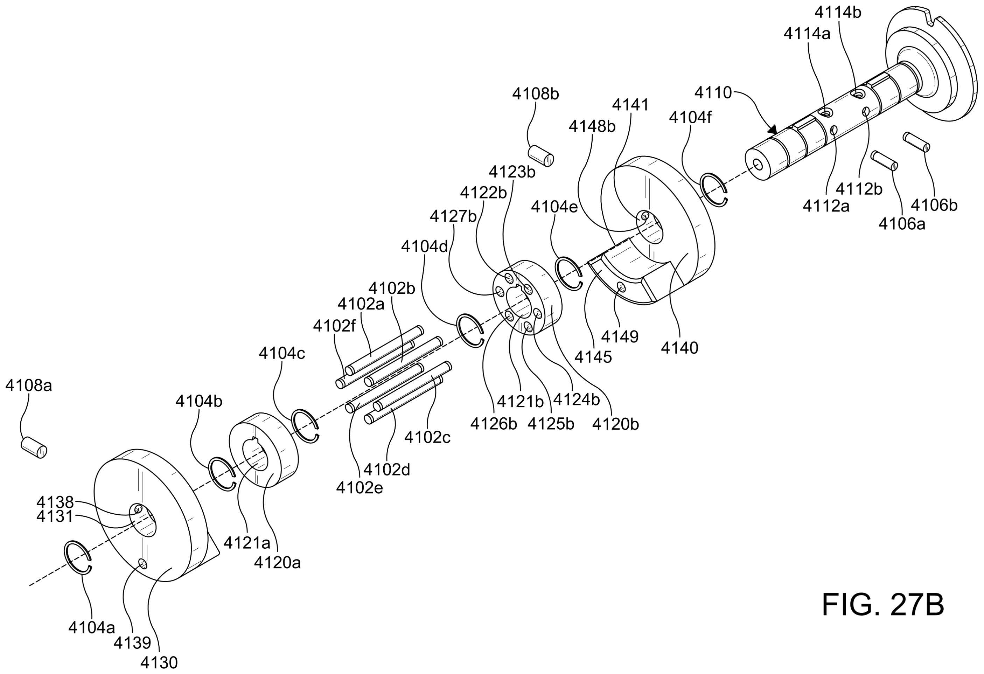

Figure 27A illustrates an example assembly 4100. The assembly 4100 may be disposed in a handle assembly of a catheter, such as the handle assemblies described herein. The assembly 4100 may comprise a plurality of pins 4102a, 4102b, 4102c, 4102d, 4102e (shown in Figure 27B), and 4102f (shown in Figure 27B). The assembly 4100 may comprise a plurality of retaining rings 4104a, 4104b (shown in Figure 27B), 4104c (shown in Figure 27B), 4104d, 4104e (shown in Figure 27B), and 4104f (shown in Figure 27B). The assembly 4100 may comprise a shaft 4110. The assembly 4100 may comprise a plurality of pin carriers 4120a and 4120b. The assembly 4100 may comprise an anterior and posterior (A/P) knob 4130. The assembly 4100 may comprise a left and right (L/R) knob 4140. The assembly 4100 and components will be described in more detail below. The assembly 4100 may allow for 4-way steering of a catheter tip. As an example, one or more features (detent features) may be configured for a home (e.g., start, initial, original, etc.) position. The assembly 4100 may be and/or comprise a cartridge.

Figure 27B illustrates an exploded view of the assembly 4100 of Figure 27A. The pins 4102a, 4102b, 4102c, 4102d, 4102e, and 4102f may comprise a cylindrical shape. The pins 4102a, 4102b, 4102c, 4102d, 4102e, and 4102f may be dowels or other component to anchor the pull lines in an adjustable fashion. The pins 4102a, 4102b, 4102c, 4102d, 4102e, and 4102f may be configured to be partially disposed in a first pin carrier 4120a of the plurality of pin carriers and partially disposed in a second pin carrier 4120b of the plurality of pin carriers. The plurality of pins 4102a, 4102b, 4102c, 4102d, 4102e, and 4102f may be configured to apply tension to one or more pull lines.

The retaining rings 4104a, 4104b, 4104c, 4104d, 4104e, and 4104f may comprise a ring body and at least partially enclosed aperture formed in the ring body and configured to receive the shaft 4110. The retaining rings 4104a, 4104b, 4104c, 4104d, 4104e, and 4104f may be configured to couple one or more components to the shaft 4110 (e.g., retain one or more components in a spatial relationship with the shaft 4110) as described in more detail below. A first retaining ring 4104a may be configured to couple the A/P knob 4130 to the shaft 4110. A second retaining ring 4104b may be configured to couple the A/P knob 4130 and/or the first pin carrier 4120a to the shaft 4110. A third retaining ring 4104c may be configured to couple the first pin carrier 4120a to the shaft 4110. A fourth retaining ring 4104d may be configured to couple the second pin carrier 4120b to the shaft 4110. A fifth retaining ring 4104e may be configured to couple the second pin carrier 4120b and/or the L/R knob 4140 to the shaft 4110. A sixth retaining ring 4104f may be configured to couple the L/R knob 4140 to the shaft 4110. As described herein, coupling of one or more components to the shaft 4110 may comprise retaining the one or more components at or adjacent a potion along the shaft 4110.

The shaft 4110 may comprise a plurality of apertures, such as a first dowel pin hole 4112a and a second dowel pin hole 4112b, configured to receive dowel pins, such as a first dowel pin 4106a and a second dowel pin 4106b. The shaft 4110 may comprise a plurality of apertures, such as a first pull line hole 4114a and a second pull line hole 4114b, configured to allow traversal of a pull line. The dowel pins may be configured to guide pull lines through the pull line holes. For example, the first dowel pin 4106a may be configured to guide one or more pulls lines through the first pull line hole 4114a, and the second dowel pin 4106b may be configured to guide one or more pulls lines through the second pull line hole 4114b.

The pin carriers 4120, such as the first pin carrier 4120a and the second pin carrier 4120b, may comprise a carrier body and a carrier aperture 4121, such as a first carrier aperture 4121a and a second carrier aperture 4121b, formed in the carrier body configured to receive the shaft 4110. The pin carriers 4120 may comprise a plurality of receptacles, such as a first pin receptacle 4122b and a second pin receptacle 4123b.

The knobs, such as the A/P knob 4130 and the L/R knob 4140, may comprise a main body and an aperture, such as aperture 4131 and aperture 4141, formed in the main body configured to receive the shaft 4110. The knobs may be configured to control deflection of a catheter tip. For example, the A/P knob 4130 may be configured to control anterior and/or posterior deflection of the catheter tip. As another example, the L/R knob 4140 may be configured to control left and/or right deflection of the catheter tip. The knobs may comprise a protruding area, such as protruding area 4145, configured to allow an associated pin carrier 4120 to reside thereon. A pull line may be coupled to (e.g., anchored, tied, attached, etc.) the protruding area, such as the protruding area 4145.

The knobs may comprise one or more selective retaining features such as ball and detent feature. Other detents or selective retaining features may be used. As an example, ball detent 4138 and ball detent 4148 may be configured to receive a retaining element such as a ball. such as ball detent 4108a and ball detent 4108b. The ball detent apertures 4138 and 4148 may be configured such that associated ball detents 4108a and 4108b are configured to reside in the ball detent apertures 4138 and 4148. A ball detent, such as the ball detents 4108a and 4108b, may couple with the shaft 4110 to lock a knob into a home (e.g., start, initial, original, etc.) position. The ball detent apertures 4138 and 4148 may comprise threading configured to receive the ball detents 4108a and 4108b. The knobs, such as the A/P knob 4130 and the L/R knob 4140, may comprise a pull line anchor aperture, such as pull line anchor aperture 4139 and pull line anchor aperture 4149. The pull line anchor apertures, such as the pull line anchor aperture 4139 and the pull line anchor aperture 4149, may be threaded. The pull line anchor apertures, such as the pull line anchor aperture 4139 and the pull line anchor aperture 4149, may be configured to receive a removable fastener (e.g., anchor, attachment point, etc.). A pull line may be attached to a removable fastener coupled to a pull line anchor aperture, such as pull line anchor aperture 4139 and pull line anchor aperture 4149.

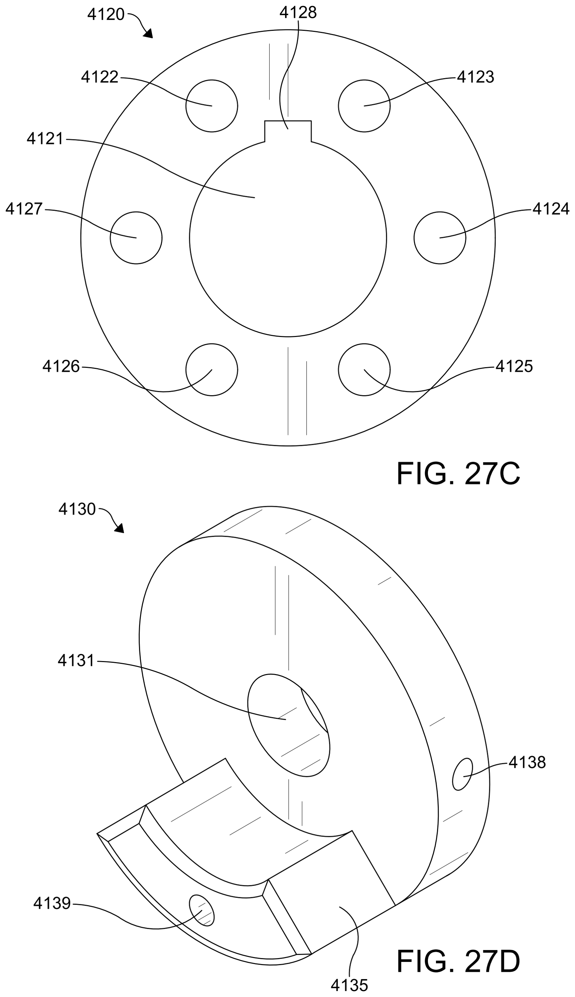

Figure 27C illustrates an example pin carrier 4120 configured to be disposed in the assembly 4100 of Figure 27A. The example pin carrier 4120 may comprise a first pin receptacle 4122, a second pin receptacle 4123, a third pin receptacle 4124, a fourth pin receptacle 4125, a fifth pin receptacle 4126, and a sixth pin receptacle 4127. A pin receptacle, such as the pin receptacles 4122-4127, may be configured such that a pin is at least partially disposed therein. A pin carrier 4120 may be configured such that the pin receptacles 4122-4127 are facing pin receptacles of an opposing pin carrier, such that a plurality of pins may be configured such that the pins are partially disposed in the pin receptacles 4122-4127 and partially disposed in the pin receptacles of the opposing pin carrier. At least one of the pins in the pin receptacles 4122-4127 may apply tension to one or more pull lines. The pin carrier 4120 may comprise a carrier aperture 4121. The carrier aperture 4121 may comprise a keyed cutout 4128. The keyed cutout 4128 may couple with a key of the shaft 4110, as described in more detail below.

Figure 27D illustrates an example knob 4130 configured to be disposed in the assembly 4100 of Figure 27A. Figure 27D shows the A/P knob 4130. The knob 4130 may comprise the aperture 4131, the protruding area 4135, the ball detent aperture 4138, and the pull line anchor aperture 4139 described above. The L/R knob 4140 may have similar features. The L/R knob 4140 may have mirrored features.

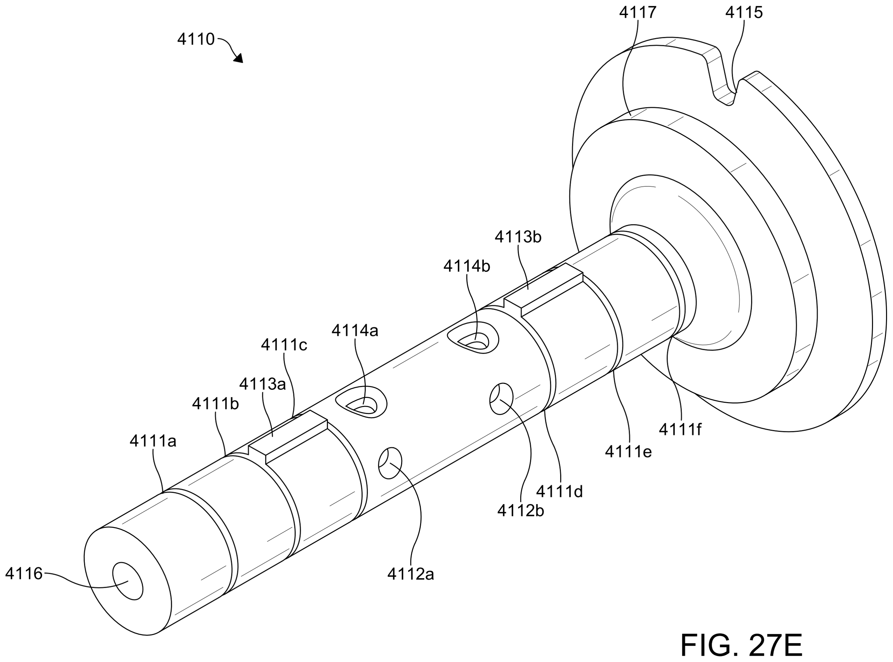

Figure 27E illustrates an example shaft 4110 configured to be disposed in the assembly 4100 of Figure 27A. The shaft 4110 may be elongate. The shaft 4110 may comprise a plurality of retaining ring grooves 4111a, 4111b, 4111c, 4111d, 4111e, and 4111f. The plurality of retaining ring grooves 4111a, 4111b, 4111c, 4111d, 4111e, and 4111f may be configured to couple with the retaining rings 4104a, 4104b, 4104c, 4104d, 4104e, and 4104f. The shaft 4110 may comprise the first dowel pin hole 4112a and the second dowel pin hole 4112b, configured to receive dowel pins, such as the first dowel pin 4106a and the second dowel pin 4106b. The shaft 4110 may comprise a plurality of keys, such as key 4113a and key 4113b, configured to couple with the keyed cutout 4128 of the pin carrier 4120, such as the first pin carrier 4120a and the second pin carrier 4120b. The shaft 4110 may comprise a lumen 4116 configured to house one or more pull lines. The one or more pull lines may exit and/or enter the lumen 4116 via the first pull line hole 4114a and/or the second pull line hole 4114b. The shaft 4110 may comprise a generally disc-shaped flange 4117 disposed adjacent a proximal end. The flange 4117 may comprise an anti-rotation feature 4115. The anti-rotation feature 4115 may comprise an indention on a rim of the flange 4117, configured to prevent the shaft 4110 from rotating.

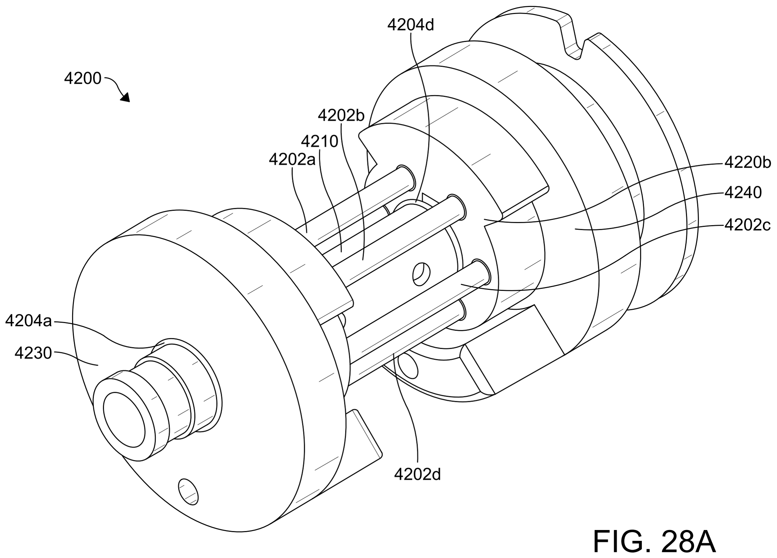

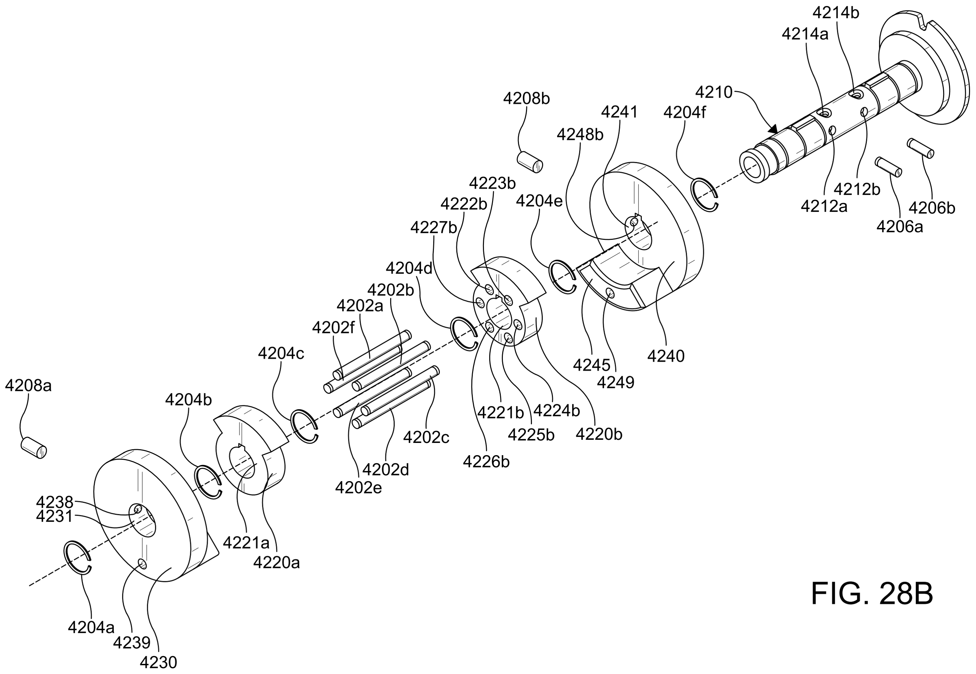

Figure 28A illustrates an example assembly 4200assembly 4200. The assembly 4200 may be disposed in a handle assembly of a catheter. The assembly 4200 may comprise a plurality of pins 4202a, 4202b, 4202c, 4202d, 4202e (shown in Figure 28B), and 4202f (shown in Figure 28B). The assembly 4200 may comprise a plurality of retaining rings 4204a, 4204b (shown in Figure 28B), 4204c (shown in Figure 28B), 4204d, 4204e (shown in Figure 28B), and 4204f (shown in Figure 28B). The assembly 4200 may comprise a shaft 4210. The assembly 4200 may comprise a plurality of pin carriers 4220a and 4220b. The assembly 4200 may comprise an anterior and posterior (A/P) knob 4230. The assembly 4200 may comprise a left and right (L/R) knob 4240. The assembly 4200 and components will be described in more detail below. The assembly 4200 may allow for 4-way steering of a catheter tip, independent tip rotation, and detent features for a home (e.g., start, initial, original, etc.) position. The assembly 4200 may be and/or comprise a cartridge.

Figure 28B illustrates an exploded view of the assembly 4200 of Figure 28A. The pins 4202a, 4202b, 4202c, 4202d, 4202e, and 4202f may comprise a cylindrical shape. The pins 4202a, 4202b, 4202c, 4202d, 4202e, and 4202f may be dowels. The pins 4202a, 4202b, 4202c, 4202d, 4202e, and 4202f may be configured to be partially disposed in a first pin carrier 4220a of the plurality of pin carriers and partially disposed in a second pin carrier 4220b of the plurality of pin carriers. The plurality of pins 4202a, 4202b, 4202c, 4202d, 4202e, and 4202f may be configured to apply tension to one or more pull lines.

The retaining rings 4204a, 4204b, 4204c, 4204d, 4204e, and 4204f may comprise a ring body and a mostly closed aperture formed in the ring body configured to receive the shaft 4210. The relationship between the retaining rings 4204a, 4204b, 4204c, 4204d, 4204e, and 4204f and the shaft will be described in more detail below. A first retaining ring 4204a may be configured to couple the A/P knob 4230 to the shaft 4210. A second retaining ring 4204b may be configured to couple the A/P knob 4230 and/or the first pin carrier 4220a to the shaft 4210. A third retaining ring 4204c may be configured to couple the first pin carrier 4220a to the shaft 4210. A fourth retaining ring 4204d may be configured to couple the second pin carrier 4220b to the shaft 4210. A fifth retaining ring 4204e may be configured to couple the second pin carrier 4220b and/or the L/R knob 4240 to the shaft 4210. A sixth retaining ring 4204f may be configured to couple the L/R knob 4240 to the shaft 4210.

The shaft 4210 may comprise a plurality of apertures, such as a first dowel pin hole 4212a and a second dowel pin hole 4212b, configured to receive dowel pins, such as a first dowel pin 4206a and a second dowel pin 4206b. The shaft 4210 may comprise a plurality of apertures, such as a first pull line hole 4214a and a second pull line hole 4214b, configured to allow traversal of a pull line. The dowel pins may be configured to guide pull lines through the pull line holes. For example, the first dowel pin 4206a may be configured to guide one or more pulls lines through the first pull line hole 4214a, and the second dowel pin 4206b may be configured to guide one or more pulls lines through the second pull line hole 4214b.

The pin carriers 4220, such as the first pin carrier 4220a and the second pin carrier 4220b, may comprise a carrier body and a carrier aperture 4221, such as a first carrier aperture 4221a and a second carrier aperture 4221b, formed in the carrier body configured to receive the shaft 4210. The pin carriers 4220 may comprise a plurality of receptacles, such as a first pin receptacle 4222b and a second pin receptacle 4223b. The pin carriers 4220 will be described in more detail below.

The knobs, such as the A/P knob 4230 and the L/R knob 4240, may comprise a main body and an aperture, such as aperture 4231 and aperture 4241, formed in the main body configured to receive the shaft 4210. The knobs may be configured to control deflection of a catheter tip. For example, the A/P knob 4230 may be configured to control anterior and/or posterior deflection of the catheter tip. As another example, the L/R knob 4240 may be configured to control left and/or right deflection of the catheter tip. The knobs may comprise a protruding area, such as protruding area 4245, configured to allow an associated pin carrier 4220 to reside thereon. A pull line may be coupled to (e.g., anchored, tied, attached, etc.) the protruding area, such as the protruding area 4245.

The knobs may comprise a ball detent aperture, such as ball detent aperture 4238 and ball detent aperture 4248, configured to receive ball detents, such as ball detent 4208a and ball detent 4208b. The ball detent apertures 4238 and 4248 may be configured such that associated ball detents 4208a and 4208b are configured to reside in the ball detent apertures 4238 and 4248. A ball detent, such as the ball detents 4208a and 4208b, may couple with the shaft 4210 to lock a knob into a home (e.g., start, initial, original, etc.) position. The ball detent apertures 4238 and 4248 may comprise threading configured to receive the ball detents 4208a and 4208b. The knobs, such as the A/P knob 4230 and the L/R knob 4240, may comprise a pull line anchor aperture, such as pull line anchor aperture 4239 and pull line anchor aperture 4249. The pull line anchor apertures, such as the pull line anchor aperture 4239 and the pull line anchor aperture 4249, may be threaded. The pull line anchor apertures, such as the pull line anchor aperture 4239 and the pull line anchor aperture 4249, may be configured to receive a removable fastener (e.g., anchor, attachment point, etc.). A pull line may be attached to a removable fastener coupled to a pull line anchor aperture, such as pull line anchor aperture 4239 and pull line anchor aperture 4249.

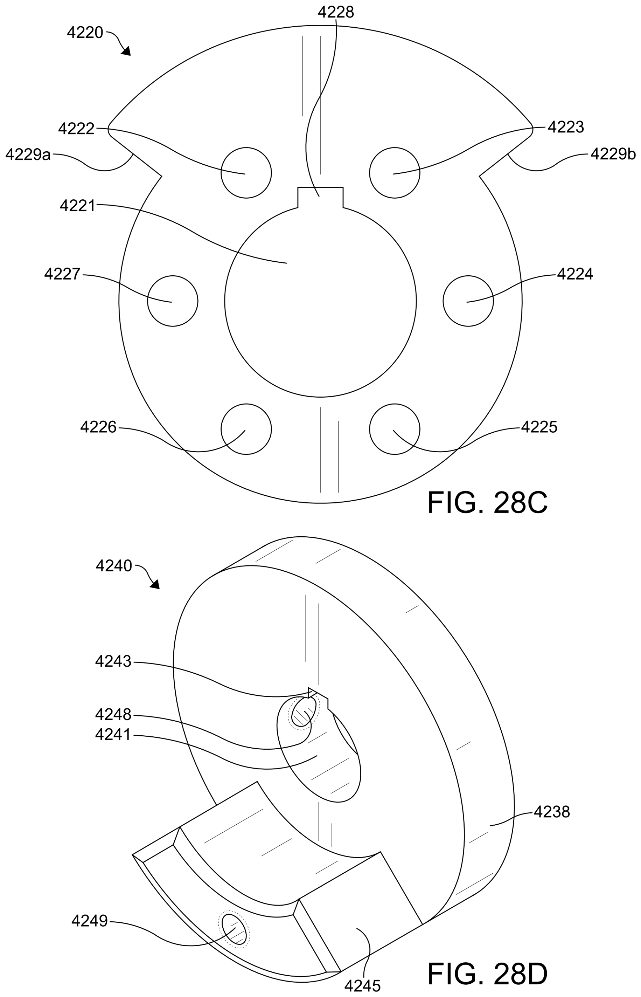

Figure 28C illustrates an example pin carrier 4220 configured to be disposed in the assembly 4200 of Figure 28A. The example pin carrier 4220 may comprise a first pin receptacle 4222, a second pin receptacle 4223, a third pin receptacle 4224, a fourth pin receptacle 4225, a fifth pin receptacle 4226, and a sixth pin receptacle 4227. A pin receptacle, such as the pin receptacles 4222-4227, may be configured such that a pin is at least partially disposed therein. A pin carrier 4220 may be configured such that the pin receptacles 4222-4227 are facing pin receptacles of an opposing pin carrier, such that a plurality of pins may be configured such that the pins are partially disposed in the pin receptacles 4222-4227 and partially disposed in the pin receptacles of the opposing pin carrier. At least one of the pins in the pin receptacles 4222-4227 may apply tension to one or more pull lines. The pin carrier 4220 may comprise a carrier aperture 4221. The carrier aperture 4221 may comprise a keyed cutout 4228. The keyed cutout 4228 may couple with a key of the shaft 4210, as described in more detail below. The example pin carrier 4220 may comprise a first rotation limiting surface 4229a and a second rotation limiting surface 4229b. The first rotation limiting surface 4229a and/or the second rotation limiting surface 4229b may act as a barrier to prevent the knobs, such as the A/P knob 4230 and the L/R knob 4240, from further rotating when contact is made with the first rotation limiting surface 4229a and/or the second rotation limiting surface 4229b, to prevent over rotation.

Figure 28D illustrates an example knob 4240 configured to be disposed in the assembly 4200 of Figure 28A. Figure 28D shows the L/R knob 4240. The knob 4240 may comprise the aperture 4241, the protruding area 4245, the ball detent aperture 4248, and the pull line anchor aperture 4249 described above. The aperture 4241 may comprise a keyway 4243 configured to allow keys of the shaft 4210 to traverse through. The A/P knob 4230 may have similar features. The A/P knob 4230 may have mirrored features.

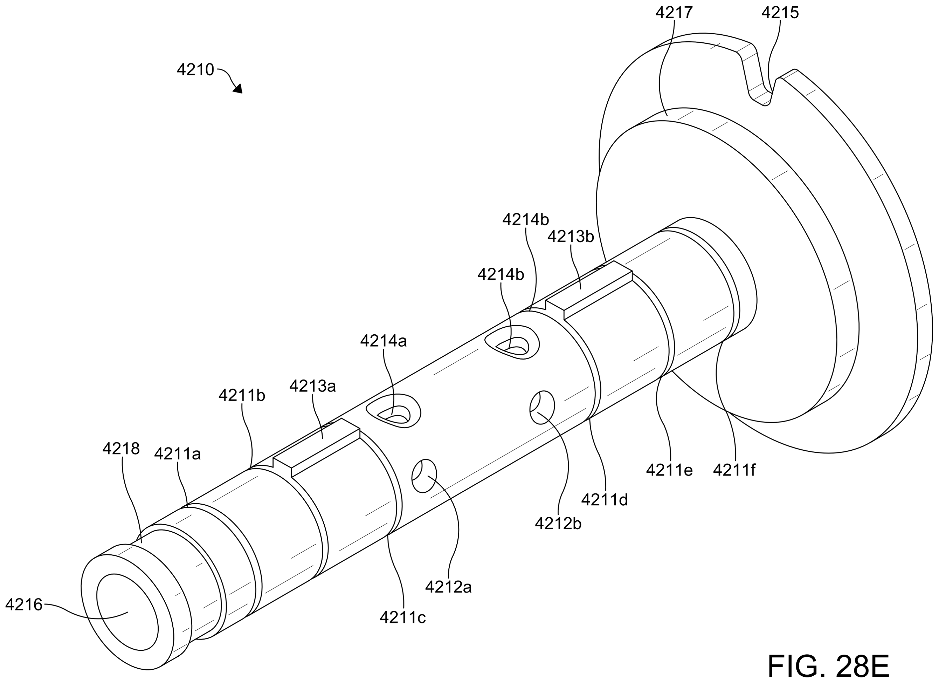

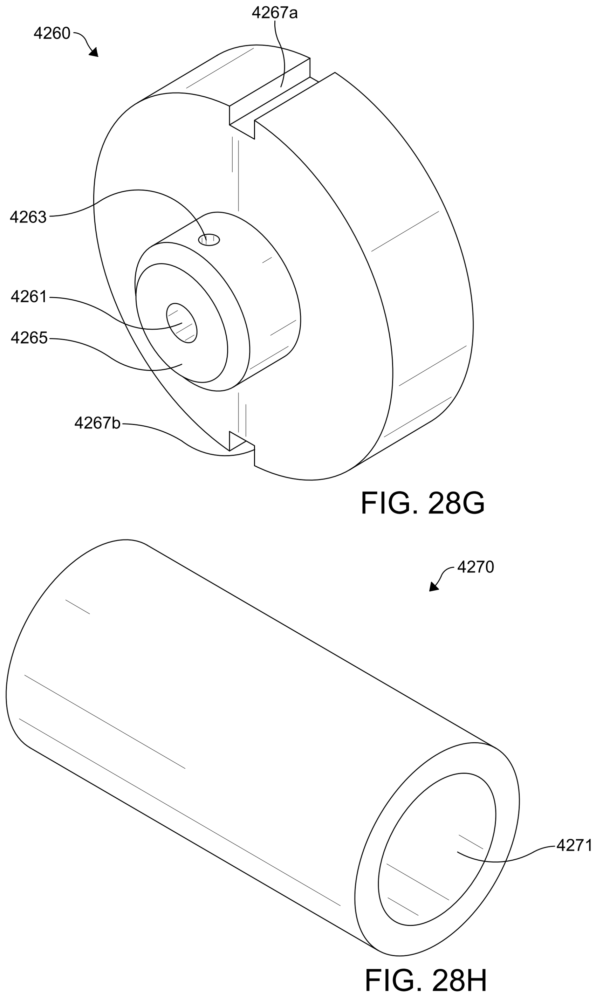

Figure 28E illustrates an example shaft 4210 configured to be disposed in the assembly 4200 of Figure 28A. The shaft 4210 may be elongate. The shaft 4210 may comprise a plurality of retaining ring grooves 4211a, 4211b, 4211c, 4211d, 4211e, and 4211f. The plurality of retaining ring grooves 4211a, 4211b, 4211c, 4211d, 4211e, and 4211f may be configured to couple with the retaining rings 4204a, 4204b, 4204c, 4204d, 4204e, and 4204f. The shaft 4210 may comprise the first dowel pin hole 4212a and the second dowel pin hole 4212b, configured to receive dowel pins, such as the first dowel pin 4206a and the second dowel pin 4206b. The shaft 4210 may comprise a plurality of keys, such as key 4213a and key 4213b, configured to couple with the keyed cutout 4228 of the pin carrier 4220, such as the first pin carrier 4220a and the second pin carrier 4220b. The shaft 4210 may comprise a lumen 4216 configured to house one or more pull lines. The one or more pull lines may exit and/or enter the lumen 4216 via the first pull line hole 4214a and/or the second pull line hole 4214b. For example, the one or more pull lines may comprise an anchor point disposed in the lumen 4216 and exit the lumen 4216 via the first pull line hole 4214a and/or the second pull line hole 4214b. As another example, the one or more pull lines may enter the lumen 4216 and exit the lumen 4216 via the first pull line hole 4214a and/or the second pull line hole 4214b. The lumen 4216 may be configured to house an inner shaft insert 4270 (shown in Figure 28H). The shaft 4210 may comprise a generally disc-shaped flange 4217 disposed adjacent a proximal end. The flange 4217 may comprise an anti-rotation feature 4215. The anti-rotation feature 4215 may comprise an indention on a rim of the flange 4217, configured to prevent the shaft 4210 from rotating. The shaft 4210 may comprise a retaining groove 4218 configured to couple to a tip rotation base 4260 (shown in Figure 28G).

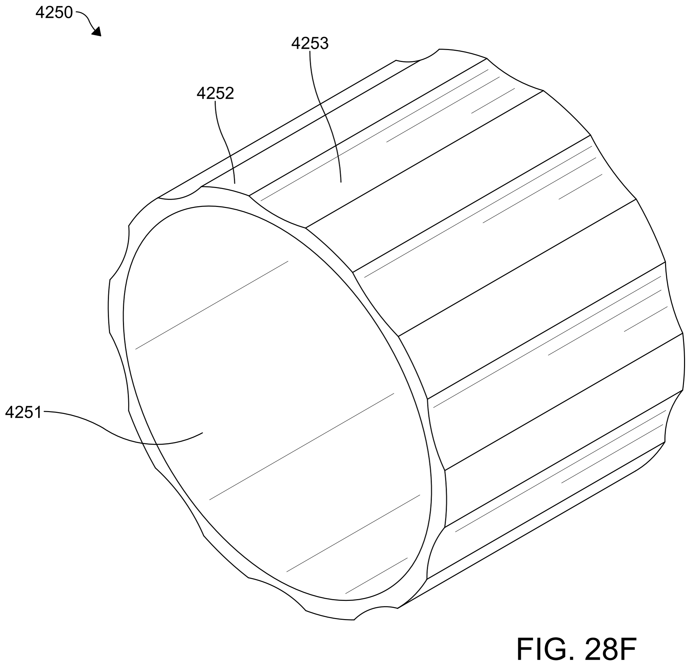

Figure 28F illustrates an example knob cover 4250 configured to be in communication with the assembly 4200 of Figure 28A. The example knob cover 4250 may be configured to surround a knob, such as the A/P knob 4230 and/or the L/R knob 4240, and increase manipulability of the knob. The knob cover 4250 may comprise a body and an aperture 4251 formed in the body configured to receive a knob. An exterior surface of the knob cover 4250 may comprise raised portions, such as raised portion 4252, and recessed portions, such as recessed portion 4253. The combination of raised portions and recessed portions may increase usability. The combination of raised portions and recessed portions may allow for fingers of a user to be coupled with one or more recessed portions.

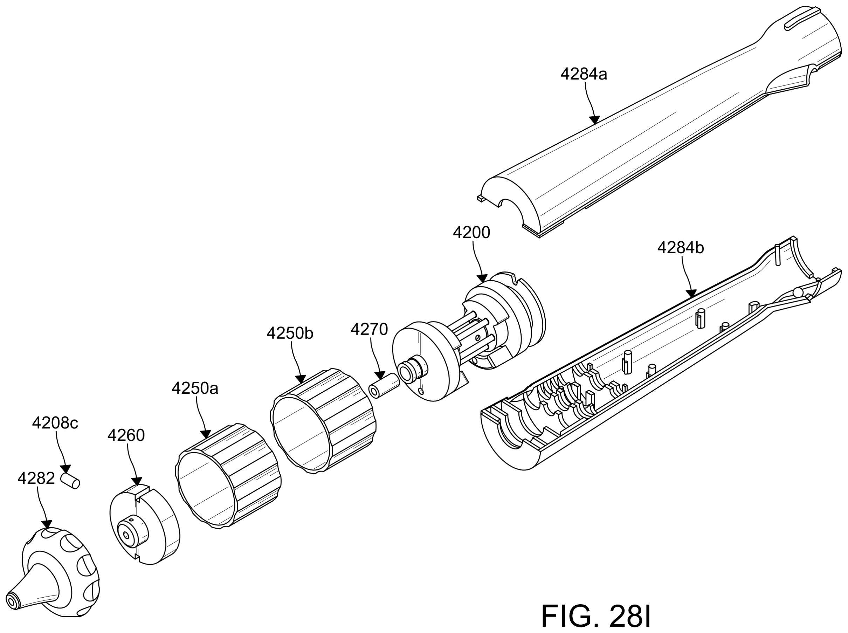

Figure 28G illustrates an example tip rotation base 4260 configured to be in communication with the assembly 4200 of Figure 28A. The tip rotation base 4260 may comprise a generally cylindrical main body configured for communication with a soft tip grip 4282, shown in Figure 281. The main body may comprise one or more indentions, such as a first indention 4267a and a second indention 4267b, configured to couple (e.g., mate, integrate, etc.) with the soft tip grip 4282. The main body may comprise one or more ball detent aperture (not shown), configured to receive one or more ball detents, such as a third ball detent 4208c (shown in Figure 28I). The one or more ball detent aperture may be threaded. The third ball detent 4208c may be configured to cause the tip rotation base 4260 to lock into a position until engagement of the third ball detent 4208c. The main body of the tip rotation base 4260 may be configured to couple with the retaining groove 4218 of the shaft 4210.

The main body may comprise a protruding area 4265. The protruding area 4265 and/or the main body may comprise an outer shaft through cavity 4261. The outer shaft through cavity 4261 may be configured to receive the shaft 4210, therein. The protruding area 4265 and/or the main body may comprise an adhesive fill cavity 4263, which extends to the outer shaft through cavity 4261 and may be configured to receive adhesive coupling the shaft 4210 with the tip rotation base 4260.

Figure 28H illustrates an example inner shaft insert 4270 configured to be in communication with the assembly 4200 of Figure 28A. The inner shaft insert 4270 may comprise a cylindrical body. The cylindrical body may comprise a lumen 4271 configured to receive one or more pull wires. An inner shaft may bond (e.g., couple, affix) within the lumen 4271 of the inner shaft insert 4270. One or more pull lines may be routed through the lumen 4271 of the inner shaft insert 4270.

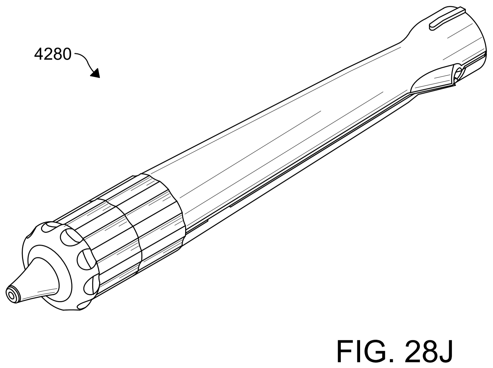

Figure 28I illustrates an exploded view of a handle assembly configured to comprise the assembly 4200 of Figure 28A. Figure 28J illustrates an assembled handle assembly 4280 configured to comprise the assembly 4200 of Figure 28A. The handle assembly 4280 may comprise the assembly 4200. When assembled, the flange 4217 of the assembly 4200 may be disposed in a plurality of handle shells, such as a first handle shell 4284a and a second handle shell 4284b. The A/P knob 4230 and the L/R knob 4240 of the assembly 4200 may be covered by a first knob cover 4250a and a second knob cover 4250b. The third ball detent 4208c may be disposed in the tip rotation base 4260. The soft tip grip 4282 may be coupled to the tip rotation base 4260.

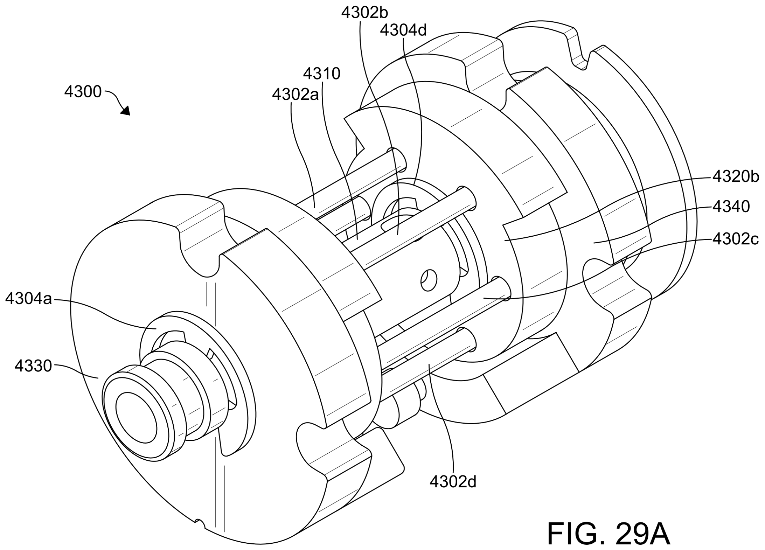

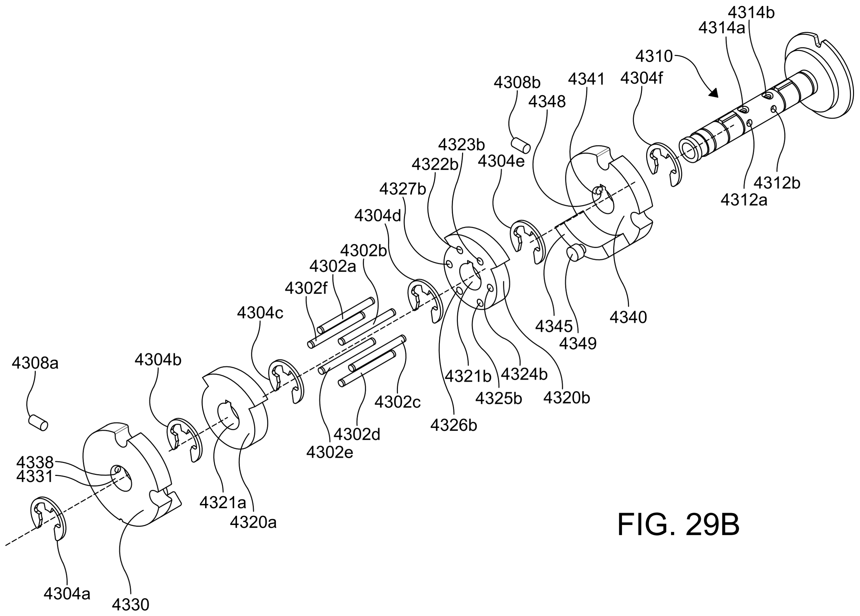

Figure 29A illustrates an example assembly 4300assembly 4300. The assembly 4300assembly 4300 may be disposed in a handle assembly of a catheter. The assembly 4300 may comprise a plurality of pins 4302a, 4302b, 4302c, 4302d, 4302e (shown in Figure 29B), and 4302f (shown in Figure 29B). The assembly 4300 may comprise a plurality of retaining rings 4304a, 4304b (shown in Figure 29B), 4304c (shown in Figure 29B), 4304d, 4304e (shown in Figure 29B), and 4304f (shown in Figure 29B). The assembly 4300 may comprise a shaft 4310. The assembly 4300 may comprise a plurality of pin carriers 4320a and 4320b. The assembly 4300 may comprise an anterior and posterior (A/P) knob 4330. The assembly 4300 may comprise a left and right (L/R) knob 4340. The assembly 4300 and components will be described in more detail below. The assembly 4300 may allow for 4-way steering of a catheter tip, independent tip rotation, and detent features for a home (e.g., start, initial, original, etc.) position. The assembly 4300 may be and/or comprise a cartridge.

Figure 29B illustrates an exploded view of the assembly 4300 of Figure 29A. The pins 4302a, 4302b, 4302c, 4302d, 4302e, and 4302f may comprise a cylindrical shape. The pins 4302a, 4302b, 4302c, 4302d, 4302e, and 4302f may dowels. The pins 4302a, 4302b, 4302c, 4302d, 4302e, and 4302f may be configured to be partially disposed in a first pin carrier 4320a of the plurality of pin carriers and partially disposed in a second pin carrier 4320b of the plurality of pin carriers. The plurality of pins 4302a, 4302b, 4302c, 4302d, 4302e, and 4302f may be configured to apply tension to one or more pull lines.

The retaining rings 4304a, 4304b, 4304c, 4304d, 4304e, and 4304f may comprise a ring body and a mostly closed aperture formed in the ring body configured to receive the shaft 4310. The retaining rings 4304a, 4304b, 4304c, 4304d, 4304e, and 4304f may comprise side loading retaining rings. The relationship between the retaining rings 4304a, 4304b, 4304c, 4304d, 4304e, and 4304f and the shaft will be described in more detail below. A first retaining ring 4304a may be configured to couple the A/P knob 4330 to the shaft 4310. A second retaining ring 4304b may be configured to couple the A/P knob 4330 and/or the first pin carrier 4320a to the shaft 4310. A third retaining ring 4304c may be configured to couple the first pin carrier 4320a to the shaft 4310. A fourth retaining ring 4304d may be configured to couple the second pin carrier 4320b to the shaft 4310. A fifth retaining ring 4304e may be configured to couple the second pin carrier 4320b and/or the L/R knob 4340 to the shaft 4310. A sixth retaining ring 4304f may be configured to couple the L/R knob 4340 to the shaft 4310.

The shaft 4310 may comprise a plurality of apertures, such as a first dowel pin hole 4312a and a second dowel pin hole 4312b, configured to receive dowel pins, such as a first dowel pin 4306a and a second dowel pin 4306b. The shaft 4310 may comprise a plurality of apertures, such as a first pull line hole 4314a and a second pull line hole 4314b, configured to allow traversal of a pull line. The shaft 4310 will be described in more detail below. The dowel pins may be configured to guide pull lines through the pull line holes. For example, the first dowel pin 4306a may be configured to guide one or more pulls lines through the first pull line hole 4314a, and the second dowel pin 4306b may be configured to guide one or more pulls lines through the second pull line hole 4314b.

The pin carriers 4320, such as the first pin carrier 4320a and the second pin carrier 4320b, may comprise a carrier body and a carrier aperture 4321, such as a first carrier aperture 4321a and a second carrier aperture 4321b, formed in the carrier body configured to receive the shaft 4310. The pin carriers 4320 may comprise a plurality of receptacles, such as a first pin receptacle 4322b and a second pin receptacle 4323b. The pin carriers 4320 will be described in more detail below.

The knobs, such as the A/P knob 4330 and the L/R knob 4340, may comprise a main body and an aperture, such as aperture 4331 and aperture 4341, formed in the main body configured to receive the shaft 4310. The knobs may be configured to control deflection of a catheter tip. For example, the A/P knob 4330 may be configured to control anterior and/or posterior deflection of the catheter tip. As another example, the L/R knob 4340 may be configured to control left and/or right deflection of the catheter tip. The knobs may comprise a protruding area, such as protruding area 4345, configured to allow an associated pin carrier 4320 to reside thereon. A pull line may be coupled to (e.g., anchored, tied, attached, etc.) the protruding area, such as the protruding area 4345. The protruding area 4345 of the knobs, such as the A/P knob 4330 and the L/R knob 4340, may comprise a pull line tie point, such as pull line tie point 4349. One or more pull lines may be attached to a pull line tie point, such as the line pull tie point 4349.

The knobs may comprise a ball detent aperture, such as ball detent aperture 4338 and ball detent aperture 4348, configured to receive ball detents, such as ball detent 4308a and ball detent 4308b. The ball detent apertures 4338 and 4348 may be configured such that associated ball detents 4308a and 4308b are configured to reside in the ball detent apertures 4338 and 4348. A ball detent, such as the ball detents 4308a and 4308b, may couple with the shaft 4310 to lock a knob into a home (e.g., start, initial, original, etc.) position. The ball detent apertures 4338 and 4348 may comprise threading configured to receive the ball detents 4308a and 4308b.

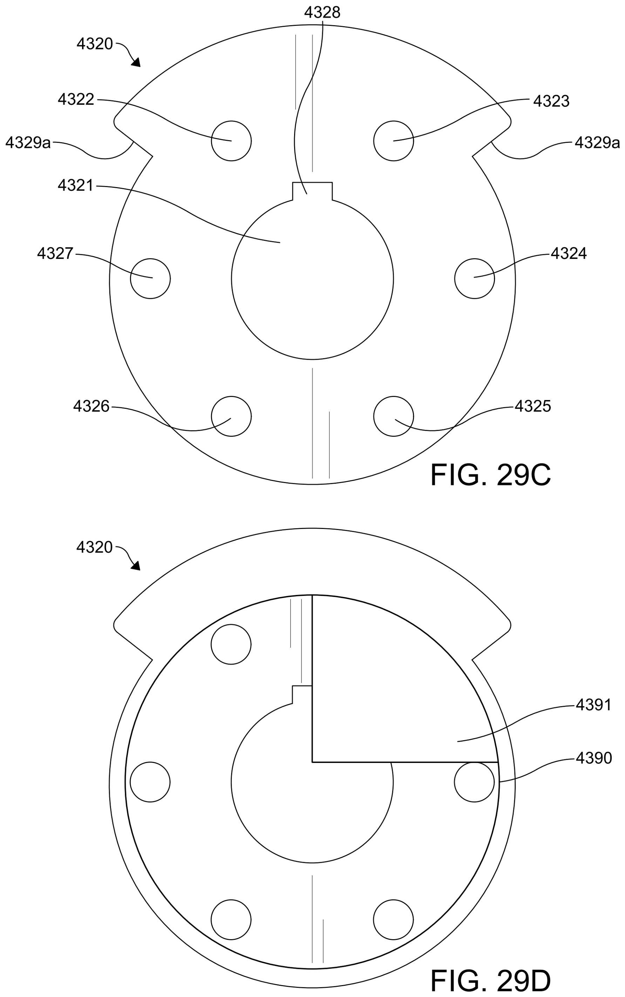

Figure 29C illustrates an example pin carrier 4320 configured to be disposed in the assembly 4300 of Figure 29A. The example pin carrier 4320 may comprise a first pin receptacle 4322, a second pin receptacle 4323, a third pin receptacle 4324, a fourth pin receptacle 4325, a fifth pin receptacle 4326, and a sixth pin receptacle 4327. A pin receptacle, such as the pin receptacles 4322-4327, may be configured such that a pin is at least partially disposed therein. A pin carrier 4320 may be configured such that the pin receptacles 4322-4327 are facing pin receptacles of an opposing pin carrier, such that a plurality of pins may be configured such that the pins are partially disposed in the pin receptacles 4322-4327 and partially disposed in the pin receptacles of the opposing pin carrier. At least one of the pins in the pin receptacles 4322-4327 may apply tension to one or more pull lines. The pin carrier 4320 may comprise a carrier aperture 4321. The carrier aperture 4321 may comprise a keyed cutout 4328. The keyed cutout 4328 may couple with a key of the shaft 4310, as described in more detail below. The example pin carrier 4320 may comprise a first rotation limiting surface 4329a and a second rotation limiting surface 4329b. The first rotation limiting surface 4329a and/or the second rotation limiting surface 4329b may act as a barrier to prevent the knobs, such as the A/P knob 4330 and the L/R knob 4340, from further rotating when contact is made with the first rotation limiting surface 4329a and/or the second rotation limiting surface 4329b, to prevent over rotation.

Figure 29D illustrates a relationship between an effective diameter 4390 created by the pin receptacles of the pin carrier 4320 and a wrap angle 4391 experienced by pull lines associated with the pin carrier 4320.

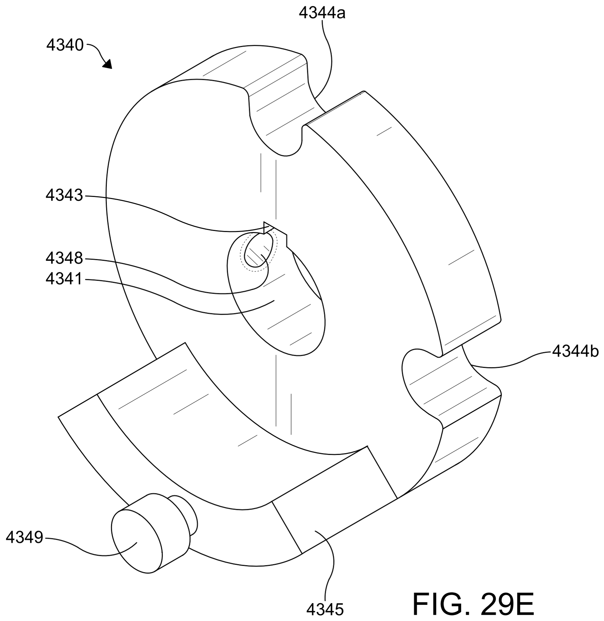

Figure 29E illustrates an example knob 4340 configured to be disposed in the assembly 4300 of Figure 29A. Figure 29E shows the L/R knob 4340. The knob 4340 may comprise the aperture 4341, the protruding area 4345, the ball detent aperture 4348, and the pull line tie point 4349 described above. The aperture 4341 may comprise a keyway 4343 configured to allow keys of the shaft 4310 to traverse through. The L/R knob 4340 may comprise a plurality of keyed features, such as a first keyed feature 4344a and a second keyed feature 4344b, configured to couple with keys of a knob cover, described below. The A/P knob 4330 may have similar features. The A/P knob 4330 may have mirrored features.

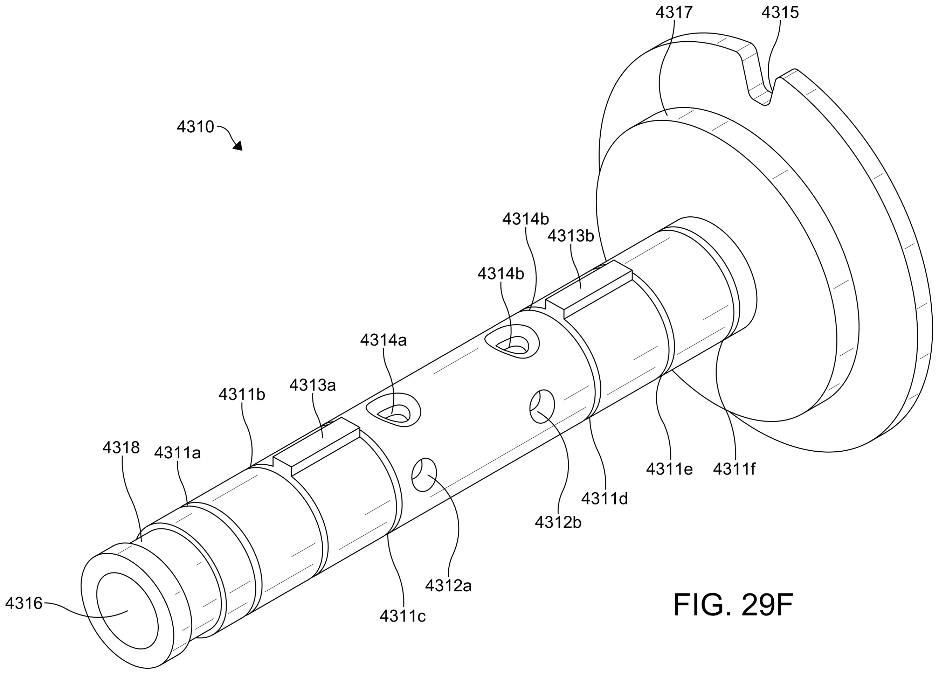

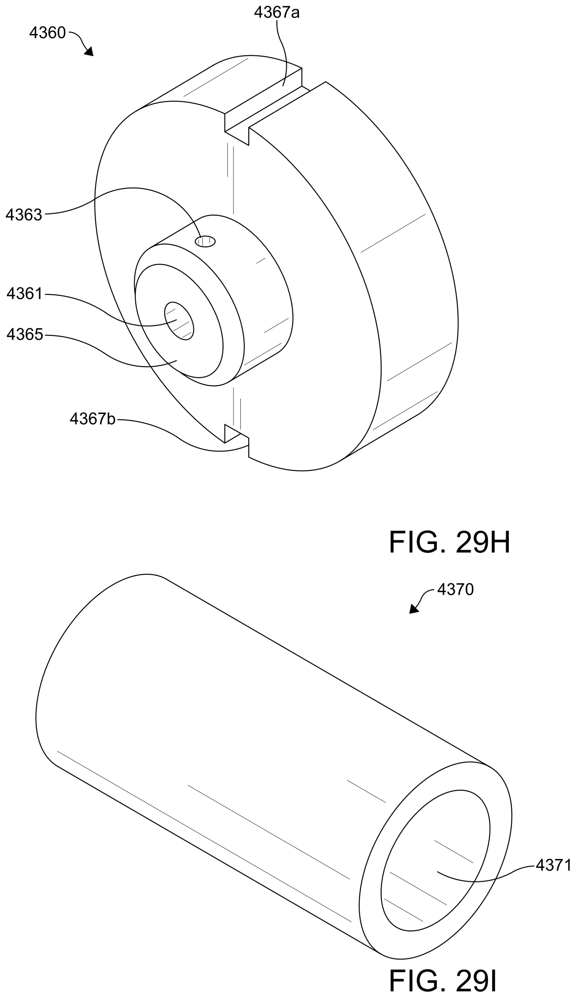

Figure 29F illustrates an example shaft 4310 configured to be disposed in the assembly 4300 of Figure 29A. The shaft 4310 may be elongate. The shaft 4310 may comprise a plurality of retaining ring grooves 4311a, 4311b, 4311c, 4311d, 4311e, and 4311f. The plurality of retaining ring grooves 4311a, 4311b, 4311c, 4311d, 4311e, and 4311f may be configured to couple with the retaining rings 4304a, 4304b, 4304c, 4304d, 4304e, and 4304f. The shaft 4310 may comprise the first dowel pin hole 4312a and the second dowel pin hole 4312b, configured to receive dowel pins, such as the first dowel pin 4306a and the second dowel pin 4306b. The shaft 4310 may comprise a plurality of keys, such as key 4313a and key 4313b, configured to couple with the keyed cutout 4328 of the pin carrier 4320, such as the first pin carrier 4320a and the second pin carrier 4320b. The shaft 4310 may comprise a lumen 4316 configured to house one or more pull lines. The one or more pull lines may exit and/or enter the lumen 4316 via the first pull line hole 4314a and/or the second pull line hole 4314b. For example, the one or more pull lines may comprise an anchor point disposed in the lumen 4316 and exit the lumen 4316 via the first pull line hole 4314a and/or the second pull line hole 4314b. As another example, the one or more pull lines may enter the lumen 4316 and exit the lumen 4316 via the first pull line hole 4314a and/or the second pull line hole 4314b. The lumen 4316 may be configured to house an inner shaft insert 4370 (shown in Figure 29I). The shaft 4310 may comprise a generally disc-shaped flange 4317 disposed adjacent a proximal end. The flange 4317 may comprise an anti-rotation feature 4315. The anti-rotation feature 4315 may comprise an indention on a rim of the flange 4317, configured to prevent the shaft 4310 from rotating. The shaft 4310 may comprise a retaining groove 4318 configured to couple to a tip rotation base 4360 (shown in Figure 29H).

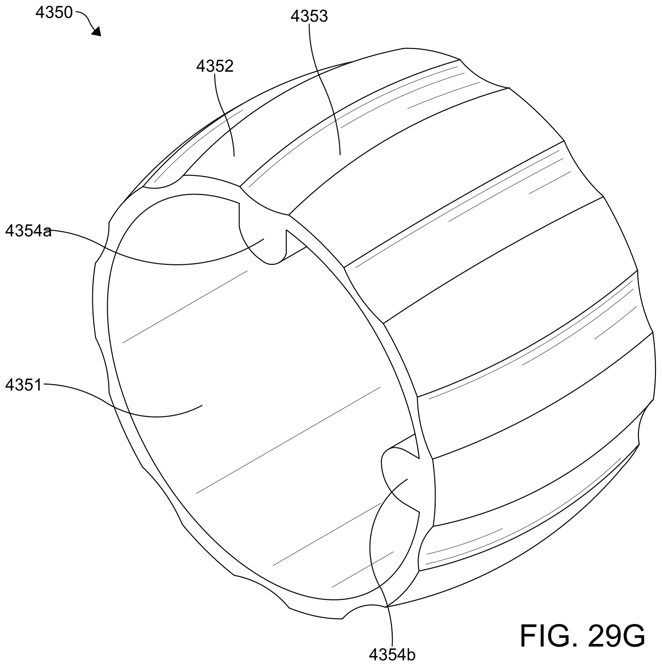

Figure 29G illustrates an example knob cover 4350 configured to be in communication with the assembly 4300 of Figure 29A. The example knob cover 4350 may be configured to surround a knob, such as the A/P knob 4330 and/or the L/R knob 4340, and increase manipulability of the knob. The knob cover 4350 may comprise a body and an aperture 4351 formed in the body configured to receive a knob. An exterior surface of the knob cover 4350 may comprise raised portions, such as raised portion 4352, and recessed portions, such as recessed portion 4353. The combination of raised portions and recessed portions may increase usability. The combination of raised portions and recessed portions may allow for fingers of a user to be coupled with one or more recessed portions. An interior surface of the knob cover 4350 may comprise keys, such as a first key 4354a and a second key 4354b, configured to couple with keyed features, such as the first keyed feature 4344a and the second keyed feature 4344b, of an associated knob. The keys coupled with the keyed features may secure the knob cover 4350 with an associated knob.

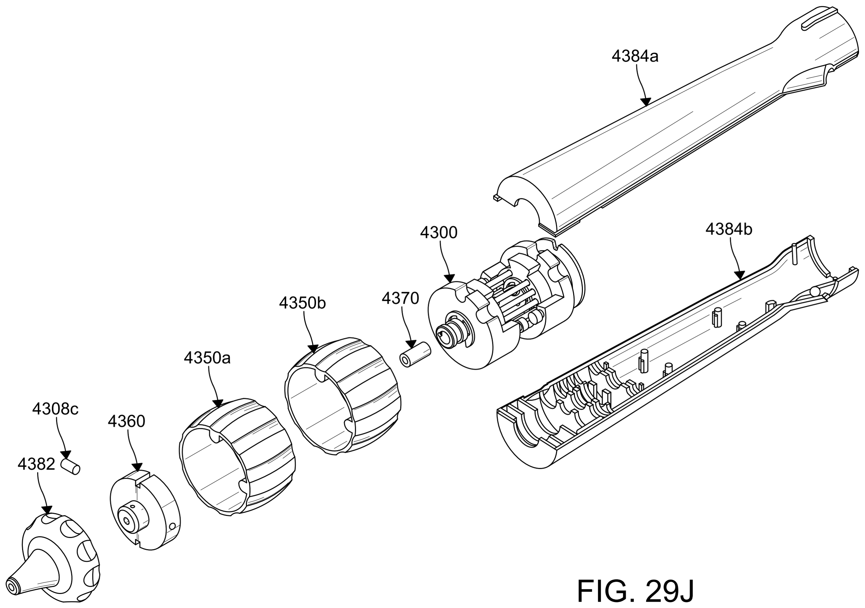

Figure 29H illustrates an example tip rotation base 4360 configured to be in communication with the assembly 4300 of Figure 29A. The tip rotation base 4360 may comprise a generally cylindrical main body configured for communication with a soft tip grip 4382, shown in Figure 29J. The main body may comprise one or more indentions, such as a first indention 4367a and a second indention 4367b, configured to couple (e.g., mate, integrate, etc.) with the soft tip grip 4382. The main body may comprise one or more ball detent aperture (not shown), configured to receive one or more ball detents, such as a third ball detent 4308c (shown in Figure 29J). The one or more ball detent aperture may be threaded. The third ball detent 4308c may be configured to cause the tip rotation base 4360 to lock into a position until engagement of the third ball detent 4308c. The main body of the tip rotation base 4360 may be configured to couple with the retaining groove 4318 of the shaft 4310.

The main body may comprise a protruding area 4365. The protruding area 4365 and/or the main body may comprise an outer shaft through cavity 4361. The outer shaft through cavity 4361 may be configured to receive the shaft 4310, therein. The protruding area 4365 and/or the main body may comprise an adhesive fill cavity 4363, which extends to the outer shaft through cavity 4361 and may be configured to receive adhesive coupling the shaft 4310 with the tip rotation base 4360.

Figure 29I illustrates an example inner shaft insert 4370 configured to be in communication with the assembly 4300 of Figure 29A. The inner shaft insert 4370 may comprise a cylindrical body. The cylindrical body may comprise a lumen 4371 configured to receive one or more pull wires. An inner shaft may bond (e.g., couple, affix) within the lumen 4371 of the inner shaft insert 4370. One or more pull lines may be routed through the lumen 4371 of the inner shaft insert 4370.

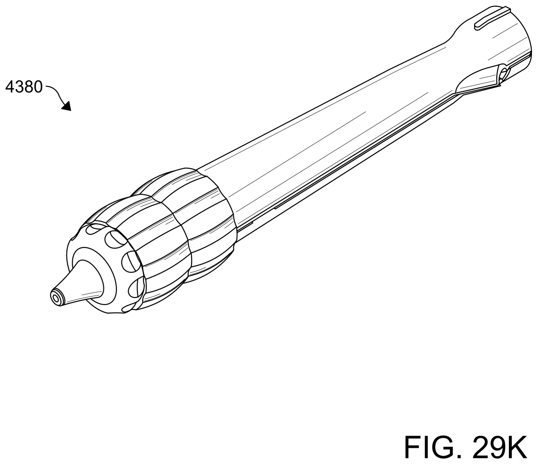

Figure 29J illustrates an exploded view of a handle assembly configured to comprise the assembly 4300 of Figure 29A. Figure 29K illustrates an assembled handle assembly 4380 configured to comprise the assembly 4300 of Figure 29A. The handle assembly 4380 may comprise the assembly 4300. When assembled, the flange 4317 of the assembly 4300 may be disposed in a plurality of handle shells, such as a first handle shell 4384a and a second handle shell 4384b. The A/P knob 4330 and the L/R knob 4340 of the assembly 4300 may be covered by a first knob cover 4350a and a second knob cover 4350b. The inner shaft insert 4370 may couple with the lumen 4316 of the shaft 4310 and/or the outer shaft through cavity 4361 of the tip rotation base 4360. The inner shaft insert 4370 may couple with the lumen 4316 of the shaft 4310 and/or the outer shaft through cavity 4361 of the tip rotation base 4360 using an adhesive. The third ball detent 4308c may be disposed in the tip rotation base 4360. The soft tip grip 4382 may be coupled to the tip rotation base 4360.

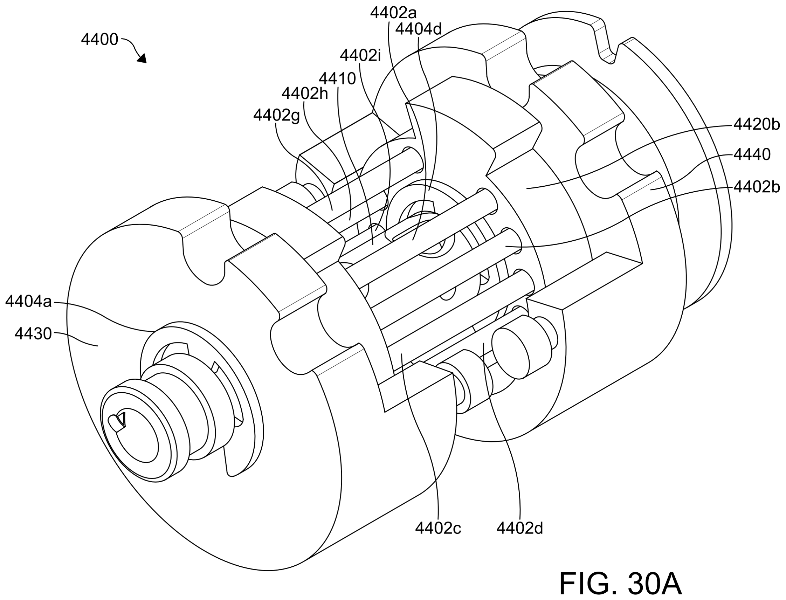

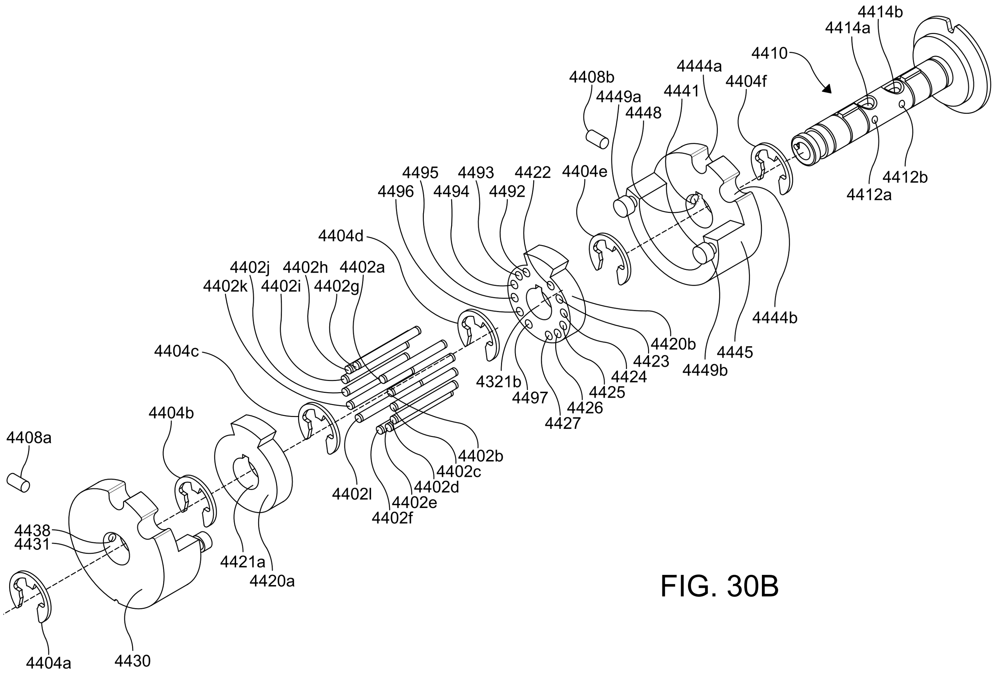

Figure 30A illustrates an example assembly 4400assembly 4400. The assembly 4400assembly 4400 may be disposed in a handle assembly of a catheter. The assembly 4400 may comprise a plurality of pins 4402a, 4402b, 4402c, 4402d, 4402e, 4402f (shown in Figure 30B), 4402g, 4402h, 4402i, 4402j (shown in Figure 30B), 4402k (shown in Figure 30B), and 44021 (shown in Figure 30B). The assembly 4400 may comprise a plurality of retaining rings 4404a, 4404b (shown in Figure 30B), 4404c (shown in Figure 30B), 4404d, 4404e (shown in Figure 30B), and 4404f (shown in Figure 30B). The assembly 4400 may comprise a shaft 4410. The assembly 4400 may comprise a plurality of pin carriers 4420a and 4420b. The assembly 4400 may comprise an anterior and posterior (A/P) knob 4430. The assembly 4400 may comprise a left and right (L/R) knob 4440. The assembly 4400 and components will be described in more detail below. The assembly 4400 may allow for 4-way steering of a catheter tip, independent tip rotation, and detent features for a home (e.g., start, initial, original, etc.) position. The assembly 4400 may be and/or comprise a cartridge.

Figure 30B illustrates an exploded view of the assembly 4400 of Figure 30A. The pins 4402a, 4402b, 4402c, 4402d, 4402e, 4402f, 4402g, 4402h, 4402i, 4402j, 4402k, and 44021 may comprise a cylindrical shape. The pins 4402a, 4402b, 4402c, 4402d, 4402e, 4402f, 4402g, 4402h, 4402i, 4402j, 4402k, and 44021 may be dowels. The pins 4402a, 4402b, 4402c, 4402d, 4402e, 4402f, 4402g, 4402h, 4402i, 4402j, 4402k, and 44021 may be configured to be partially disposed in a first pin carrier 4420a of the plurality of pin carriers and partially disposed in a second pin carrier 4420b of the plurality of pin carriers. The plurality of pins 4402a, 4402b, 4402c, 4402d, 4402e, 4402f, 4402g, 4402h, 4402i, 4402j, 4402k, and 44021 may be configured to apply tension to one or more pull lines.

The retaining rings 4404a, 4404b, 4404c, 4404d, 4404e, and 4404f may comprise a ring body and a mostly closed aperture formed in the ring body configured to receive the shaft 4410. The retaining rings 4404a, 4404b, 4404c, 4404d, 4404e, and 4404f may comprise side loading retaining rings. The relationship between the retaining rings 4404a, 4404b, 4404c, 4404d, 4404e, and 4404f and the shaft will be described in more detail below. A first retaining ring 4404a may be configured to couple the A/P knob 4430 to the shaft 4410. A second retaining ring 4404b may be configured to couple the A/P knob 4430 and/or the first pin carrier 4420a to the shaft 4410. A third retaining ring 4404c may be configured to couple the first pin carrier 4420a to the shaft 4410. A fourth retaining ring 4404d may be configured to couple the second pin carrier 4420b to the shaft 4410. A fifth retaining ring 4404e may be configured to couple the second pin carrier 4420b and/or the L/R knob 4440 to the shaft 4410. A sixth retaining ring 4404f may be configured to couple the L/R knob 4440 to the shaft 4410.

The shaft 4410 may comprise a plurality of apertures, such as a first dowel pin hole 4412a and a second dowel pin hole 4412b, configured to receive dowel pins, such as a first dowel pin 4406a and a second dowel pin 4406b. The shaft 4410 may comprise a plurality of apertures, such as a first pull line hole 4414a and a second pull line hole 4414b, configured to allow traversal of a pull line. The shaft 4410 will be described in more detail below. The dowel pins may be configured to guide pull lines through the pull line holes. For example, the first dowel pin 4406a may be configured to guide one or more pulls lines through the first pull line hole 4414a, and the second dowel pin 4406b may be configured to guide one or more pulls lines through the second pull line hole 4414b.

The pin carriers 4420, such as the first pin carrier 4420a and the second pin carrier 4420b, may comprise a carrier body and a carrier aperture 4421, such as a first carrier aperture 4421a and a second carrier aperture 4421b, formed in the carrier body configured to receive the shaft 4410. The pin carriers 4420 may comprise a plurality of receptacles, such as a first pin receptacle 4422b and a second pin receptacle 4423b. The pin carriers 4420 will be described in more detail below.

The knobs, such as the A/P knob 4430 and the L/R knob 4440, may comprise a main body and an aperture, such as aperture 4431 and aperture 4441, formed in the main body configured to receive the shaft 4410. The knobs may be configured to control deflection of a catheter tip. For example, the A/P knob 4430 may be configured to control anterior and/or posterior deflection of the catheter tip. As another example, the L/R knob 4440 may be configured to control left and/or right deflection of the catheter tip. The knobs may comprise a protruding area, such as protruding area 4445, configured to allow an associated pin carrier 4420 to reside thereon. A pull line may be coupled to (e.g., anchored, tied, attached, etc.) the protruding area, such as the protruding area 4445. The protruding area 4445 may comprise of the knobs, such as the A/P knob 4430 and the L/R knob 4440, may comprise a plurality of pull line tie points, such as a first pull line tie point 4449a and a second pull line tie point 4449b. The first pull line tie point 4449a and the second pull line tie point 4449b may be disposed on opposing ends of the protruding area 4445 as shown. One or more pull lines may be attached to one or more pull line tie points, such as the first pull line tie point 4449a and the second pull line tie point 4449b.

The knobs may comprise a ball detent aperture, such as ball detent aperture 4438 and ball detent aperture 4448, configured to receive ball detents, such as ball detent 4408a and ball detent 4408b. The ball detent apertures 4438 and 4448 may be configured such that associated ball detents 4408a and 4408b are configured to reside in the ball detent apertures 4438 and 4448. A ball detent, such as the ball detents 4408a and 4408b, may couple with the shaft 4410 to lock a knob into a home (e.g., start, initial, original, etc.) position. The ball detent apertures 4438 and 4448 may comprise threading configured to receive the ball detents 4408a and 4408b.

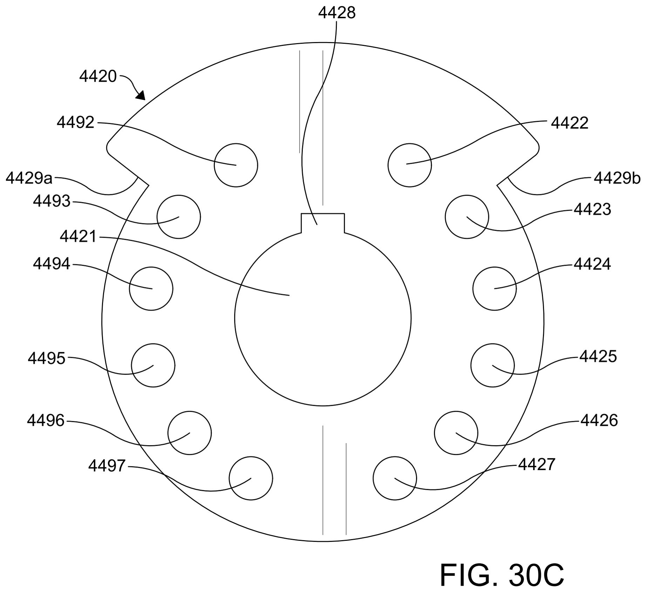

Figure 30C illustrates an example pin carrier 4420 configured to be disposed in the assembly 4400 of Figure 30A. The example pin carrier 4420 may comprise a first pin receptacle 4422, a second pin receptacle 4423, a third pin receptacle 4424, a fourth pin receptacle 4425, a fifth pin receptacle 4426, a sixth pin receptacle 4427, a seventh pin receptacle 4492, an eighth pin receptacle 4493, a ninth pin receptacle 4494, a tenth pin receptacle 4495, an eleventh pin receptacle 4496, and a twelfth pin receptacle 4497. A pin receptacle, such as the pin receptacles 4422-4427 and 4429-4497, may be configured such that a pin is at least partially disposed therein. A pin carrier 4420 may be configured such that the pin receptacles 4422-4427 and 4429-4497 are facing pin receptacles of an opposing pin carrier, such that a plurality of pins may be configured such that the pins are partially disposed in the pin receptacles 4422-4427 and 4429-4497 and partially disposed in the pin receptacles of the opposing pin carrier. At least one of the pins in the pin receptacles 4422-4427 and 4429-4497 may apply tension to one or more pull lines.

The pin carrier 4420 may comprise a carrier aperture 4421. The carrier aperture 4421 may comprise a keyed cutout 4428. The keyed cutout 4428 may couple with a key of the shaft 4410, as described in more detail below. The example pin carrier 4420 may comprise a first rotation limiting surface 4429a and a second rotation limiting surface 4429b. The first rotation limiting surface 4429a and/or the second rotation limiting surface 4429b may act as a barrier to prevent the knobs, such as the A/P knob 4430 and the L/R knob 4440, from further rotating when contact is made with the first rotation limiting surface 4429a and/or the second rotation limiting surface 4429b, to prevent over rotation.

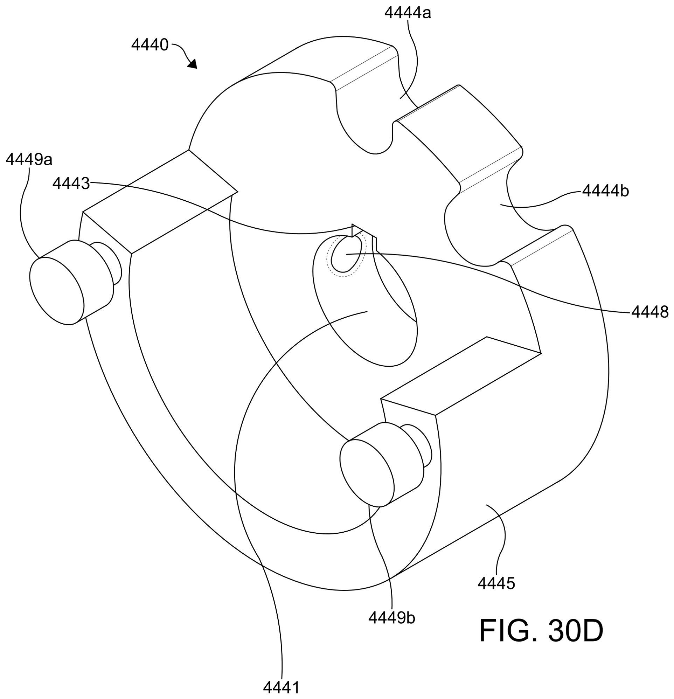

Figure 30D illustrates an example knob 4440 configured to be disposed in the assembly 4400 of Figure 30A. Figure 30D shows the L/R knob 4440. The knob 4440 may comprise the aperture 4441, the protruding area 4445, the ball detent aperture 4448, the first pull line tie point 4449a, and the second pull line tie point 4449b described above. The aperture 4441 may comprise a keyway 4443 configured to allow keys of the shaft 4410 to traverse through. The L/R knob 4440 may comprise a plurality of keyed features, such as a first keyed feature 4444a and a second keyed feature 4444b, configured to couple with keys of a knob cover, described below. The A/P knob 4430 may have similar features. The A/P knob 4430 may have mirrored features.

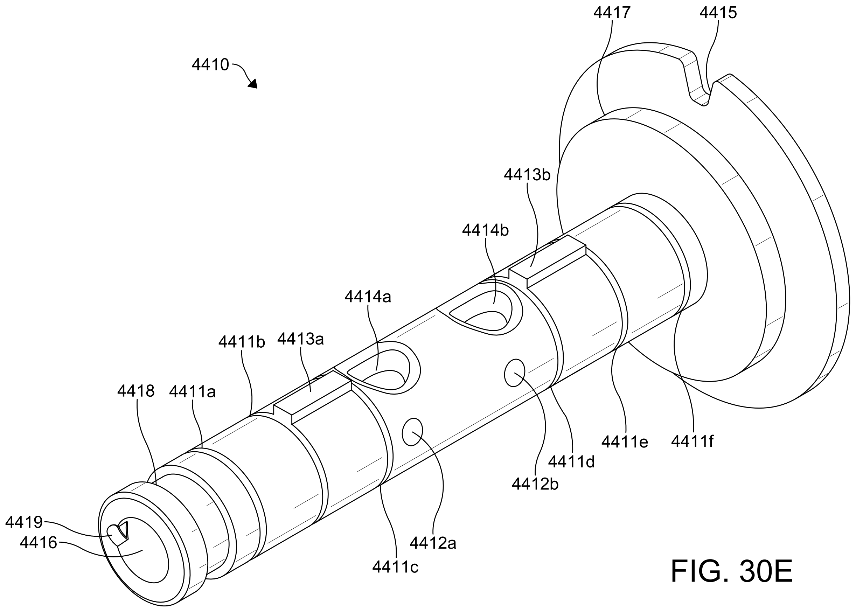

Figure 30E illustrates an example shaft 4410 configured to be disposed in the assembly 4400 of Figure 30A. The shaft 4410 may be elongate. The shaft 4410 may comprise a plurality of retaining ring grooves 4411a, 4411b, 4411c, 4411d, 4411e, and 4411f. The plurality of retaining ring grooves 4411a, 4411b, 4411c, 4411d, 4411e, and 4411f may be configured to couple with the retaining rings 4404a, 4404b, 4404c, 4404d, 4404e, and 4404f. The shaft 4410 may comprise the first dowel pin hole 4412a and the second dowel pin hole 4412b, configured to receive dowel pins, such as the first dowel pin 4406a and the second dowel pin 4406b. The shaft 4410 may comprise a plurality of keys, such as key 4413a and key 4413b, configured to couple with the keyed cutout 4428 of the pin carrier 4420, such as the first pin carrier 4420a and the second pin carrier 4420b.

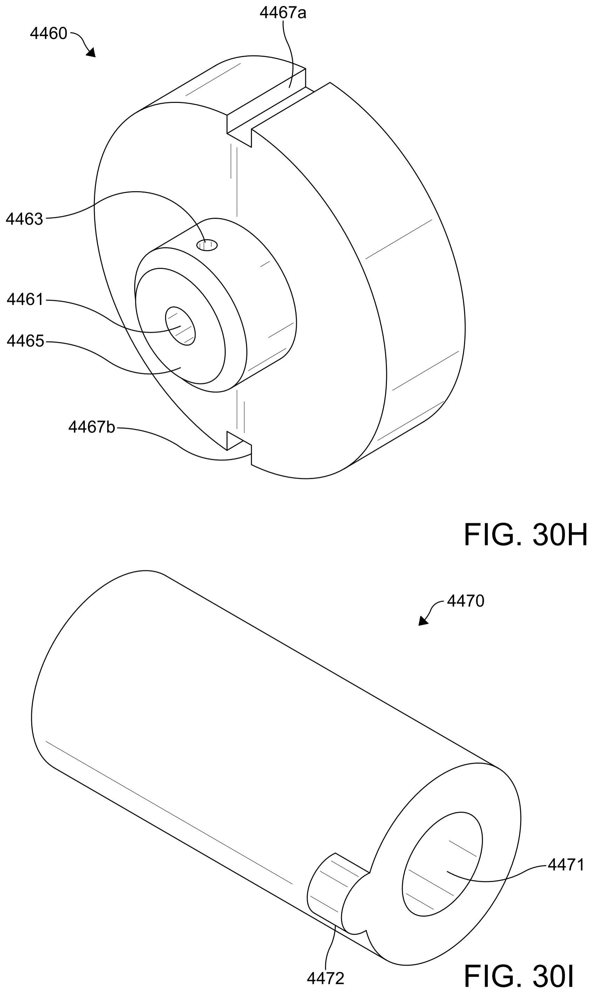

The shaft 4410 may comprise a lumen 4416 configured to house one or more pull lines. The one or more pull lines may exit and/or enter the lumen 4416 via the first pull line hole 4414a and/or the second pull line hole 4414b. For example, the one or more pull lines may comprise an anchor point disposed in the lumen 4416 and exit the lumen 4416 via the first pull line hole 4414a and/or the second pull line hole 4414b. As another example, the one or more pull lines may enter the lumen 4416 exit the lumen 4416 via the first pull line hole 4414a and/or the second pull line hole 4414b. The lumen 4416 may be configured to house an inner shaft insert 4470 (shown in Figure 30I). The lumen 4416 may comprise a keyed feature 4419 configured to couple with a key 4472 of the inner shaft insert 4470. The shaft 4410 may comprise a generally disc-shaped flange 4417 disposed adjacent a proximal end. The flange 4417 may comprise an anti-rotation feature 4415. The anti-rotation feature 4415 may comprise an indention on a rim of the flange 4417, configured to prevent the shaft 4410 from rotating. The shaft 4410 may comprise a retaining groove 4418 configured to couple to a tip rotation base 4460 (shown in Figure 30H).

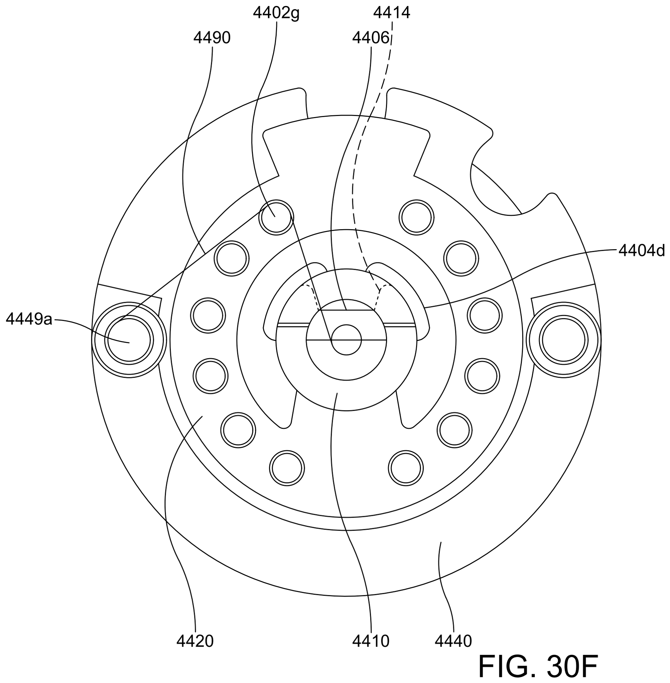

Figure 30F shows a relationships between several components of the assembly 4400 to apply tension to a pull line 4490. As shown, a dowel pin 4406 may be configured to keep the pull line 4490 at a vertical midpoint of a circle created by the lumen 4416 of the shaft 4410. The pull line 4490 may exit the shaft 4410 via the pull line hole 4414. The pull line hole 4414 may comprise a taper. The taper may reduce pull line 4490 interference. The pull line 4490 may wrap around the pin 4402g of the pin carrier 4420. The pull line 4490 may be coupled to the first pull line tie point 4449a of the L/R knob 4440. The fourth retaining ring 4404d may be configured to couple the second pin carrier 4420b to the shaft 4410. Moving the pull line tie points, such as the first pull line tie point 4449a and the second pull line tie point 4449b, closer to the pull line hole 4414 reduces an initial wrap angle. Increasing the number and frequency of pins, such as 4402a-1, and reducing the space between the pins causes less aggressive pull line 4490 wrapping.

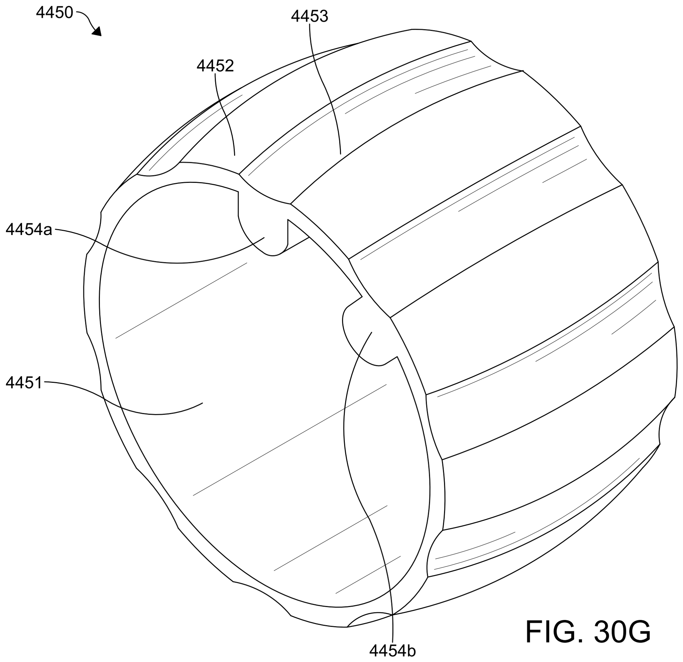

Figure 30G illustrates an example knob cover 4450 configured to be in communication with the assembly 4400 of Figure 30A. The example knob cover 4450 may be configured to surround a knob, such as the A/P knob 4430 and/or the L/R knob 4440, and increase manipulability of the knob. The knob cover 4450 may comprise a body and an aperture 4451 formed in the body configured to receive a knob. An exterior surface of the knob cover 4450 may comprise raised portions, such as raised portion 4452, and recessed portions, such as recessed portion 4453. The combination of raised portions and recessed portions may increase usability. The combination of raised portions and recessed portions may allow for fingers of a user to be coupled with one or more recessed portions. An interior surface of the knob cover 4450 may comprise keys, such as a first key 4454a and a second key 4454b, configured to couple with keyed features, such as the first keyed feature 4444a and the second keyed feature 4444b, of an associated knob. The keys coupled with the keyed features may secure the knob cover 4450 with an associated knob.

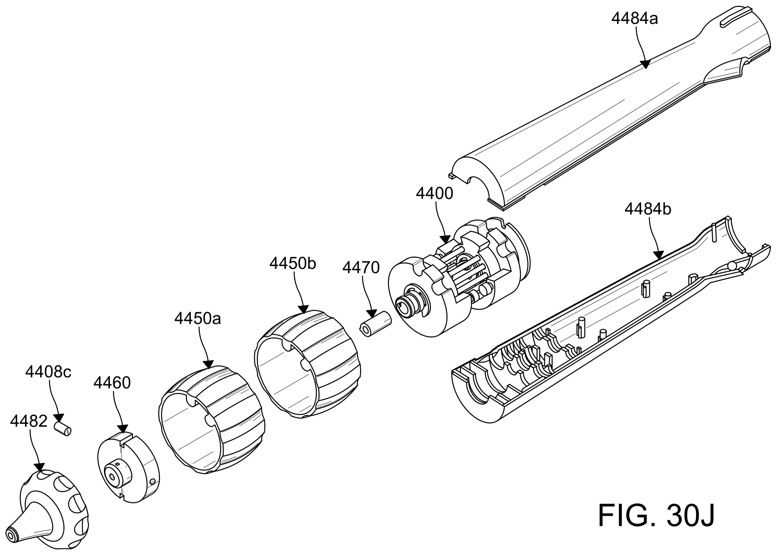

Figure 30H illustrates an example tip rotation base 4460 configured to be in communication with the assembly 4400 of Figure 30A. The tip rotation base 4460 may comprise a generally cylindrical main body configured for communication with a soft tip grip 4482, shown in Figure 30J. The main body may comprise one or more indentions, such as a first indention 4467a and a second indention 4467b, configured to couple (e.g., mate, integrate, etc.) with the soft tip grip 4482. The main body may comprise one or more ball detent aperture (not shown), configured to receive one or more ball detents, such as a third ball detent 4408c (shown in Figure 30J). The one or more ball detent aperture may be threaded. The third ball detent 4408c may be configured to cause the tip rotation base 4460 to lock into a position until engagement of the third ball detent 4408c. The main body of the tip rotation base 4460 may be configured to couple with the retaining groove 4418 of the shaft 4410.

The main body may comprise a protruding area 4465. The protruding area 4465 and/or the main body may comprise an outer shaft through cavity 4461. The outer shaft through cavity 4461 may be configured to receive the shaft 4410, therein. The protruding area 4465 and/or the main body may comprise an adhesive fill cavity 4463, which extends to the outer shaft through cavity 4461 and may be configured to receive adhesive coupling the inner shaft insert 4470 with the tip rotation base 4460.

Figure 30I illustrates an example inner shaft insert 4470 configured to be in communication with the assembly 4400 of Figure 30A. The inner shaft insert 4470 may comprise a cylindrical body. The cylindrical body may comprise a lumen 4471 configured to receive one or more pull wires. The cylindrical body may comprise a key 4472 configured to couple with the lumen 4416 of the shaft 4410. An inner shaft may bond (e.g., couple, affix) within the lumen 4471 of the inner shaft insert 4470. One or more pull lines may be routed through the lumen 4471 of the inner shaft insert 4470.

Figure 30J illustrates an exploded view of a handle assembly configured to comprise the assembly 4400 of Figure 30A. The handle assembly may comprise the assembly 4400. When assembled, the flange 4417 of the assembly 4400 may be disposed in a plurality of handle shells, such as a first handle shell 4484a and a second handle shell 4484b. The A/P knob 4430 and the L/R knob 4440 of the assembly 4400 may be covered by a first knob cover 4450a and a second knob cover 4450b. The inner shaft insert 4470 may couple with the lumen 4416 of the shaft 4410 and/or the outer shaft through cavity 4461 of the tip rotation base 4460. The inner shaft insert 4470 may couple with the lumen 4416 of the shaft 4410 and/or the outer shaft through cavity 4461 of the tip rotation base 4460 using an adhesive. The third ball detent 4408c may be disposed in the tip rotation base 4460. The soft tip grip 4482 may be coupled to the tip rotation base 4460.

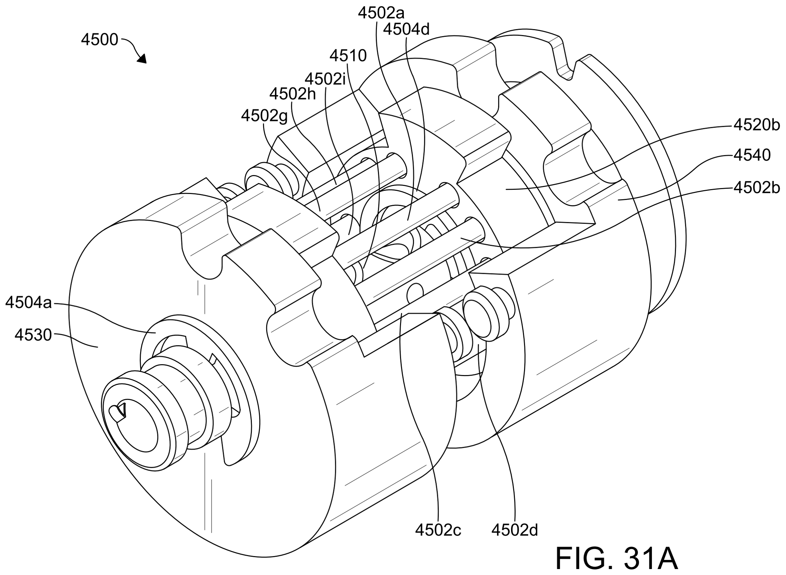

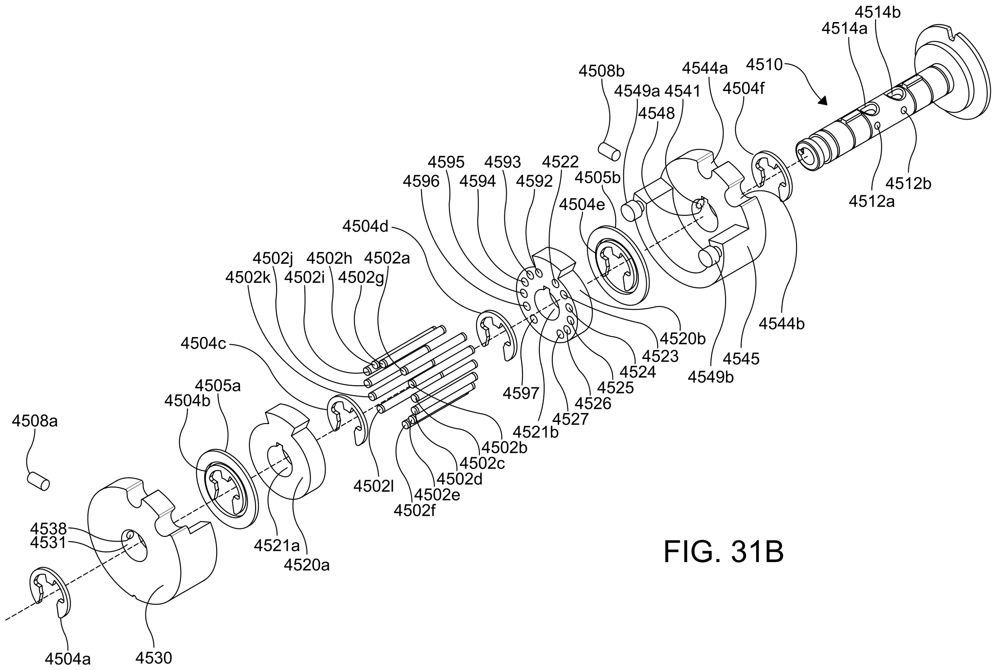

Figure 31A illustrates an example assembly 4500assembly 4500. The assembly 4500assembly 4500 may be disposed in a handle assembly of a catheter. The assembly 4500 may comprise a plurality of pins 4502a, 4502b, 4502c, 4502d, 4502e, 4502f (shown in Figure 31B), 4502g, 4502h, 4502i, 4502j (shown in Figure 31B), 4502k (shown in Figure 31B), and 45021 (shown in Figure 31B). The assembly 4500 may comprise a plurality of retaining rings 4504a, 4504b (shown in Figure 31B), 4504c (shown in Figure 31B), 4504d, 4504e (shown in Figure 31B), and 4504f (shown in Figure 31B). The assembly 4500 may comprise a shaft 4510. The assembly 4500 may comprise a plurality of pin carriers 4520a and 4520b. The assembly 4500 may comprise an anterior and posterior (A/P) knob 4530. The assembly 4500 may comprise a left and right (L/R) knob 4540. The assembly 4500 and components will be described in more detail below. The assembly 4500 may allow for 4-way steering of a catheter tip, independent tip rotation, detent features for a home (e.g., start, initial, original, etc.) position, and auto-lock features. The assembly 4500 may be and/or comprise a cartridge.

Figure 31B illustrates an exploded view of the assembly 4500 of Figure 31A. The pins 4502a, 4502b, 4502c, 4502d, 4502e, 4502f, 4502g, 4502h, 4502i, 4502j, 4502k, and 45021 may comprise a cylindrical shape. The pins 4502a, 4502b, 4502c, 4502d, 4502e, 4502f, 4502g, 4502h, 4502i, 4502j, 4502k, and 45021 may be dowels. The pins 4502a, 4502b, 4502c, 4502d, 4502e, 4502f, 4502g, 4502h, 4502i, 4502j, 4502k, and 45021 may be configured to be partially disposed in a first pin carrier 4520a of the plurality of pin carriers and partially disposed in a second pin carrier 4520b of the plurality of pin carriers. The plurality of pins 4502a, 4502b, 4502c, 4502d, 4502e, 4502f, 4502g, 4502h, 4502i, 4502j, 4502k, and 45021 may be configured to apply tension to one or more pull lines.

The retaining rings 4504a, 4504b, 4504c, 4504d, 4504e, and 4504f may comprise a ring body and a mostly closed aperture formed in the ring body configured to receive the shaft 4510. The retaining rings 4504a, 4504b, 4504c, 4504d, 4504e, and 4504f may comprise side loading retaining rings. The relationship between the retaining rings 4504a, 4504b, 4504c, 4504d, 4504e, and 4504f and the shaft will be described in more detail below. A first retaining ring 4504a may be configured to couple the A/P knob 4530 to the shaft 4510. A second retaining ring 4504b may be configured to couple the A/P knob 4530 and/or the first pin carrier 4520a to the shaft 4510. A third retaining ring 4504c may be configured to couple the first pin carrier 4520a to the shaft 4510. A fourth retaining ring 4504d may be configured to couple the second pin carrier 4520b to the shaft 4510. A fifth retaining ring 4504e may be configured to couple the second pin carrier 4520b and/or the L/R knob 4540 to the shaft 4510. A sixth retaining ring 4504f may be configured to couple the L/R knob 4540 to the shaft 4510.

The assembly 4500 may comprise a plurality of auto-lock rings (e.g., tool locks, etc.), such as a first auto-lock ring 4505a and a second auto-lock ring 4505b. The auto-lock rings may comprise a friction fit with the knobs, such as the A/P knob 4530 and/or the L/R knob 4540, such that the auto-lock rings movable but does not move back to an original position except by action of a user. The auto-lock rings may comprise a substantially flat, disc shape. The auto-locks may comprise an aperture, through which a retaining ring may be disposed. The retaining rings positioned between the knobs and the pin carrier may be surrounded by auto-locks. For example, the first auto-lock ring 4505a may enclose an exterior surface of the second retaining ring 4504b configured to couple the A/P knob 4530 and/or the first pin carrier 4520a to the shaft 4510. As another example, the second auto-lock ring 4505b may enclose an exterior surface of the fifth retaining ring 4504e configured to couple the second pin carrier 4520b and/or the L/R knob 4540 to the shaft 4510.

The shaft 4510 may comprise a plurality of apertures, such as a first dowel pin hole 4512a and a second dowel pin hole 4512b, configured to receive dowel pins, such as a first dowel pin 4506a and a second dowel pin 4506b. The shaft 4510 may comprise a plurality of apertures, such as a first pull line hole 4514a and a second pull line hole 4514b, configured to allow traversal of a pull line. The shaft 4510 will be described in more detail below. The dowel pins may be configured to guide pull lines through the pull line holes. For example, the first dowel pin 4506a may be configured to guide one or more pulls lines through the first pull line hole 4514a, and the second dowel pin 4506b may be configured to guide one or more pulls lines through the second pull line hole 4514b.

The pin carriers 4520, such as the first pin carrier 4520a and the second pin carrier 4520b, may comprise a carrier body and a carrier aperture 4521, such as a first carrier aperture 4521a and a second carrier aperture 4521b, formed in the carrier body configured to receive the shaft 4510. The pin carriers 4520 may comprise a plurality of receptacles, such as a first pin receptacle 4522b and a second pin receptacle 4523b. The pin carriers 4520 will be described in more detail below.

The knobs, such as the A/P knob 4530 and the L/R knob 4540, may comprise a main body and an aperture, such as aperture 4531 and aperture 4541, formed in the main body configured to receive the shaft 4510. The knobs may be configured to control deflection of a catheter tip. For example, the A/P knob 4530 may be configured to control anterior and/or posterior deflection of the catheter tip. As another example, the L/R knob 4540 may be configured to control left and/or right deflection of the catheter tip. The knobs may comprise a protruding area, such as protruding area 4545, configured to allow an associated pin carrier 4520 to reside thereon. A pull line may be coupled to (e.g., anchored, tied, attached, etc.) the protruding area, such as the protruding area 4545. The protruding area 4545 may comprise of the knobs, such as the A/P knob 4530 and the L/R knob 4540, may comprise a plurality of pull line tie points, such as a first pull line tie point 4549a and a second pull line tie point 4549b. The first pull line tie point 4549a and the second pull line tie point 4549b may be disposed on opposing ends of the protruding area 4545 as shown. One or more pull lines may be attached to one or more pull line tie points, such as the first pull line tie point 4549a and the second pull line tie point 4549b.

The knobs may comprise a ball detent aperture, such as ball detent aperture 4538 and ball detent aperture 4548, configured to receive ball detents, such as ball detent 4508a and ball detent 4508b. The ball detent apertures 4538 and 4548 may be configured such that associated ball detents 4508a and 4508b are configured to reside in the ball detent apertures 4538 and 4548. A ball detent, such as the ball detents 4508a and 4508b, may couple with the shaft 4310 to lock a knob into a home (e.g., start, initial, original, etc.) position. The ball detent apertures 4538 and 4548 may comprise threading configured to receive the ball detents 4508a and 4508b.

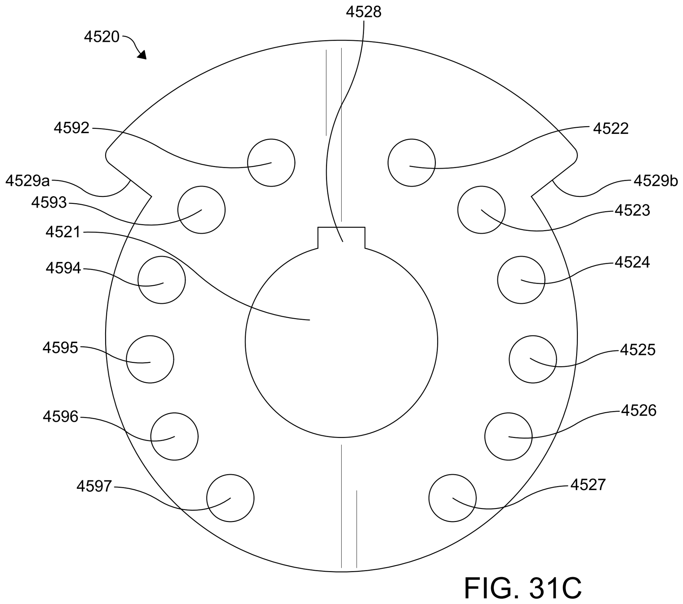

Figure 31C illustrates an example pin carrier 4520 configured to be disposed in the assembly 4500 of Figure 31A. The example pin carrier 4520 may comprise a first pin receptacle 4522, a second pin receptacle 4523, a third pin receptacle 4524, a fourth pin receptacle 4525, a fifth pin receptacle 4526, a sixth pin receptacle 4527, a seventh pin receptacle 4592, an eighth pin receptacle 4593, a ninth pin receptacle 4594, a tenth pin receptacle 4595, an eleventh pin receptacle 4596, and a twelfth pin receptacle 4597. A pin receptacle, such as the pin receptacles 4522-4527 and 4529-4597, may be configured such that a pin is at least partially disposed therein. A pin carrier 4520 may be configured such that the pin receptacles 4522-4527 and 4529-4597 are facing pin receptacles of an opposing pin carrier, such that a plurality of pins may be configured such that the pins are partially disposed in the pin receptacles 4522-4527 and 4529-4597 and partially disposed in the pin receptacles of the opposing pin carrier. At least one of the pins in the pin receptacles 4522-4527 and 4529-4597 may apply tension to one or more pull lines.

The pin carrier 4520 may comprise a carrier aperture 4521. The carrier aperture 4521 may comprise a keyed cutout 4528. The keyed cutout 4528 may couple with a key of the shaft 4510, as described in more detail below. The example pin carrier 4520 may comprise a first rotation limiting surface 4529a and a second rotation limiting surface 4529b. The first rotation limiting surface 4529a and/or the second rotation limiting surface 4529b may act as a barrier to prevent the knobs, such as the A/P knob 4530 and the L/R knob 4540, from further rotating when contact is made with the first rotation limiting surface 4529a and/or the second rotation limiting surface 4529b, to prevent over rotation.

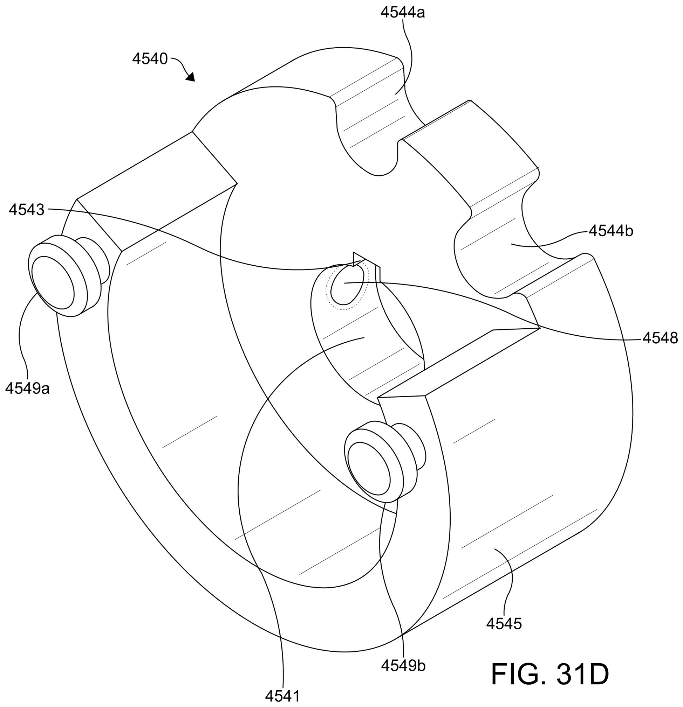

Figure 31D illustrates an example knob 4540 configured to be disposed in the assembly 4500 of Figure 31A. Figure 31D shows the L/R knob 4540. The knob 4540 may comprise the aperture 4541, the protruding area 4545, the ball detent aperture 4548, the first pull line tie point 4549a, and the second pull line tie point 4549b described above. The aperture 4541 may comprise a keyway 4543 configured to allow keys of the shaft 4510 to traverse through. The L/R knob 4540 may comprise a plurality of keyed features, such as a first keyed feature 4544a and a second keyed feature 4544b, configured to couple with keys of a knob cover, described below. The A/P knob 4530 may have similar features. The A/P knob 4530 may have mirrored features.

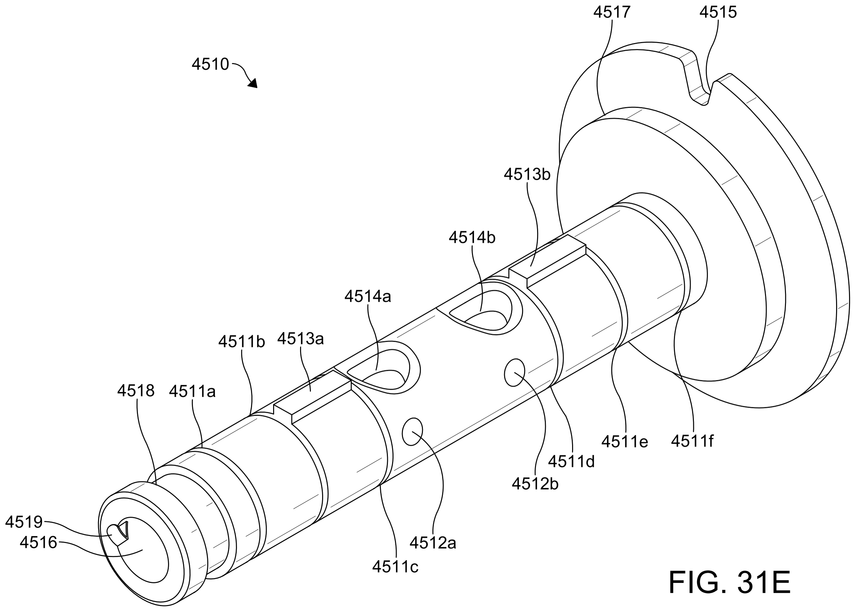

Figure 31E illustrates an example shaft 4510 configured to be disposed in the assembly 4500 of Figure 31A. The shaft 4510 may be elongate. The shaft 4510 may comprise a plurality of retaining ring grooves 4511a, 4511b, 4511c, 4511d, 4511e, and 4511f. The plurality of retaining ring grooves 4511a, 4511b, 4511c, 4511d, 4511e, and 4511f may be configured to couple with the retaining rings 4504a, 4504b, 4504c, 4504d, 4504e, and 4504f. The shaft 4510 may comprise the first dowel pin hole 4512a and the second dowel pin hole 4512b, configured to receive dowel pins, such as the first dowel pin 4506a and the second dowel pin 4506b. The shaft 4510 may comprise a plurality of keys, such as key 4513a and key 4513b, configured to couple with the keyed cutout 4528 of the pin carrier 4520, such as the first pin carrier 4520a and the second pin carrier 4520b.

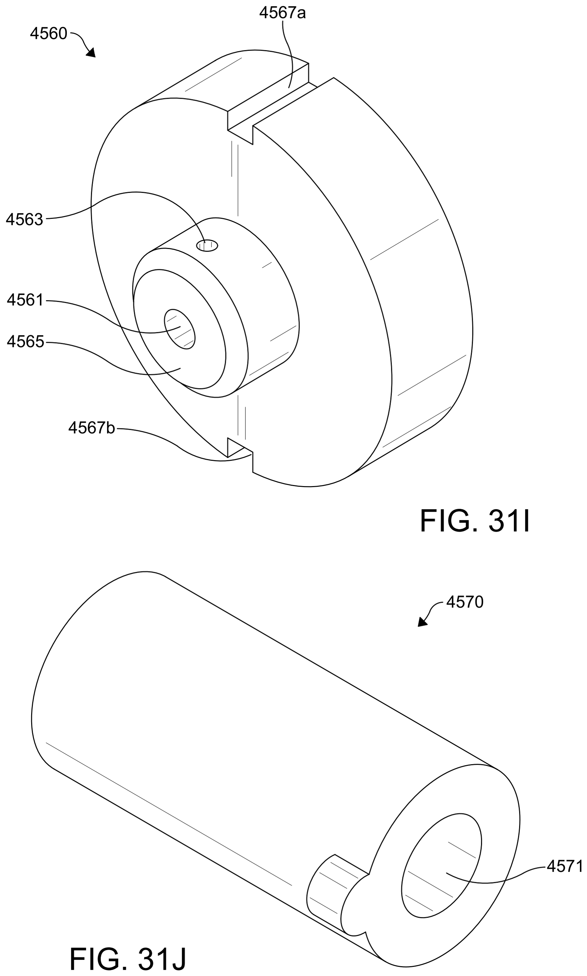

The shaft 4510 may comprise a lumen 4516 configured to house one or more pull lines. The one or more pull lines may exit and/or enter the lumen 4516 via the first pull line hole 4514a and/or the second pull line hole 4514b. For example, the one or more pull lines may comprise an anchor point disposed in the lumen 4516 and exit the lumen 4516 via the first pull line hole 4514a and/or the second pull line hole 4514b. As another example, the one or more pull lines may enter the lumen 4516 exit the lumen 4516 via the first pull line hole 4514a and/or the second pull line hole 4514b. The lumen 4516 may be configured to house an inner shaft insert 4570 (shown in Figure 31J). The lumen 4516 may comprise a keyed feature 4519 configured to couple with a key 4572 of the inner shaft insert 4570. The shaft 4510 may comprise a generally disc-shaped flange 4517 disposed adjacent a proximal end. The flange 4517 may comprise an anti-rotation feature 4515. The anti-rotation feature 4515 may comprise an indention on a rim of the flange 4517, configured to prevent the shaft 4510 from rotating. The shaft 4510 may comprise a retaining groove 4518 configured to couple to a tip rotation base 4560 (shown in Figure 31J).

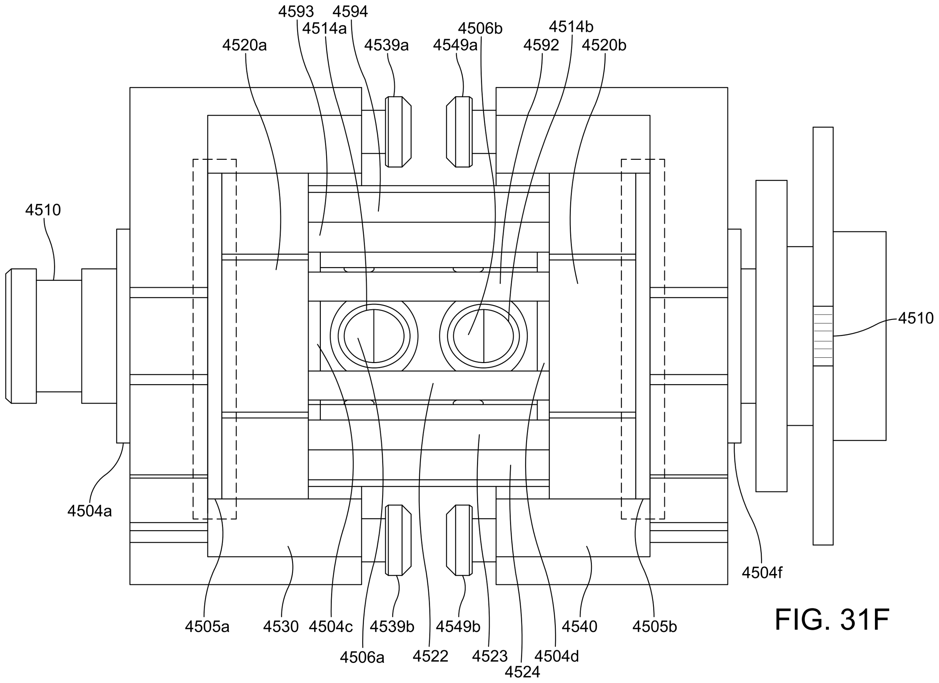

Figure 31F illustrates a top-down view of the assembly 4500 of Figure 31A. As shown, the assembly 4500 may comprise the retaining rings 4504a, 4504b (not shown), 4504c, 4504d, 4504e (not shown), and 4504f, the shaft 4510, the first pin carrier 4520a, the second pin carrier 4520b, the pins 4522-4527 and 4592-4597, the A/P knob 4530 comprising an associated first pull line tie point 4539a, and an associated second pull line tie point 4539b, the L/R knob 4540 comprising the associated first pull line tie point 4549a, and the associated second pull line tie point 4549b. The shaft 4510 may comprise the first pull line hole 4514a. The first dowel pin 4506a may be help guide one or more pull lines through the first pull line hole 4514a. The shaft 4510 may comprise the second pull line hole 4514b. The second dowel pin 4506b may help guide one or more pull lines through the second pull line hole 4514b. The first auto-lock 4505a may surround the second retaining ring 4504b and may be disposed between the A/P knob 4530 and the first pin carrier 4520a. The second auto-lock 4505b may surround the fifth retaining ring 4504e and may be disposed between the L/R knob 4540 and the second pin carrier 4520b. The first auto-lock 4505a may comprise a friction fit with the A/P knob 4530, such that the first auto-lock 4505a is movable but does not move back to an original position except by action of a user. The second auto-lock 4505b may comprise a friction fit with the L/R knob 4540, such that the second auto-lock 4505b is movable but does not move back to an original position except by action of a user.

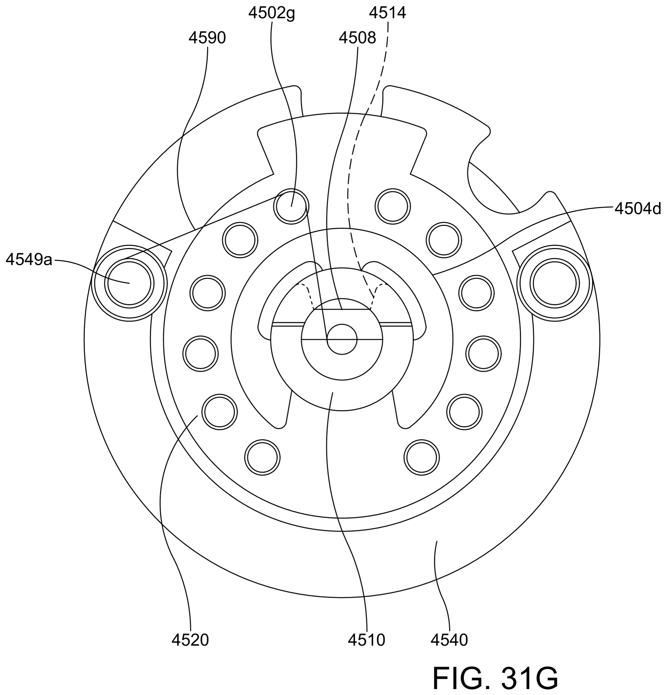

Figure 31G shows a relationships between several components of the assembly 4500 to apply tension to a pull line 4590. As shown, a dowel pin 4506 may be configured to keep the pull line 4590 at a vertical midpoint of a circle created by the lumen 4516 of the shaft 4510. The pull line 4590 may exit the shaft 4510 via the pull line hole 4514. The pull line hole 4514 may comprise a taper. The taper may reduce pull line 4590 interference. The pull line 4590 may wrap around the pin 4502g of the pin carrier 4520. The pull line 4590 may be coupled to the first pull line tie point 4549a of the L/R knob 4540. The fourth retaining ring 4504d may be configured to couple the second pin carrier 4520b to the shaft 4510. Moving the pull line tie points, such as the first pull line tie point 4549a and the second pull line tie point 4549b, closer to the pull line hole 4514 reduces an initial wrap angle. Increasing the number and frequency of pins, such as 4502a-1, and reducing the space between the pins causes less aggressive pull line 4590 wrapping.

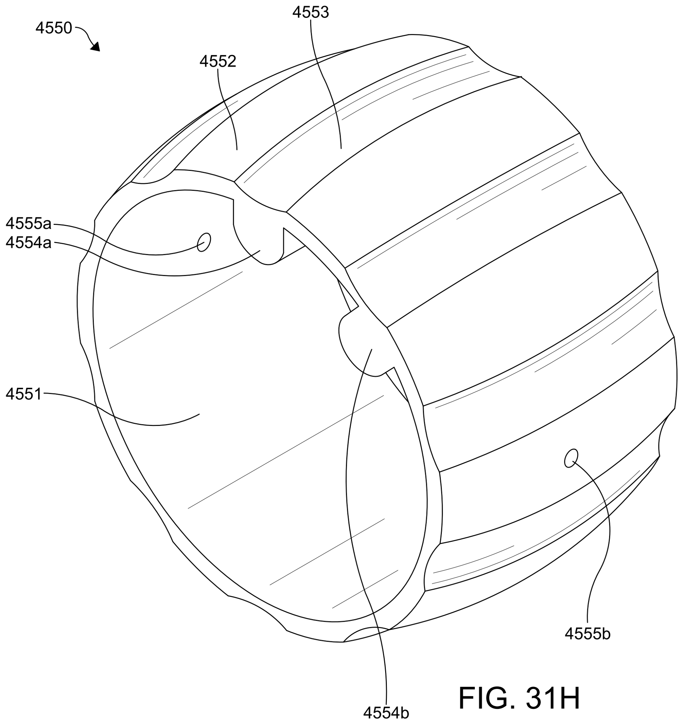

Figure 31H illustrates an example knob cover 4550 configured to be in communication with the assembly 4500 of Figure 31A. The example knob cover 4550 may be configured to surround a knob, such as the A/P knob 4530 and/or the L/R knob 4540, and increase manipulability of the knob. The knob cover 4550 may comprise a body and an aperture 4551 formed in the body configured to receive a knob. An exterior surface of the knob cover 4550 may comprise raised portions, such as raised portion 4552, and recessed portions, such as recessed portion 4553. The combination of raised portions and recessed portions may increase usability. The combination of raised portions and recessed portions may allow for fingers of a user to be coupled with one or more recessed portions. An interior surface of the knob cover 4550 may comprise keys, such as a first key 4554a and a second key 4554b, configured to couple with keyed features, such as the first keyed feature 4544a and the second keyed feature 4544b, of an associated knob. The keys coupled with the keyed features may secure the knob cover 4550 with an associated knob. The knob cover 4550 may comprise a plurality of adhesive fill apertures, such as a first adhesive fill aperture 4555a and a second adhesive fill aperture 4555b. After a knob cover 4550 is disposed over an associated knob, an adhesive syringe may be placed in the first adhesive fill aperture 4555a and/or the second adhesive fill aperture 4555b and adhesive material may be dispensed. The adhesive material dispensed from an adhesive syringe through the first adhesive fill aperture 4555a and/or the second adhesive fill aperture 4555b may bond the knob cover 4550 to an associated knob.

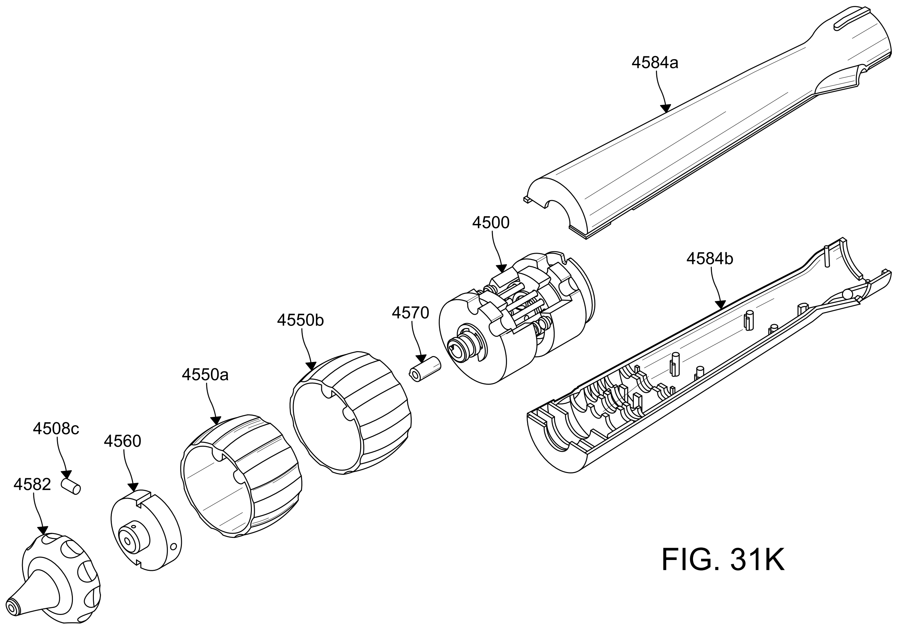

Figure 31I illustrates an example tip rotation base 4560 configured to be in communication with the assembly 4500 of Figure 31A. The tip rotation base 4560 may comprise a generally cylindrical main body configured for communication with a soft tip grip 4582, shown in Figure 31K. The main body may comprise one or more indentions, such as a first indention 4567a and a second indention 4567b, configured to couple (e.g., mate, integrate, etc.) with the soft tip grip 4582. The main body may comprise one or more ball detent aperture (not shown), configured to receive one or more ball detents, such as a third ball detent 4508c (shown in Figure 31K). The one or more ball detent aperture may be threaded. The third ball detent 4508c may be configured to cause the tip rotation base 4560 to lock into a position until engagement of the third ball detent 4508c. The main body of the tip rotation base 4560 may be configured to couple with the retaining groove 4518 of the shaft 4510.

The main body may comprise a protruding area 4565. The protruding area 4565 and/or the main body may comprise an outer shaft through cavity 4561. The outer shaft through cavity 4561 may be configured to receive the shaft 4510, therein. The protruding area 4565 and/or the main body may comprise an adhesive fill cavity 4563, which extends to the outer shaft through cavity 4561 and may be configured to receive adhesive coupling the shaft 4510 with the tip rotation base 4560.

Figure 31J illustrates an example inner shaft insert 4570 configured to be in communication with the assembly 4500 of Figure 31A. The inner shaft insert 4570 may comprise a cylindrical body. The cylindrical body may comprise a lumen 4571 configured to receive one or more pull wires. The cylindrical body may comprise a key 4572 configured to couple with the lumen 4516 of the shaft 4510. An inner shaft may bond (e.g., couple, affix) within the lumen 4571 of the inner shaft insert 4570. One or more pull lines may be routed through the lumen 4571 of the inner shaft insert 4570.

Figure 31K illustrates an exploded view of a handle assembly configured to comprise the assembly 4500 of Figure 31A. The handle assembly may comprise the assembly 4500. When assembled, the flange 4517 of the assembly 4500 may be disposed in a plurality of handle shells, such as a first handle shell 4584a and a second handle shell 4584b. The A/P knob 4530 and the L/R knob 4540 of the assembly 4500 may be covered by a first knob cover 4550a and a second knob cover 4550b. The inner shaft insert 4570 may couple with the lumen 4516 of the shaft 4510 and/or the outer shaft through cavity 4561 of the tip rotation base 4560. The inner shaft insert 4570 may couple with the lumen 4516 of the shaft 4510 and/or the outer shaft through cavity 4561 of the tip rotation base 4560 using an adhesive. The third ball detent 4508c may be disposed in the tip rotation base 4560. The soft tip grip 4582 may be coupled to the tip rotation base 4560.

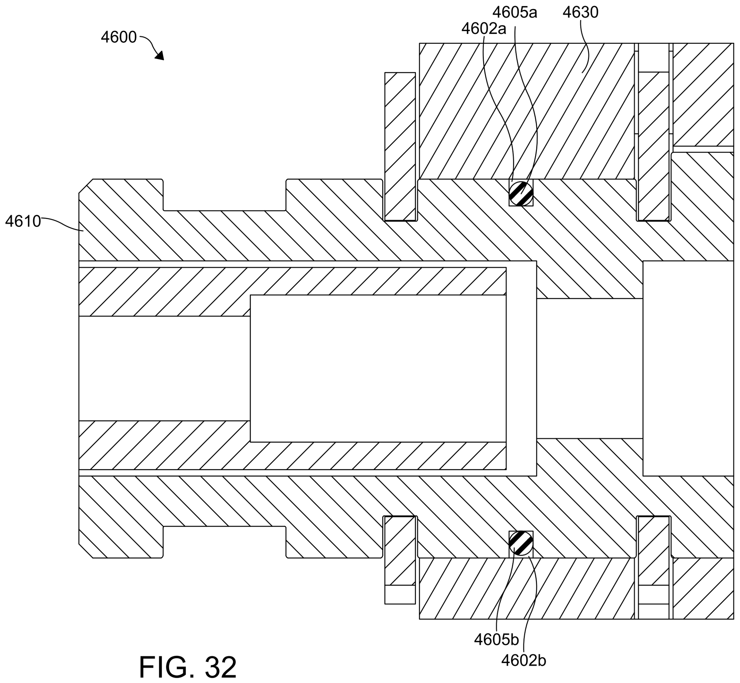

Figure 32 shows an alternate example design 4600 for auto-lock rings. As shown, one or more auto-lock rings, such as a first auto-lock ring 4605a and a second auto-lock ring 4605b, may be placed into grooves on a shaft 4610. The auto-lock rings 4605a and 4605b may interface via one or more through holes, such as a first through hole 4602a and a second through hole 4602b, with one or more knobs, such as a first knob 4630 and a second knob 4640. The auto-lock rings 4605a and 4605b in the alternate example design 4600 are different from previously discussed auto-lock rings, such as auto-lock rings 4505a and 4505b, that are disposed between flat surfaces of a knob and a pin carrier.

### EXAMPLE CLAUSES

Example Clause 1: An assembly for controlling one or more of a deflection and rotation of a medical tool, the assembly may include: an elongate shaft having a generally disc-shaped flange disposed adjacent a proximal end thereof; a first control knob having a first main body and a first aperture formed in the first main body configured to receive the elongate shaft therethrough; a second control knob having a second main body and a second aperture formed in the second main body configured to receive the elongate shaft therethrough; a first pin carrier having a first carrier body and a first carrier aperture formed in the first carrier body configured to receive the elongate shaft therethrough; a second pin carrier having a second carrier body and a second carrier aperture formed in the second carrier body configured to receive the elongate shaft therethrough; a plurality of pins disposed partially within the first pin carrier and disposed partially within the second pin carrier; and a plurality of pull lines disposed through at least a portion of the elongate shaft and extending external to the elongate shaft about one or more of the plurality of pins, where at least a first pull line of the plurality of pull lines is coupled to the first control knob and configured to move in response to an adjustment of the first control knob, where at least a second pull line of the plurality of pull lines is coupled to the second control knob and is configured to move in response to an adjustment of the second control knob, and where the plurality of pins are configured to apply tension to at least one of the first pull line or the second pull line.

Example Clause 2: The assembly of Example Clause 1, where the first pin carrier and the second pin carrier are configured to may include six pins.

Example Clause 3: The assembly of Example Clause 1 or Example Clause 2, where the first pin carrier and the second pin carrier are configured to may include twelve pins.

Example Clause 4: The assembly of any one of Example Clauses 1-3, where the elongate shaft may include at least one aperture configured to receive a dowel pin.

Example Clause 5: The assembly of any one of Example Clauses 1-4, where at least one of the first control knob and the second control knob may include an aperture configured to receive a ball detent.

Example Clause 6: The assembly of any one of Example Clauses 1-5, where the first control knob may include a first protruding portion, and where the first protruding portion is configured to be in communication with the first pin carrier.

Example Clause 7: The assembly of any one of Example Clauses 1-6, where the second control knob may include a second protruding portion, and where the second protruding portion is configured to be in communication with the second pin carrier.

Example Clause 8: The assembly of any one of Example Clauses 1-7, where the first pin carrier may include a first plurality of pin depressions, where the second pin carrier may include a second plurality of pin depressions, where each pin depression is configured to receive at least a portion of a pin, and where the first pin carrier and the second pin carrier are configured such that a pin is capable of being simultaneously disposed partially in the first pin carrier and partially in the second pin carrier.

Example Clause 9: The assembly of any one of Example Clauses 1-8, where one or more of the first pin carrier or the second pin carrier may include at least one surface configured to limit rotation of one or more of the first control knob or the second control knob.

Example Clause 10: The assembly of any one of Example Clauses 1-9, where at least two of the elongate shaft, the first control knob, the second control knob, the first pin carrier, or the second pin carrier are coupled together using at least one retaining ring.

Example Clause 11: The assembly of any one of Example Clauses 1-10, where the elongate shaft may include at least one groove configured to receive the at least one retaining ring.

Example Clause 12: The assembly of any one of Example Clauses 1-11, where the first control knob and the elongate shaft are coupled together using at least one retaining ring.

Example Clause 13: The assembly of any one of Example Clauses 1-12, where the second control knob and the elongate shaft are coupled together using at least one retaining ring.

Example Clause 14: The assembly of any one of Example Clauses 1-13, where the first pin carrier and the elongate shaft are coupled together using at least one retaining ring.

Example Clause 15: The assembly of any one of Example Clauses 1-14, where the second pin carrier and the elongate shaft are coupled together using at least one retaining ring.

Example Clause 16: The assembly of any one of Example Clauses 1-15, further may include at least one tool lock may include a lock body and a lock aperture formed in the lock body configured to receive the elongate shaft therethrough, where the tool lock may include a friction fit with at least one of the first control knob or the second control knob such that the tool lock is movable but does not move back to an original position except by action of a user.

Example Clause 17: The assembly of any one of Example Clauses 1-16, where the elongate shaft may include at least one key feature, and where at least one of the first pin carrier or the second pin carrier may include a keyed feature configured to receive the at least one key feature.

Example Clause 18: The assembly of any one of Example Clauses 1-17, where one or more of the first control knob or the second control knob may include a keyed feature configurated for a knob cover.

Example Clause 19: The assembly of any one of Example Clauses 1-18, where the generally disc-shaped flange may include a registration configured to prevent rotation of the elongate shaft.

Example Clause 20: The assembly of any one of Example Clauses 1-19, where at least one of the first control knob or the second control knob is configured to control deflection of an extendable catheter in a right and/or left direction.

Example Clause 21: The assembly of any one of Example Clauses 1-20, where at least one of the first control knob or the second control knob is configured to control deflection of an extendable catheter in an anterior and/or posterior direction.

Example Clause 22: An assembly for controlling one or more of a deflection and rotation of a medical tool, the assembly may include: an elongate shaft having a generally disc-shaped flange disposed adjacent a proximal end thereof; a first control knob having a first main body and a first aperture formed in the first main body configured to receive the elongate shaft therethrough, where the first control knob may include a first keyed feature configured for a first knob cover, and where the first control knob may include a first anchor; a second control knob having a second main body and a second aperture formed in the second main body configured to receive the elongate shaft therethrough, where the second control knob may include a second keyed feature configured for a second knob cover, and where the second control knob may include a second anchor; a first pin carrier having a first carrier body and a first carrier aperture formed in the first carrier body configured to receive the elongate shaft therethrough; a second pin carrier having a second carrier body and a second carrier aperture formed in the second carrier body configured to receive the elongate shaft therethrough; a plurality of pins disposed partially within the first pin carrier and disposed partially within the second pin carrier; and a plurality of pull lines disposed through at least a portion of the elongate shaft and extending external to the elongate shaft about one or more of the plurality of pins, where at least a first pull line of the plurality of pull lines is coupled to the first anchor and configured to move in response to an adjustment of the first control knob, where at least a second pull line of the plurality of pull lines is coupled to the second anchor and is configured to move in response to an adjustment of the second control knob, and where the plurality of pins are configured to apply tension to at least one of the first pull line or the second pull line.

Example Clause 23: The assembly of Example Clause 22, where the first pin carrier and the second pin carrier are configured to may include six pins.

Example Clause 24: The assembly of Example Clause 22 or Example Clause 23, where the first pin carrier and the second pin carrier are configured to may include twelve pins.

Example Clause 25: The assembly of any one of Example Clauses 22-24, where the elongate shaft may include at least one aperture configured to receive a dowel pin.

Example Clause 26: The assembly of any one of Example Clauses 22-25, where at least one of the first control knob and the second control knob may include an aperture configured to receive a ball detent.

Example Clause 27: The assembly of any one of Example Clauses 22-26, where the first control knob may include a first protruding portion, and where the first protruding portion is configured to be in communication with the first pin carrier.

Example Clause 28: The assembly of any one of Example Clauses 22-27, where the second control knob may include a second protruding portion, and where the second protruding portion is configured to be in communication with the second pin carrier.

Example Clause 29: The assembly of any one of Example Clauses 22-28, where the first pin carrier may include a first plurality of pin depressions, where the second pin carrier may include a second plurality of pin depressions, where each pin depression is configured to receive at least a portion of a pin, and where the first pin carrier and the second pin carrier are configured such that a pin is capable of being simultaneously disposed partially in the first pin carrier and partially in the second pin carrier.

Example Clause 30: The assembly of any one of Example Clauses 22-29, where one or more of the first pin carrier or the second pin carrier may include at least one surface configured to limit rotation of one or more of the first control knob or the second control knob.

Example Clause 31: The assembly of any one of Example Clauses 22-30, where at least two of the elongate shaft, the first control knob, the second control knob, the first pin carrier, or the second pin carrier are coupled together using at least one retaining ring.

Example Clause 32: The assembly of any one of Example Clauses 22-31, where the elongate shaft may include at least one groove configured to receive the at least one retaining ring.

Example Clause 33: The assembly of any one of Example Clauses 22-32, where the first control knob and the elongate shaft are coupled together using at least one retaining ring.

Example Clause 34: The assembly of any one of Example Clauses 22-33, where the second control knob and the elongate shaft are coupled together using at least one retaining ring.

Example Clause 35: The assembly of any one of Example Clauses 22-34, where the first pin carrier and the elongate shaft are coupled together using at least one retaining ring.

Example Clause 36: The assembly of any one of Example Clauses 22-35, where the second pin carrier and the elongate shaft are coupled together using at least one retaining ring.

Example Clause 37: The assembly of any one of Example Clauses 22-36, further may include at least one tool lock may include a lock body and a lock aperture formed in the lock body configured to receive the elongate shaft therethrough, where the tool lock may include a friction fit with at least one of the first control knob or the second control knob such that the tool lock is movable but does not move back to an original position except by action of a user.

Example Clause 38: The assembly of any one of Example Clauses 22-37, where the elongate shaft may include at least one key feature, and where at least one of the first pin carrier or the second pin carrier may include a keyed feature configured to receive the at least one key feature.

Example Clause 39: The assembly of any one of Example Clauses 22-38, where the generally disc-shaped flange may include a registration configured to prevent rotation of the elongate shaft.

Example Clause 40: The assembly of any one of Example Clauses 22-39, where at least one of the first control knob or the second control knob is configured to control deflection of an extendable catheter in a right and/or left direction.

Example Clause 41: The assembly of any one of Example Clauses 22-40, where at least one of the first control knob or the second control knob is configured to control deflection of an extendable catheter in an anterior and/or posterior direction.

Example Clause 42: An assembly for controlling one or more of a deflection and rotation of a medical tool, the assembly may include: an elongate shaft having a generally disc-shaped flange disposed adjacent a proximal end thereof; a first control knob having a first main body and a first aperture formed in the first main body configured to receive the elongate shaft therethrough, where the first control knob may include a first keyed feature configured for a first knob cover, and where the first control knob may include a first anchor and a second anchor; a second control knob having a second main body and a second aperture formed in the second main body configured to receive the elongate shaft therethrough, where the second control knob may include a second keyed feature configured for a second knob cover, and where the second control knob may include a third anchor and a fourth anchor; a first pin carrier having a first carrier body and a first carrier aperture formed in the first carrier body configured to receive the elongate shaft therethrough; a second pin carrier having a second carrier body and a second carrier aperture formed in the second carrier body configured to receive the elongate shaft therethrough; a plurality of pins disposed partially within the first pin carrier and disposed partially within the second pin carrier; and a plurality of pull lines disposed through at least a portion of the elongate shaft and extending external to the elongate shaft about one or more of the plurality of pins, where at least a first pull line of the plurality of pull lines is coupled to the first anchor and configured to move in response to an adjustment of the first control knob, where at least a second pull line of the plurality of pull lines is coupled to the second anchor and configured to move in response to an adjustment of the first control knob, where at least a third pull line of the plurality of pull lines is coupled to the third anchor and is configured to move in response to an adjustment of the second control knob, where at least a fourth pull line of the plurality of pull lines is coupled to the fourth anchor and is configured to move in response to an adjustment of the second control knob, and where the plurality of pins are configured to apply tension to at least one of the first pull line, the second pull line, the third pull line, or the fourth pull line.

Example Clause 43: The assembly of Example Clause 42, where the first pin carrier and the second pin carrier are configured to may include six pins.

Example Clause 44: The assembly of Example Clause 42 or Example Clause 43, where the first pin carrier and the second pin carrier are configured to may include twelve pins.

Example Clause 45: The assembly of any one of Example Clauses 42-44, where the elongate shaft may include at least one aperture configured to receive a dowel pin.

Example Clause 46: The assembly of any one of Example Clauses 42-45, where at least one of the first control knob and the second control knob may include an aperture configured to receive a ball detent.

Example Clause 47: The assembly of any one of Example Clauses 42-46, where the first control knob may include a first protruding portion, and where the first protruding portion is configured to be in communication with the first pin carrier.

Example Clause 48: The assembly of any one of Example Clauses 42-47, where the second control knob may include a second protruding portion, and where the second protruding portion is configured to be in communication with the second pin carrier.

Example Clause 49: The assembly of any one of Example Clauses 42-48, where the first pin carrier may include a first plurality of pin depressions, where the second pin carrier may include a second plurality of pin depressions, where each pin depression is configured to receive at least a portion of a pin, and where the first pin carrier and the second pin carrier are configured such that a pin is capable of being simultaneously disposed partially in the first pin carrier and partially in the second pin carrier.

Example Clause 50: The assembly of any one of Example Clauses 42-49, where one or more of the first pin carrier or the second pin carrier may include at least one surface configured to limit rotation of one or more of the first control knob or the second control knob.

Example Clause 51: The assembly of any one of Example Clauses 42-50, where at least two of the elongate shaft, the first control knob, the second control knob, the first pin carrier, or the second pin carrier are coupled together using at least one retaining ring.

Example Clause 52: The assembly of any one of Example Clauses 42-51, where the elongate shaft may include at least one groove configured to receive the at least one retaining ring.

Example Clause 53: The assembly of any one of Example Clauses 42-52, where the first control knob and the elongate shaft are coupled together using at least one retaining ring.

Example Clause 54: The assembly of any one of Example Clauses 42-53, where the second control knob and the elongate shaft are coupled together using at least one retaining ring.

Example Clause 55: The assembly of any one of Example Clauses 42-54, where the first pin carrier and the elongate shaft are coupled together using at least one retaining ring.

Example Clause 56: The assembly of any one of Example Clauses 42-55, where the second pin carrier and the elongate shaft are coupled together using at least one retaining ring.

Example Clause 57: The assembly of any one of Example Clauses 42-56, further may include at least one tool lock may include a lock body and a lock aperture formed in the lock body configured to receive the elongate shaft therethrough, where the tool lock may include a friction fit with at least one of the first control knob or the second control knob such that the tool lock is movable but does not move back to an original position except by action of a user.

Example Clause 58: The assembly of any one of Example Clauses 42-57, where the elongate shaft may include at least one key feature, and where at least one of the first pin carrier or the second pin carrier may include a keyed feature configured to receive the at least one key feature.

Example Clause 59: The assembly of any one of Example Clauses 42-58, where the generally disc-shaped flange may include a registration configured to prevent rotation of the elongate shaft.

Example Clause 60: The assembly of any one of Example Clauses 42-59, where at least one of the first control knob or the second control knob is configured to control deflection of an extendable catheter in a right and/or left direction.

Example Clause 61: The assembly of any one of Example Clauses 42-60, where at least one of the first control knob or the second control knob is configured to control deflection of an extendable catheter in an anterior and/or posterior direction.

The foregoing disclosure provides illustration and description but is not intended to be exhaustive or to limit the implementations to the precise form disclosed. Modifications may be made in light of the above disclosure or may be acquired from practice of the implementations. As used herein, satisfying a threshold may, depending on the context, refer to a value being greater than the threshold, greater than or equal to the threshold, less than the threshold, less than or equal to the threshold, equal to the threshold, and/or the like, depending on the context. Although particular combinations of features are recited in the claims and/or disclosed in the specification, these combinations are not intended to limit the disclosure of various implementations. In fact, many of these features may be combined in ways not specifically recited in the claims and/or disclosed in the specification

Although each dependent claim listed below may directly depend on only one claim, the disclosure of various implementations includes each dependent claim in combination with every other claim in the claim set. No element, act, or instruction used herein should be construed as critical or essential unless explicitly described as such. Also, as used herein, the articles "a" and "an" are intended to include one or more items and may be used interchangeably with "one or more." Further, as used herein, the article "the" is intended to include one or more items referenced in connection with the article "the" and may be used interchangeably with "the one or more." Furthermore, as used herein, the term "set" is intended to include one or more items (e.g., related items, unrelated items, a combination of related and unrelated items, and/or the like), and may be used interchangeably with "one or more." Where only one item is intended, the phrase "only one" or similar language is used. Also, as used herein, the terms "has," "have," "having," or the like are intended to be open-ended terms. Further, the phrase "based on" is intended to mean "based, at least in part, on" unless explicitly stated otherwise. Also, as used herein, the term "or" is intended to be inclusive when used in a series and may be used interchangeably with "and/or," unless explicitly stated otherwise (e.g., if used in combination with "either" or "only one of").

## Claims

1. An assembly for controlling one or more of a deflection and rotation of a medical tool, the assembly comprising:
an elongate shaft having a flange disposed adjacent a proximal end thereof;
a first control knob having a first main body and a first aperture formed in the first main body configured to receive the elongate shaft therethrough;
a second control knob having a second main body and a second aperture formed in the second main body configured to receive the elongate shaft therethrough;
a first pin carrier having a first carrier body and a first carrier aperture formed in the first carrier body configured to receive the elongate shaft therethrough;
a second pin carrier having a second carrier body and a second carrier aperture formed in the second carrier body configured to receive the elongate shaft therethrough;
a plurality of pins disposed partially within the first pin carrier and disposed partially within the second pin carrier; and
a plurality of pull lines disposed through at least a portion of the elongate shaft and extending external to the elongate shaft about one or more of the plurality of pins, wherein at least a first pull line of the plurality of pull lines is coupled to the first control knob and configured to move in response to an adjustment of the first control knob, wherein at least a second pull line of the plurality of pull lines is coupled to the second control knob and is configured to move in response to an adjustment of the second control knob, and wherein the plurality of pins are configured to apply tension to at least one of the first pull line or the second pull line.

2. The assembly of claim 1, wherein the first pin carrier and the second pin carrier are configured to comprise six pins or twelve pins.

3. The assembly of claim 1 or claim 2, wherein the elongate shaft comprises at least one aperture configured to receive a dowel pin.

4. The assembly of any preceding claim, wherein at least one of the first control knob and the second control knob comprise an aperture configured to receive a ball detent.

5. The assembly of any preceding claim, wherein the first control knob comprises a first protruding portion, and wherein the first protruding portion is configured to be in communication with the first pin carrier and/or wherein the second control knob comprises a second protruding portion, and wherein the second protruding portion is configured to be in communication with the second pin carrier.

6. The assembly of any preceding claim, wherein the elongate shaft comprises at least one key feature, and wherein at least one of the first pin carrier or the second pin carrier comprise a keyed feature configured to receive the at least one key feature.

7. The assembly of any preceding claim, wherein one or more of the first control knob or the second control knob comprise a keyed feature configurated for a knob cover.

8. The assembly of claim 1, wherein the flange comprises a registration configured to prevent rotation of the elongate shaft.

9. An assembly for controlling one or more of a deflection and rotation of a medical tool, the assembly comprising:
an elongate shaft having a flange disposed adjacent a proximal end thereof;
a first control knob having a first main body and a first aperture formed in the first main body configured to receive the elongate shaft therethrough, wherein the first control knob comprises a first keyed feature configured for a first knob cover, and wherein the first control knob comprises a first anchor;
a second control knob having a second main body and a second aperture formed in the second main body configured to receive the elongate shaft therethrough, wherein the second control knob comprises a second keyed feature configured for a second knob cover, and wherein the second control knob comprises a second anchor;
a first pin carrier having a first carrier body and a first carrier aperture formed in the first carrier body configured to receive the elongate shaft therethrough;
a second pin carrier having a second carrier body and a second carrier aperture formed in the second carrier body configured to receive the elongate shaft therethrough;
a plurality of pins disposed partially within the first pin carrier and disposed partially within the second pin carrier; and
a plurality of pull lines disposed through at least a portion of the elongate shaft and extending external to the elongate shaft about one or more of the plurality of pins, wherein at least a first pull line of the plurality of pull lines is coupled to the first anchor and configured to move in response to an adjustment of the first control knob, wherein at least a second pull line of the plurality of pull lines is coupled to the second anchor and is configured to move in response to an adjustment of the second control knob, and wherein the plurality of pins are configured to apply tension to at least one of the first pull line or the second pull line.

10. The assembly of any of claims 1 to 5 or claim 9, wherein the first pin carrier comprises a first plurality of pin depressions, wherein the second pin carrier comprises a second plurality of pin depressions, wherein each pin depression is configured to receive at least a portion of a pin, and wherein the first pin carrier and the second pin carrier are configured such that a pin is capable of being simultaneously disposed partially in the first pin carrier and partially in the second pin carrier.

11. The assembly of any of claims 1 to 5, 9 or 10, wherein one or more of the first pin carrier or the second pin carrier comprise at least one surface configured to limit rotation of one or more of the first control knob or the second control knob.

12. The assembly of any of claims 1 to 5, 9, 10 or 11, further comprising at least one tool lock comprising a lock body and a lock aperture formed in the lock body configured to receive the elongate shaft therethrough, wherein the tool lock comprises a friction fit with at least one of the first control knob or the second control knob such that the tool lock is movable but does not move back to an original position except by action of a user.

13. An assembly for controlling one or more of a deflection and rotation of a medical tool, the assembly comprising:
an elongate shaft having a flange disposed adjacent a proximal end thereof;
a first control knob having a first main body and a first aperture formed in the first main body configured to receive the elongate shaft therethrough;
a second control knob having a second main body and a second aperture formed in the second main body configured to receive the elongate shaft therethrough;
a first pin carrier having a first carrier body and a first carrier aperture formed in the first carrier body configured to receive the elongate shaft therethrough;
a second pin carrier having a second carrier body and a second carrier aperture formed in the second carrier body configured to receive the elongate shaft therethrough;
a plurality of pins disposed partially within the first pin carrier and disposed partially within the second pin carrier; and
a plurality of pull lines disposed through at least a portion of the elongate shaft and extending external to the elongate shaft about one or more of the plurality of pins, wherein at least a first pull line of the plurality of pull lines is configured to move in response to an adjustment of the first control knob, wherein at least a second pull line of the plurality of pull lines is configured to move in response to an adjustment of the second control knob, and wherein the plurality of pins are configured to apply tension to at least one of the first pull line or the second pull.

14. The assembly of claim 13, further comprising an elongate catheter in communication with the plurality of pull lines such that tension in one or more of the plurality of pull lines controls a steering of the catheter.

15. The assembly of claim 14, wherein at least one of the first control knob or the second control knob is configured to control deflection of the catheter in a right and/or left direction and wherein at least one of the first control knob or the second control knob is configured to control deflection of the catheter in an anterior and/or posterior direction.
